(19) **Europäisches Patentamt European Patent Office Office européen des brevets**

(11) **EP 4 674 954 A1**

(12) **EUROPEAN PATENT APPLICATION**
published in accordance with Art. 153(4) EPC

(43) Date of publication:
07.01.2026 Bulletin 2026/02

(21) Application number: 24763146.8

(22) Date of filing: 27.02.2024

(51) International Patent Classification (IPC):
C12N 5/0783 (2010.01)   C07K 19/00 (2006.01)
C12N 15/62 (2006.01)   C12N 5/10 (2006.01)
A61K 39/00 (2006.01)   A61P 35/00 (2006.01)

(52) Cooperative Patent Classification (CPC):
A61K 39/00; A61P 35/00; C07K 19/00; C12N 5/06;
C12N 5/10; C12N 15/62

(86) International application number:
PCT/CN2024/078802

(87) International publication number:
WO 2024/179465 (06.09.2024 Gazette 2024/36)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 27.02.2023  CN 202310170703

(71) Applicant: Shanghai Juncell Therapeutics Co.,
Ltd.
Shanghai 201800 (CN)

(72) Inventors:
• JIN, Huajun
  Shanghai 201800 (CN)

• XU, Fuhui
  Shanghai 201800 (CN)
• HE, Zhou
  Shanghai 201800 (CN)
• HUANG, Chen
  Shanghai 201800 (CN)

(74) Representative: Nederlandsch Octrooibureau
P.O. Box 29720
2502 LS The Hague (NL)

Remarks:
The complete document including Reference
Table(s) and the Sequence Listing(s) can be
downloaded from the EPO website

(54) **TUMOR INFILTRATING LYMPHOCYTE EXPRESSING MEMBRANE-BOUND CYTOKINE**

(57)     Provided is a tumor infiltrating lymphocyte expressing a membrane-bound cytokine, specifically a modified TIL expressing membrane-anchored IL-7. The TIL can be effectively activated and proliferated, can be efficiently detected and sorted, and can effectively mediate molecular braking.

EP 4 674 954 A1

**Description**

TECHNICAL FIELD

The present invention relates to the field of biotechnology, and in particular to a tumor infiltrating lymphocyte expressing a membrane-bound cytokine and a preparation method thereof.

BACKGROUND

Tumor infiltrating lymphocyte (TIL) therapy is an emerging immune cell therapy that has initially shown excellent efficacy against solid tumors. The TILs present in the tumor are a highly heterogeneous group that is immunosuppressed by the tumor microenvironment, have limited tumor cytotoxic effects and a limited persistence duration in the body. Current TIL therapy involves culturing reactivated and expanded TIL populations from solid tumor tissues of patients *in vitro,* which are then reinfused into the patients' bodies to improve targeted cytotoxicity against solid tumors. However, the immunosuppressive microenvironment inside the tumor still has an inhibitory effect on the infused TILs, and the activity and persistence of the infused TILs in the body still need to be elevated.

Modifying the unmodified natural TILs to be infused with exogenous genes is one of the methods to solve the problems above. However, there are currently few types of genetically modified TILs in the art, and there is an urgent need for more engineering technologies that can enhance the proliferative ability and tumor cytotoxic efficacy of TILs and prolong persistence of TILs in the body.

SUMMARY

The first aspect of the present invention provides a modified TIL, which expresses a membrane-anchored cytokine, and the cytokine is a membrane-anchored IL-7.

In one or more embodiments, the membrane-anchored IL-7 comprises IL-7 or a functional fragment thereof and a transmembrane domain. Preferably, IL-7 or a functional fragment thereof is located in the N-terminus of the transmembrane domain.

In one or more embodiments, the amino acid sequence of the IL-7 is shown in SEQ ID NO:7; its exemplary coding sequence is shown in SEQ ID NO:8.

In one or more embodiments, the transmembrane domain is a protein transmembrane domain. Preferably, the protein transmembrane domain comprises any one selected from the group consisting of a CD8 transmembrane domain, a CD28 transmembrane domain, and an IL-7R α transmembrane domain. Preferably, the CD8 transmembrane domain is shown in SEQ ID NO:3, and its exemplary coding sequence is shown in SEQ ID NO:4.

In one or more embodiments, the transmembrane domain is a GPI anchoring domain. Preferably, the GPI anchoring domain comprises a membrane anchoring domain of one or more GPI-anchored membrane proteins selected from the group consisting of: CD44, CD56, CD73, CD55, Thy1, AchE, IAP, ALPP, CD59, CD14, CD16, CD24, CD28, CD48, CD52, CD58, CD66a, CD66c, CD66d, CD66e, CD67, CD87, CD108, CD157, uPAR, JMH protein, GDNFR, CNTFR, TAG-1, PrP, phosphatidylinositol protein, semaphorin 7, CEA, GFR, Ly6G, transferrin receptor, contactin (F3), and T-cadherin. More preferably, the GPI anchoring domain is selected from the membrane anchoring region of one or more GPI-anchored membrane proteins selected from the group consisting of: CD52, CD48, CD55, ALPP, CD90.

In one or more embodiments, the membrane-anchored IL-7 further comprises a membrane surface tag. Preferably, the membrane surface tag is located between IL-7 and the transmembrane domain. Preferably, the membrane-anchored IL-7 sequentially comprises, from the N-terminus to the C-terminus, IL-7, a membrane surface tag, and a transmembrane domain.

In one or more embodiments, the membrane surface tag comprises an extracellular domain of a B-cell surface antigen or a variant thereof that retains the ability to bind to an antibody. Preferably, the B-cell surface antigen is BCMA, and the membrane surface tag is an extracellular domain of BCMA or a variant thereof that retains the ability to bind to an anti-BCMA antibody. Preferably, the variant is a truncated variant, an insertion variant, a deletion variant, a substitution variant, or a combination thereof.

In one or more embodiments, the BCMA extracellular domain is shown in SEQ ID NO:21.

In one or more embodiments, the BCMA extracellular domain variant is a truncated variant, such as shown in SEQ ID NO:23.

In one or more embodiments, the BCMA extracellular domain variant is a truncated substitution variant, such as shown in SEQ ID NO:25.

In one or more embodiments, the membrane-anchored IL-7 further comprises a linker between IL-7 and the membrane surface tag. In one or more embodiments, the linker is a rigid linker or a flexible linker, preferably a rigid linker. Preferably, the sequence of the rigid linker is selected from any one of SEQ ID NO:9, 11, 15, and 17.

In one or more embodiments, the membrane-anchored IL-7 further comprises a hinge region or a linker located at the N-terminus of the transmembrane domain (e.g., between the membrane surface tag and the transmembrane domain). Hinge regions include, but are not limited to, CD4 extracellular hinge region, CD8 extracellular hinge region, CD28 extracellular hinge region, IgG1Fc hinge region, and IgG4Fc hinge region. Preferably, the hinge region is a CD8 extracellular hinge region. Preferably, the sequence of the CD8 extracellular hinge region is as shown in SEQ ID NO: 19. The linker is a rigid linker or a flexible linker, preferably a rigid linker. Preferably, the sequence of the rigid linker is selected from any one of SEQ ID NO: 9, 11, 15, 17.

Preferably, the linker is located between IL-7 and the membrane surface tag and/or between the membrane surface tag and the transmembrane domain.

In one or more embodiments, the membrane-anchored IL-7 further comprises a signal peptide. The signal peptide is located at the N-terminus of the membrane-anchored IL-7. Preferably, the signal peptide is a CD52 signal peptide or an IL-7 signal peptide. Preferably, the CD52 signal peptide is shown in SEQ ID NO: 1; its exemplary coding sequence is shown in SEQ ID NO: 2. Preferably, the IL-7 signal peptide is shown in SEQ ID NO: 5; its exemplary coding sequence is shown in SEQ ID NO: 6.

In one or more embodiments, the membrane-anchored IL-7 comprises, sequentially linked: an optional signal peptide, IL-7, an optional linker, an extracellular domain of BCMA or a variant thereof, an optional hinge region or linker, and a transmembrane domain, wherein the signal peptide is a CD52 signal peptide or an IL-7 signal peptide, and the transmembrane domain is a CD8 transmembrane domain or CD52.

In one or more embodiments, the membrane-anchored IL-7 comprises, sequentially linked: a signal peptide, IL-7, a linker, a BCMA extracellular domain or a variant thereof, and a transmembrane domain.

In one or more embodiments, the membrane-anchored IL-7 comprises, sequentially linked:

a CD52 signal peptide, IL-7, a linker, a BCMA extracellular domain or variant thereof, and a CD8 transmembrane domain,

a CD52 signal peptide, IL-7, a linker, a BCMA extracellular domain or variant thereof, and CD52,

a CD52 signal peptide, IL-7, a linker, a BCMA extracellular domain or variant thereof, a hinge region or linker, and CD52,

a CD52 signal peptide, IL-7, a BCMA extracellular domain or variant thereof, a hinge region or linker, and CD52,

an IL-7 signal peptide, IL-7, a linker, a BCMA extracellular domain or variant thereof, and a CD8 transmembrane domain,

an IL-7 signal peptide, IL-7, a linker, a BCMA extracellular domain or variant thereof, and CD52.

In one or more embodiments, the amino acid sequence of the membrane-anchored IL-7 is any one or more selected from SEQ ID NO: 31, 33, 35, 37, 39, 41, 43, 45 and 47.

In one or more embodiments, the nucleic acid sequence encoding the membrane-anchored IL-7 is any one or more of the nucleic acid sequences encoding the above mentioned SEQ ID NO: 31, 33, 35, 37, 39, 41, 43, 45, and 47. Preferably, the nucleic acid sequence encoding the membrane-anchored IL-7 is any one or more selected from SEQ ID NO: 32, 34, 36, 38, 40, 42, 44, 46, and 48.

In a first group of embodiments of the first aspect, the TIL is prepared by the following method: incubating a sample containing TIL with a TIL seed cell medium to obtain a TIL population.

In one or more embodiments, the sample is selected from the group consisting of: ascites, surgically removed primary lesion samples, synchronous and metachronous surgically removed metastatic lesion samples, biopsy samples, and body fluids.

In one or more embodiments, the sample is a tissue containing tumor cells. Preferably, the tissue containing tumor cells is subjected to pretreatment, including fragmentation and/or dissociation of the tumor tissue. The tumor cells are derived from a tumor selected from the group consisting of: melanoma, glioma, thyroid tumor, gastric cancer, lung cancer, gastrointestinal stromal tumor, intestinal cancer, liver cancer, cervical cancer, ovarian cancer, breast cancer, endometrial stromal sarcoma, pelvic poorly differentiated adenocarcinoma, gallbladder cancer, bile duct cancer, head and neck cancer, colorectal cancer, glioblastoma, pancreatic cancer, bladder cancer, prostate cancer, renal cancer, and osteosarcoma. Preferably, the tissue containing tumor cells is a tumor tissue or body fluid from a cancer subject.

In one or more embodiments, the body fluid comprises blood, pleural fluid, interstitial fluid, lymphatic fluid, and/or ascites.

In one or more embodiments, the seed cell culture medium comprises the following components: cell culture components, cytokines and immune checkpoint antibodies or their antigen-binding fragments, wherein the cytokines include IL-2, the immune checkpoints include: PD-1, LAG-3, TIGIT and/or CTLA-4, and the cell culture components are serum-containing medium or serum-free medium.

In one or more embodiments, the TIL seed cell medium comprises any one of the following combinations of components: 1) IL-2, IL-6, IL-21, IFN-γ, anti-TIGIT antibody, anti-PD-1 antibody, TNF-α and a basal medium; 2) IL-2, IL-4, IL-10, IL-21, anti-CD137 antibody, anti-LAG3 antibody, anti-PD-1 antibody, TNF-α and a basal medium; 3) IL-2, IL-7, IL-12, IL-21, anti-CD137 antibody, anti-CD28 antibody, anti-PD-1 antibody and a basal medium; 4) IL-1β, IL-2, IL-7, G-CSF, GM-CSF, IFN-γ, anti-LAG3 antibody, anti-PD-1 antibody, TNF-α and a basal medium; 5) IL-2, IL-4, IL-12, GM-CSF, M-CSF, IFN-β, IFN-γ, anti-TIGIT antibody, anti-CTLA-4 antibody and a basal medium; 6) IL-2, IL-7, IL-15, GM-CSF, anti-CD137 antibody, anti-PD-1 antibody, TNF-α and a basal medium; 7) IL-2, IL-4, IL-10, IL-15, G-CSF, M-CSF, anti-CD28 antibody, anti-OX-40 antibody, anti-PD-1 antibody and a basal medium; 8) IL-2, IL-7, IL-15, IFN-γ, anti-CD137 antibody, anti-CD40 antibody, anti-OX-40 antibody, anti-TIGIT antibody, anti-PD-1 antibody and a basal medium; 9) IL-2, IL-7, IL-15, GM-CSF, IFN-γ, anti-CD137 antibody, anti-CD28 antibody, anti-PD-1 antibody, TNF-α and a basal medium; 10) IL-2, IL-7, IL-12, G-CSF, GM-CSF, IFN-α, IFN-γ, anti-CD28 antibody, anti-CD40 antibody, anti-TIGIT antibody, anti-PD-1 antibody, TNF-α and a basal medium; 11) IL-2, IL-7, IL-15, GM-CSF, anti-PD-1 antibody, RRx-001, CAL-101 and a basal medium; 12) IL-2, IL-7, IL-15, GM-CSF, M-CSF, anti-PD-1 antibody, CNI-1493 and a basal medium medium; 13) IL-2, IL-7, IL-15, anti-CD137 antibody, anti-CD28 antibody, anti-LAG3 antibody, anti-PD-1 antibody, dasatinib and a basal medium; 14) IL-2, IL-6, IL-12, G-CSF, M-CSF, IFN-β, IFN-γ, anti-CTLA-4 antibody, anti-PD-1 antibody, dasatinib, LYC-55716, GENE-1858 and a basal medium; 15) IL-1α, IL-2, I L-9, IL-15, GM-CSF, anti-CD137 antibody, anti-CD28 antibody, anti-LAG3 antibody, anti-TIGIT antibody, CNI-1493 and a basal medium; 16) IL-2, IL-7, IL-12, IL-15, IL-21, G-CSF, M-CSF, IFN-γ, anti-CD28 antibody, anti-CD40 antibody, anti-LAG3 antibody, anti-PD-1 antibody, CNI-1493, dasatinib, GNE-1858 and a basal medium. Preferably, the combination of the above components further comprises serum and/or antibiotics.

In a second group of embodiments of the first aspect, the TIL is prepared by the following method: (1) incubating a sample containing TIL with a TIL seed cell medium to obtain a first TIL population, and (2) incubating the first TIL population with a TIL expansion medium to obtain a second TIL population.

In one or more embodiments, the sample is selected from the group consisting of ascites, surgically removed primary lesion samples, synchronous and metachronous surgically removed metastatic lesion samples, biopsy samples, and body fluids.

In one or more embodiments, the sample is a tissue containing tumor cells. Preferably, the tissue containing tumor cells is subjected to pretreatment, including fragmentation and/or dissociation of the tumor tissue. The tumor cells are derived from a tumor selected from the group consisting of: melanoma, glioma, thyroid tumor, gastric cancer, lung cancer, gastrointestinal stromal tumor, intestinal cancer, liver cancer, cervical cancer, ovarian cancer, breast cancer, endometrial stromal sarcoma, pelvic poorly differentiated adenocarcinoma, gallbladder cancer, bile duct cancer, head and neck cancer, colorectal cancer, glioblastoma, pancreatic cancer, bladder cancer, prostate cancer, renal cancer, and osteosarcoma. Preferably, the tissue containing tumor cells is a tumor tissue or body fluid from a cancer subject.

In one or more embodiments, the body fluid comprises blood, pleural fluid, interstitial fluid, lymphatic fluid, and/or ascites.

In one or more embodiments, the TIL seed cell medium described in step (1) comprises any one of the following combinations of components: 1) IL-2, IL-6, IL-21, IFN-γ, anti-TIGIT antibody, anti-PD-1 antibody, TNF-α, and basal medium; 2) IL-2, IL-4, IL-10, IL-21, anti-CD137 antibody, anti-LAG3 antibody, anti-PD-1 antibody, TNF-α, and basal medium; 3) IL-2, IL-7, IL-12, IL-21, anti-CD137 antibody, anti-CD28 antibody, anti-PD-1 antibody, and basal medium; 4) IL-1β, IL-2, IL-7, G-CSF, GM-CSF, IFN-γ, anti-LAG3 antibody, anti-PD-1 antibody, TNF-α, and basal medium; 5) IL-2, IL-4, IL-12, GM-CSF, M-CSF, IFN-β, IFN-γ, anti-TIGIT antibody, anti-CTLA-4 antibody, and basal medium; 6) IL-2, IL-7, IL-15, GM-CSF, anti-CD137 antibody, anti-PD-1 antibody, TNF-α, and basal medium; 7) IL-2, IL-4, IL-10, IL-15, G-CSF, M-CSF, anti-CD28 antibody, anti-OX-40 antibody, anti-PD-1 antibody, and basal medium; 8) IL-2, IL-7, IL-15, IFN-γ, anti-CD137 antibody, anti-CD40 antibody, anti-OX-40 antibody, anti-TIGIT antibody, anti-PD-1 antibody, and basal medium; 9) IL-2, IL-7, IL-15, GM-CSF, IFN-γ, anti-CD137 antibody, anti-CD28 antibody, anti-PD-1 antibody, TNF-α, and basal medium; 10) IL-2, IL-7, IL-12, G-CSF, GM-CSF, IFN-α, IFN-γ, anti-CD28 antibody, anti-CD40 antibody, anti-TIGIT antibody, anti-PD-1 antibody, TNF-α, and basal medium; 11) IL-2, IL-7, IL-15, GM-CSF, anti-PD-1 antibody, RRx-001, CAL-101, and basal medium; 12) IL-2, IL-7, IL-15, GM-CSF, M-CSF, anti-PD-1 antibody, CNI-1493, and basal medium; 13) IL-2, IL-7, IL-15, anti-CD137 antibody, anti-CD28 antibody, anti-LAG3 antibody, anti-PD-1 antibody, dasatinib, and basal medium; 14) IL-2, IL-6, IL-12, G-CSF, M-CSF, IFN-β, IFN-γ, anti-CTLA-4 antibody, anti-PD-1 antibody, dasatinib, LYC-55716, GENE-1858, and basal medium; 15) IL-1α, IL-2, IL-9, IL-15, GM-CSF, anti-CD137 antibody, anti-CD28 antibody, anti-LAG3 antibody, anti-TIGIT antibody, CNI-1493, and basal medium; 16) IL-2, IL-7, IL-12, IL-15, IL-21, G-CSF, M-CSF, IFN-γ, anti-CD28 antibody, anti-CD40 antibody, anti-LAG3 antibody, anti-PD-1 antibody, CNI-1493, dasatinib, GNE-1858, and basal medium. Preferably, the above combinations of components further include serum and/or antibiotics.

In one or more embodiments, the TIL expansion medium in step (2) comprises IL-2, IL-7, IL-15 and an immune checkpoint antibody or an antigen-binding fragment thereof, preferably, the immune checkpoint antibody comprises a PD-1 antibody.

In one or more embodiments, the TIL expansion medium further comprises platelets or platelets. The platelets are allogeneic platelets or subject's own platelets.

In one or more embodiments, the TIL expansion medium in step (2) comprises any one of the following combinations of components: 17) IL-2, IL-7, IL-15, anti-PD-1 antibody, IL-21, IL-12, anti-CD3 antibody, anti-CD28 antibody, and GM-CSF; 18) IL-2, IL-7, IL-15, anti-PD-1 antibody, IL-21, IL-12, anti-CD3 antibody, anti-CD28 antibody, anti-CD137 antibody, and anti-GITR antibody; 19) IL-2, IL-7, IL-15, anti-PD-1 antibody, anti-CD3 antibody, and anti-CD28 antibody, preferably, the anti-CD3 antibody and anti-CD28 antibody are coupled to a matrix; 20) IL-2, IL-7, IL-15, anti-PD-1 antibody, IL-21, IL-12, anti-CD3 antibody, anti-CD28 antibody, and GM-CSF, preferably, the anti-CD3 antibody and anti-CD28 antibody are coupled to a matrix; 21) IL-2, IL-7, IL-15, anti-PD-1 antibody, anti-CD3 antibody, anti-CD28 antibody, and anti-CD137 antibody, preferably, the anti-CD3 antibody, anti-CD137 antibody, and anti-CD28 antibody are coupled to a matrix; 22) IL-2, IL-7, IL-15, anti-PD-1 antibody, anti-GITR antibody, anti-CD3 antibody, anti-CD28 antibody, TWS119, and CD137 antibody, preferably, the anti-CD3 antibody, anti-CD137 antibody, and anti-CD28 antibody are coupled to a matrix; 23) IL-2, IL-7, IL-15, anti-PD-1 antibody, anti-TIGIT antibody, anti-CD3 antibody, anti-CD28 antibody, anti-CD137 antibody, and GM-CSF, preferably, the anti-CD3 antibody, anti-CD137 antibody, and anti-CD28 antibody are coupled to a matrix; 24) IL-2, IL-7, IL-15, anti-LAG3 antibody, IL-21, IL-12, anti-CD3 antibody, anti-CD28 antibody, anti-CD137 antibody, and anti-GITR antibody, preferably, the anti-CD3 antibody, anti-CD137 antibody, and anti-CD28 antibody are coupled to a matrix; 25) IL-2, IL-7, IL-15, anti-PD-1 antibody, anti-TIGIT antibody, anti-CD3 antibody, anti-CD28 antibody, anti-CD137 antibody, anti-CD40 antibody, anti-OX-40 antibody, and autologous platelets, preferably, the anti-CD3 antibody, anti-CD137 antibody, and anti-CD28 antibody are coupled to a matrix; 26) IL-2, IL-7, IL-15, anti-PD-1 antibody, anti-CTLA-4 antibody, anti-CD3 antibody, anti-CD28 antibody, anti-CD137 antibody, anti-GITR antibody, GM-CSF, TWS119, and allogeneic platelets, preferably, the anti-CD3 antibody and anti-CD28 antibody are coupled to a matrix.

In one or more embodiments, the TIL expansion medium further comprises serum and/or platelets, and a basal medium.

In one or more embodiments, the basal medium in the seed cell culture medium and the expansion medium is selected from any one or more of AIM-V, X-VIVO, DMEM, RPMI1640, OpTmizerTM, and FUJIFILM Irvin MHM-C.

In one or more embodiments, the serum is selected from human AB serum, subject autologous serum, or animal-derived serum.

The present invention further provides a method for preparing the modified TIL described in any embodiment herein, comprising introducing the encoding nucleic acid sequence of the membrane-anchored IL-7 into the TIL.

In one or more embodiments, prior to introduction, the TIL does not express the membrane-anchored IL-7.

In one or more embodiments, the TIL that does not express the membrane-anchored IL-7 is an unmodified TIL.

In one or more embodiments, the unmodified TIL is prepared by the method for preparing TIL described in any one of the first or second group of embodiments of the first aspect of the present invention.

In one or more embodiments, the method comprises: introducing into the TIL a nucleic acid construct expressing the membrane-anchored IL-7, such as an expression vector or mRNA. Preferably, the expression vector is a viral vector or a non-viral vector. Preferably, the expression vector is an integrative vector or a non-integrative vector. Preferably, the viral vector is a lentiviral vector.

In one or more embodiments, the non-viral vector is a vector based on a transposon system. Preferably, the non-viral vector is a vector based on a PiggyBac transposon system or a Sleeping Beauty transposon system. Preferably, the non-viral vector is a pKB20 or pNB328 vector.

In one or more embodiments, the expression vector comprises the nucleic acid sequence encoding the membrane-anchored IL-7 described in any embodiment of the first aspect of the present invention. Preferably, the expression vector is a pKB20 expression vector comprising the nucleic acid sequence encoding the membrane-anchored IL-7 described in any embodiment of the first aspect of the present invention.

In one or more embodiments, the method comprises introducing the nucleic acid construct into unmodified TIL by electroporation or liposome nanoparticles (LNP).

In one or more embodiments, the method comprises: 1) incubating a sample containing TIL with a TIL seed cell medium as described in any one of the second group of embodiments of the first aspect of the present invention to obtain a first TIL population; 2) introducing the nucleic acid construct expressing membrane-anchored IL-7 into the first TIL population by electroporation or liposome nanoparticles to obtain a second TIL population; and 3) incubating the second TIL population with a TIL expansion medium as described in any one of the second group of embodiments of the first aspect of the present invention to obtain a third TIL population. Other features of the method are as described in any one of the second group of embodiments of the first aspect of the present invention.

The present invention further provides a modified TIL prepared by the method for preparing a modified TIL described in any embodiment herein.

The present invention further provides use of the modified TIL described in any embodiment herein in the preparation of a medicament for preventing or treating tumors.

The present invention also provides a method for preventing or treating tumors, comprising administering to a subject in need thereof the modified TIL described in any one of the embodiments herein.

In one or more embodiments, the tumor is selected from one or more of the following: melanoma, glioma, thyroid tumor, gastric cancer, lung cancer, gastrointestinal stromal tumor, intestinal cancer, liver cancer, cervical cancer, ovarian cancer, breast cancer, endometrial stromal sarcoma, pelvic poorly differentiated adenocarcinoma, gallbladder cancer, bile duct cancer, head and neck cancer, colorectal cancer, glioblastoma, pancreatic cancer, bladder cancer, prostate cancer, kidney cancer and osteosarcoma.

The present invention further provides a polypeptide, comprising, sequentially linked: an optional signal peptide, IL-7, an optional linker, the extracellular domain of BCMA or a variant thereof, an optional hinge region or linker, and a transmembrane domain, wherein the signal peptide is a CD52 signal peptide or an IL-7 signal peptide, and the transmembrane domain is a CD8 transmembrane domain or CD52.

In one or more embodiments, the polypeptide comprises, sequentially linked:

a CD52 signal peptide, IL-7, a linker, the extracellular domain of BCMA or a variant thereof, and a CD8 transmembrane domain;

a CD52 signal peptide, IL-7, a linker, the extracellular domain of BCMA or a variant thereof, hinge region or linker, and CD52;

a CD52 signal peptide, IL-7, the extracellular domain of BCMA or a variant thereof, hinge region or linker, and CD52;

an IL-7 signal peptide, IL-7, a linker, the extracellular domain of BCMA or a variant thereof, and a CD8 transmembrane domain;

an IL-7 signal peptide, IL-7, a linker, the extracellular domain of BCMA or a variant thereof, and CD52.

Advantages of the present invention:

In the present invention, anchoring of the cytokine IL-7 on the surface of TIL is achieved through GPI anchor protein, which can effectively activate and proliferate TIL, significantly improve its tumor cytotoxicity, and compared with the fixation through the conventional amino acid transmembrane domain, the expression intensity of IL-7 on the membrane surface anchored by GPI has been significantly improved. Meanwhile, the present invention introduces the wild type or mutant sequence of BCMA, a small molecular weight protein not present on the surface of T cells, as a detection and sorting label in the structure of membrane anchored IL-7. In addition to effectively enabling the detection and sorting of TIL positive for membrane-anchored IL-7 expression, in the presence of antibodies, it can also effectively mediate ADCC and CDC effects *in vivo,* exert a molecular brake effect through prompt killing TILs expressing membrane-anchored IL-7 that contain the extracellular domain of BCMA, thus greatly improving the safety of clinical application of TILs expressing membrane-anchored IL-7.

## Description of drawings

Figure 1. RTCA killing curve of T002-TIL and T002-2 cells on homologous melanoma cells;

Figure 2. Tumor volume change curves of *In vivo* cytotoxic effect of T014-9 cells on SKOV-3 320 CDX mouse model;

Figure 3. Fluorescent *In vivo* imaging results of *In vivo* cytotoxic effect of T014-9 cells on SKOV-3 320 CDX mouse model;

Figure 4. Tumor volume change curves of *In vivo* cytotoxic effect of T011-TIL and T011-9 cells on patient-matched PDX mouse model;

Figure 5. Imaging evaluation results of tumor lesions in Patient T011 before and after TIL infusion therapy;

Figure 6. Imaging evaluation results of tumor lesions in Patient T012 before and after TIL infusion therapy;

Figure 7. Imaging evaluation results of tumor lesions in Patient T013 before and after TIL infusion therapy;

Figure 8. Imaging evaluation results of tumor lesions in Patient T014 before and after TIL infusion therapy.

## DETAILED DESCRIPTION

The inventors found that by anchoring the cytokine IL-7 and BCMA (B-cell maturation antigen) moiety on the surface of TIL, it is possible to effectively activate and expand TILs, detect and sort TILs expressing membrane-anchored IL-7, and efficiently mediate the molecular brake effect.

## Definition of terms

In the present application, the term "interferon" generally refers to a family of proteins with high species specificity that inhibit viral replication and cell proliferation and regulate immune responses. Typical suitable interferons include, but are not limited to: recombinant interferon $\alpha$-2b, recombinant interferon $\alpha$-2a, recombinant interferon $\alpha$-2C, interferon $\alpha$-n1, consensus interferon $\alpha$, or interferon $\alpha$-n3.

In this application, the term "colony stimulating factor" generally refers to a class of secreted glycoproteins that bind to receptor proteins on the surface of hematopoietic stem cells, thereby activating intracellular signal transduction pathways, which can lead to cell proliferation and differentiation into specific types of blood cells (usually white blood cells, for red blood cell formation, see erythropoietin). They can be artificially synthesized and administered exogenously.

In this application, the term "antigen binding fragment" generally refers to a portion of an antibody molecule that contains amino acids responsible for the specific binding between the antibody and the antigen. The portion of the antigen that is specifically recognized and bound by the antibody is called an "epitope" as described above. As described above, an antigen binding domain may typically comprise an antibody light chain variable region (VL) and an antibody heavy chain variable region (VH); however, it does not necessarily comprise both. A Fd fragment, for example, has two VH regions and generally retains some antigen binding functions of a complete antigen binding domain. Examples of antigen-binding fragments of antibodies include (1) a Fab fragment, a monovalent fragment having VL, VH, a constant light chain (CL), and a CH1 domain; (2) a F(ab')2 fragment, a bivalent fragment having two Fab fragments connected by a disulfide bridge at the hinge region; (3) a Fd fragment having two VH and CH1 domains; (4) a Fv fragment having the VL and VH domains of a single arm of an antibody, (5) a dAb fragment (Ward et al., "Binding Activities of a Repertoire of Single Immunoglobulin Variable Domains Secreted From Escherichia coli," Nature 341:544-546 (1989), which is incorporated herein by reference in its entirety), which has a VH domain; (6) isolated complementarity determining regions (CDR), and (7) single-chain Fv (scFv), for example derived from a scFV-library. Although the two domains VL and VH of the Fv fragment are encoded by independent genes, they can be joined using recombinant methods through synthetic linkers, which enable them to be prepared as a single protein chain (called single-chain Fv (scFv)) in which the VL and VH regions are paired to form a monovalent molecule (see, for example, Huston et al., "Protein Engineering of Antibody Binding Sites: Recovery of Specific Activity in an Anti-Digoxin Single-Chain Fv Analogue Produced in Escherichia coli," Proc. Natl. Acad. Sci. USA 85: 5879-5883 (1988)). These antibody fragments are obtained using conventional techniques known to those skilled in the art, and the functions of the fragments are evaluated in the same manner as intact antibodies. In this application, the term "antigen binding fragment" also includes ligands that specifically bind to antigens, particularly cell surface receptor molecules. Ligands are generally polypeptides or compounds that can bind to receptor proteins (such as the antigen) with high affinity and specificity to induce a functional response. For example, the ligand may include CD40L, a ligand of CD40, CD137L, a ligand of CD137, CD80, a ligand of CD28, and/or OX-40L, a ligand of OX-40.

In this application, the term "immune checkpoint" generally refers to a group of molecules on the cell surface of CD4+ and CD8+ T cells. These molecules can be effectively used as "brakes" to down-regulate or inhibit anti-tumor immune responses. These inhibitory molecules can be inhibited by inhibiting DNA, RNA or protein levels.

In the present application, the term "activating antibody" generally refers to the antibody targeting surface receptors of immune cells such as T cells, such as TCR and/or co-stimulatory receptors. These antibodies can activate the activation signal pathways downstream of these receptors by binding to TCR and/or co-stimulatory receptors, thereby significantly enhancing the activation and proliferation levels of the immune cells.

In this application, the term "costimulatory receptor" generally refers to a type of receptors expressed on the surface of immune cells such as T cells, which are required for activating immune responses. In the presence of antigen-presenting cells and when the primary signal has been provided, costimulatory receptors trigger the costimulatory signal by binding to their corresponding activating antibodies, ligands, or antigen-binding fragments, thereby fully activating immune effector cells. The activation of signaling pathways mediated by costimulatory receptors usually plays a key role in the effective occurrence of immune responses. Common costimulatory receptors include but are not limited to CD28, CD40, CD137, and OX-40.

In this application, the term "tumor necrosis factor TNF" generally refers to TNF protein, and is not a superfamily of TNF ligands. The Genbank accession number of human TNF$\alpha$ is NP_000585.2. The term "TNF$\alpha$" encompasses precursor forms of TNF$\alpha$, pro-TNF$\alpha$, full-length TNF$\alpha$, and any form of TNF$\alpha$ produced by intracellular processes. The term also encompasses naturally occurring and non-naturally occurring variants of TNF$\alpha$, such as splice variants, allelic variants, and isoforms. TNF$\alpha$ can bind to two receptors, TNFR1 (TNF receptor type 1; CD120a; p55/60) and TNFR2 (TNF receptor type 2; CD120b; p75/80). TNF$\alpha$ acts as a proinflammatory cytokine, for example, in neuroinflammation. TNF$\alpha$ functions as a proinflammatory cytokine and plays a role, for example, in neuroinflammation.

In the present application, the term "tumor infiltrating lymphocytes" generally refers to infiltrating lymphocytes separated from tumor tissue. Immunohistochemical staining shows that tumor infiltrating lymphocytes may include CD3+ T cells, CD20+, CD79$\alpha$+ B cells, plasma cells and CD56+ NK cells; they may also include T cells, a small amount of B cells, NK cells, macrophages and dendritic cells. The tumor infiltrating lymphocytes can produce highly efficient and specific anti-tumor cytotoxic effects through direct cytotoxic T lymphocytes and secreted cytokines. In the present application, the tumor infiltrating lymphocytes may include mononuclear leukocytes that leave the bloodstream and migrate to a tumor. The tumor infiltrating lymphocytes may be selected from the group consisting of T cells, B cells, natural killer cells, and natural killer T cells.

In the present application, the term "seed cells" generally refers to tumor infiltrating lymphocytes cultured from tumor tissue using any suitable medium including the medium of the present application. These cells can be used as a starting cell population for further expansion of the culture, or as a cell population for terminal use, such as a cell population for reinfusion therapy of an individual subject. The seed cells can be obtained from tumor tissue after any different culture durations, and the culture duration of the seed cells can be determined based on the subsequent use of the seed cells, and is not limited to a specific culture duration.

In this application, the term "IL-15" generally refers to a proinflammatory cytokine that acts as a potent growth, survival, and activation factor for T cells, particularly intestinal intraepithelial lymphocyte (IEL) and natural killer (NK) cells, and may also be referred to as "IL-15 antigen" and "interleukin 15". Increased expression of IL-15 has been demonstrated in a variety of inflammatory diseases, including CD, rheumatoid arthritis (RA), and psoriasis (Malamut et al., 2010). IL-15 is regarded as a central regulator of CD immunopathology and a non-redundant driver of lymphomagenesis in RCD.

In this application, the term "IL-2" generally refers to a protein derived from a lymphokine produced by normal peripheral blood lymphocytes and present in the body at low concentrations. Initially, Morgan et al. (1976) Science 193: 1007-1008 described IL-2, initially called T cell growth factor, because it can induce the proliferation of stimulated T lymphocytes. The term also includes proteins modified after the expression of IL-2, such as glycosylation, acetylation, phosphorylation, etc.

In this application, the term "IL-7" generally refers to a protein encoded by the IL-7 gene. IL-7 is a hematopoietic growth factor secreted by stromal cells in the bone marrow and thymus. It can be produced by keratinocytes, dendritic cells, hepatocytes, neurons and epithelial cells. IL-7 can be involved in the effects on T cells and B cells.

In this application, the term "PD-1" generally refers to an immunoinhibitory receptor belonging to the CD28 family. PD-1 is primarily expressed *In vivo* on previously activated T cells and binds two ligands, PD-1 and PD-2. The complete hPD-1 sequence can be found in GenBank Accession No. U64863.

In the present application, the term "LYC-55716" generally refers to a ROR γ agonist. Its CAS number is 2055536-64-4.

In the present application, the term "GNE-1858" generally refers to a HPK1 inhibitor, with a CAS number of T11438.

In the present application, the term "TWS119" generally refers to a GSK-3 inhibitor, with a CAS number of 601514-19-6.

In the present application, the term "RRX-001" generally refers to an epigenetic modulator with radiosensitizing activity. This RRX-001 can downregulate the CD47/SIRPα axis and repolarize TAMs (tumor-associated macrophages) and other immunosuppressive cells in the tumor microenvironment into an immunostimulatory phenotype. Its CAS number is 925206-65-1.

In this application, the term "CNI-1493" generally refers to a compound that can block the production of inflammatory cytokines (including TNF), and its CAS number is 164301-51-3.

In this application, the term "anchoring" refers to fixing the part of interest to a substance through intermolecular interactions (chemical bonds, van der Waals forces and hydrogen bonds). Membrane-anchored cytokines refer to cytokines that are fixed to the cell membrane.

Herein, the term "inducing TIL" refers to the process of isolating TILs from animal tumors. After the TILs are expanded to a certain number *in vitro,* they can be infused back into the animal body to achieve the effect of controlling tumor growth and metastasis.

The present invention is suitable for isolating TIL from any tumor tissue, including tumor tissue obtained by open surgery or minimally invasive surgery. Exemplary tumor tissues include tumor tissues of gastric cancer, thyroid tumors, gallbladder cancer, bile duct cancer, lung cancer, melanoma, head and neck cancer, breast cancer, ovarian cancer, cervical cancer, liver cancer, colorectal cancer, glioblastoma, pancreatic cancer, bladder cancer, prostate cancer, kidney cancer, osteosarcoma and other cancers.

Detailed description of the invention

The present invention first provides a modified TIL that expresses membrane-anchored IL-7. Membrane-anchored IL-7 is a polypeptide comprising one or more IL-7 or functional fragments thereof, and a transmembrane domain, and optionally further comprising a membrane surface tag. The sequence of IL-7 is shown in SEQ ID NO: 7. The functional fragment of IL-7 has the usual meaning in the art, and the sequence is known to those skilled in the art.

Although the inventors have found that the expression intensity of GPI-anchored membrane surface IL-7 is significantly improved, certain effects can also be achieved using a conventional protein transmembrane domain. The transmembrane domain can be the transmembrane domain of the membrane surface tag, or the transmembrane domain of other transmembrane proteins, as long as it can anchor the membrane-anchored IL-7 to the cell membrane surface and has no negative impacts on its activity and function. Therefore, those skilled in the art can select a suitable transmembrane domain as needed (e.g., according to the structure or composition of the membrane). Transmembrane domains include, but are not limited to, any one of the transmembrane domains selected from the group consisting of CD28, CD8, CD134 (OX40), CD137 (4-1BB), LCK, ICOS, DAP10, siglec-9, siglec-10, siglec-15, TLR1, TLR2, TLR3, TLR4, TLR5, TLR6, TLR7, TLR8, TLR9, IL-2Rβ, IL-2Rγ, IL-4Rα, IL-7Rα, IL-10R, IL-12R, IL-15R, IL-21R, CD27, and CD40. Exemplary transmembrane domains include CD28 transmembrane domain, CD8 transmembrane domain, and IL-7R transmembrane domain. The transmembrane domain is connected to its adjacent structure (e.g., a membrane surface tag) via a hinge region or a linker. Therefore, the polypeptide can be a membrane surface protein or a transmembrane protein.

Of course, the transmembrane domain is preferably a GPI anchor domain, which may include one or more membrane anchor regions of GPI membrane anchor proteins selected from the group consisting of:CD44, CD56, CD73, CD55, Thy1, AchE, IAP, ALPP, CD59, CD14, CD16, CD24, CD28, CD48, CD52, CD58, CD66a, CD66c, CD66d, CD66e, CD67, CD87, CD108, CD157, uPAR, JMH protein, GDNFR, CNTFR, TAG-1, PrP, phosphatidylinositol protein, semaphorin 7, CEA, GFR, Ly6G, transferrin receptor, contactin (F3) and T-cadherin. Preferably, the GPI anchor region is selected from one or more membrane anchor regions of GPI membrane anchor proteins selected from the group consisting of: CD52, CD48, CD55, ALPP, CD90. The amino acid sequence of the GPI anchor region of CD52 is shown in SEQ ID NO:29, and its exemplary coding sequence is shown in SEQ ID NO:30.

The membrane surface tag is located at the N-terminus or C-terminus of the IL-7. Therefore, the membrane surface cytokine may have the structure of: membrane surface tag-IL-7-transmembrane domain, or IL-7-membrane surface tag-transmembrane domain. In the embodiment of multiple IL-7s, the membrane surface tag may be located at the N-terminus or C-terminus of any IL-7. For example, in the case of two IL-7s, the membrane surface cytokine may have the structure of: membrane surface tag-first IL-7-second IL-7-transmembrane domain, or first IL-7-membrane surface tag-second IL-7-transmembrane domain, or first IL-7-second IL-7-membrane surface tag-transmembrane domain, and so on. Preferably, the IL-7 is located at the N-terminus of the membrane surface tag.

In the embodiment of multiple membrane surface tags, the multiple membrane surface tags can be connected in series or dispersed at any position outside the membrane surface cytokine (such as the N-terminus and C-terminus of any IL-7), as long as they are all located at the N-terminus of the transmembrane domain and do not affect the functions of the membrane surface tags and the membrane surface cytokines.

The N-terminus of the transmembrane domain (e.g., between the membrane surface tag and the transmembrane domain) may have a hinge region or a linker, or may have any sequence that does not affect the function of the membrane surface cytokine, such as a signal peptide, or other polypeptides that need to be expressed on the membrane surface. Hinge regions include, but are not limited to: CD4 extracellular hinge region, CD8 extracellular hinge region, CD28 extracellular hinge region, IgG1Fc hinge region, and IgG4Fc hinge region. Preferably, the hinge region is a CD8 extracellular hinge region. Preferably, the sequence of the CD8 extracellular hinge region is shown in SEQ ID NO: 19; its exemplary coding sequence is shown in SEQ ID NO: 20. The linker is a rigid linker or a flexible linker, and its sequence is shown in SEQ ID NO: 9, 11, 13, 15, 17; its exemplary coding sequence is shown in SEQ ID NO: 10, 12, 14, 16, or 18, respectively. Preferably, the sequence of the rigid linker is any one selected from SEQ ID NO: 9, 11, 15, 17.

In the embodiment containing the membrane surface tag, a linker may be positioned between IL-7 and the membrane surface tag. In one or more embodiments, the linker is a rigid linker or a flexible linker, preferably a rigid linker. Preferably, the sequence of the rigid linker is selected from any one of SEQ ID NO: 9, 11, 15, 17.

In certain embodiments, a linker may be positioned between IL-7 and the membrane surface tag and between the membrane surface tag and the transmembrane domain.

Herein, the membrane surface tag (or membrane surface tag) comprises the BCMA extracellular domain or a variant thereof that retains the function of binding to an anti-BCMA antibody. The BCMA extracellular domain is shown in SEQ ID NO: 21; its exemplary coding sequence is shown in SEQ ID NO: 22. The variant may be a truncated variant, an insertion variant, a deletion variant, a substitution variant, or a combination thereof that retains the function of binding to an anti-BCMA antibody. The sequence of the anti-BCMA antibody is not limited and may be any anti-BCMA antibody known to those skilled in the art. Exemplarily: the truncated variant of the BCMA extracellular domain is shown in SEQ ID NO: 23, and its exemplary coding sequence is shown in SEQ ID NO: 24; the substitution variant of the BCMA extracellular domain is shown in SEQ ID NO: 25, and its exemplary coding sequence is shown in SEQ ID NO: 26; the truncated substitution variant of the BCMA extracellular domain is shown in SEQ ID NO: 27, and its exemplary coding sequence is shown in SEQ ID NO: 28.

The membrane-anchored IL-7 may further comprise a signal peptide. Those skilled in the art can select a suitable signal peptide according to the desired expression form. The signal peptide may be cleaved off during the secretion of the polypeptide. Exemplarily, the signal peptide is located at the N-terminus of the membrane-anchored IL-7. Preferably, the signal peptide is a CD52 signal peptide or an IL-7 signal peptide. Preferably, the CD52 signal peptide is shown in SEQ ID NO: 1; its exemplary coding sequence is shown in SEQ ID NO: 2. Preferably, the IL-7 signal peptide is shown in SEQ ID NO: 5; its exemplary coding sequence is shown in SEQ ID NO: 6.

In some embodiments, the present invention provides a polypeptide or a TIL expressing the polypeptide, wherein the polypeptide is a membrane-anchored IL-7, comprising sequentially linked: an optional signal peptide, IL-7, an optional linker, a BCMA extracellular domain or a variant thereof, an optional hinge region or linker, and a transmembrane domain, wherein the signal peptide is a CD52 signal peptide or an IL-7 signal peptide, and the transmembrane domain is a CD8 transmembrane domain or CD52. In some preferred embodiments, the membrane-anchored IL-7 comprises sequentially linked: a signal peptide, IL-7, a linker, a BCMA extracellular domain or a variant thereof, and a transmembrane domain. In some preferred embodiments, the membrane-anchored IL-7 comprises the following: (1) a CD52 signal peptide, IL-7, a linker, a BCMA extracellular domain or a variant thereof, and a CD8 transmembrane domain, (2) a CD52 signal peptide, IL-7, a linker, a BCMA extracellular domain or a variant thereof, and CD52, (3) a CD52 signal peptide, IL-7, a linker, a BCMA extracellular domain or a variant thereof, a hinge region or a linker, and CD52, (4) a CD52 signal peptide, IL-7, a BCMA extracellular domain or a variant thereof, a hinge region or a linker, and CD52, (5) an IL-7 signal peptide, IL-7, a linker, a BCMA extracellular domain or a variant thereof, and a CD8 transmembrane domain, or (6) an IL-7 signal peptide, IL-7, a linker, a BCMA extracellular domain or a variant thereof, and CD52. In an exemplary embodiment, the amino acid sequence of the membrane-anchored IL-7 is any one or more selected from SEQ ID NO: 31, 33, 35, 37, 39, 41, 43 and 45. Its exemplary encoding nucleic acid sequence is any one or more selected from SEQ ID NO: 32, 34, 36, 38, 40, 42, 44 and 46.

The present invention further includes fragments, derivatives and analogs of the above-mentioned polypeptides. As used herein, the terms "fragment", "derivative" and "analog" refer to polypeptides that substantially retain the same biological function or activity as the polypeptide. A fragment, derivative or analog of a polypeptide or domain may be (i) a polypeptide or domain in which one or more conservative or non-conservative amino acid residues (preferably conservative amino acid residues) are substituted, and such substituted amino acid residues may or may not be encoded by the genetic code, or (ii) a polypeptide or domain having a substitution group in one or more amino acid residues, or (iii) a polypeptide formed by fusion of a mature polypeptide or domain with another compound (such as a compound that prolongs the half-life of the polypeptide, such as polyethylene glycol), or (iv) a polypeptide formed by fusion of an additional amino acid sequence to the polypeptide or domain sequence (such as a leader sequence or secretory sequence or a sequence or proprotein sequence used to purify the polypeptide, or a fusion protein formed with an antigen IgG fragment). According to the teachings herein, these fragments, derivatives and analogs belong to the well-known scope of those skilled in the art.

The term "variant" or "mutant" refers to a peptide or polypeptide that, compared to a reference sequence, has an altered amino acid sequence through insertion, deletion, or substitution of one or more amino acids while retaining at least one biological activity. Mutants described in any embodiment herein include those having at least 70%, preferably at least 80%, more preferably at least 85%, more preferably at least 90%, more preferably at least 95%, and most preferably at least 97% sequence identity to a reference sequence (such as the amino acid sequences encoded by SEQ ID NOs: 2, 4, 56, 58, 60, 62, 64, 66, 68, 70, 72, 74, 76, 78, 80, 82, 84, 86, 88, 90, 92, 94, 96, 98, 100, 12, or 14 described herein) and retaining the biological activity of the reference sequence (e.g., as a chimeric antigen receptor, surface cytokine-expressing protein, or signal-transducing receptor). Sequence identity between two aligned sequences can be calculated using, for example, NCBI's BLASTp. Mutants also include amino acid sequences having one or more mutations (insertions, deletions, or substitutions) in the amino acid sequence of the reference sequence while still retaining the biological activity of the reference sequence. The term "multiple mutations" typically refers to 1 to 10 mutations, such as 1 to 8, 1 to 5, or 1 to 3 mutations. Substitutions are preferably conservative substitutions. For example, in the art, conservative substitutions using amino acids with similar or comparable properties generally do not alter the function of a protein or polypeptide. "Amino acids with similar or comparable properties" include, for example, families of amino acid residues with similar side chains, including amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), β-branched side chains (e.g., threonine, valine, isoleucine), and aromatic side chains (e.g., tyrosine, phenylalanine, tryptophan, histidine). Therefore, replacement of one or several amino acid residues in a polypeptide of the present invention with another residue from the same side chain family will not substantially affect its activity.

Truncated variants of the polypeptide or domain can also be used in the present invention, as long as the variant retains substantially the same biological function or activity as the polypeptide or domain, such as a truncated variant of the BCMA extracellular domain that retains the antigen-antibody reactivity of the BCMA extracellular domain with an antibody.

The polypeptides described herein may be modified polypeptides. Modifications (usually without changing the primary structure) include: chemical derivatization of polypeptides *in vivo* or *in vitro* such as acetylation or carboxylation. Modifications also include glycosylation, such as those produced by glycosylation modification during the synthesis and processing of the polypeptide or in further processing steps. This modification can be accomplished by exposing the polypeptide to a glycosylation enzyme (such as a mammalian glycosylase or deglycosylation enzyme). Modified forms also include sequences with phosphorylated amino acid residues (such as phosphotyrosine, phosphoserine, phosphothreonine). Also included are polypeptides that have been modified to improve their anti-proteolytic properties or optimize their solubility properties.

Nucleic acid molecules encoding the polypeptide or domain are also within the scope of this invention. Nucleic acid molecules can be in the form of DNA or RNA. The nucleic acid molecules described herein include sequences altered by codon optimization, as long as the amino acid sequences encoded by the nucleic acid molecules remain unchanged. Sequences optimized through codon optimization can show more suitable expressivity for specific species. Methods for codon optimization of nucleic acid molecule sequences are well known in the art. The coding region sequence encoding the mature polypeptide can be a degenerate variant. As used herein, "degenerate variant" refers to a nucleic acid sequence that encodes an amino acid sequence or a domain fragment thereof encoded by the nucleic acid sequence shown in the Sequence Listing, but is different from the corresponding nucleic acid sequence.

Nucleic acid molecules encoding mature polypeptides include: coding sequences encoding mature polypeptides only; coding sequences of mature polypeptides and various additional coding sequences; coding sequences of mature polypeptides (and optional additional coding sequences) and non-coding sequences. The term "nucleic acid molecules encoding polypeptides" may include nucleic acid molecules encoding the polypeptides, or may include nucleic acid molecules further including additional coding and/or non-coding sequences.

The nucleic acid molecule of the present invention may be a coding sequence for a polypeptide or a domain, or an expression frame for a polypeptide or a domain. Herein, a coding sequence refers to a portion of a nucleic acid sequence that directly determines the amino acid sequence of its protein product (e.g., CAR, single-chain antibody, hinge region, transmembrane domain, intracellular signal region, or cytokine protein). The boundaries of the coding sequence are usually determined by the ribosome binding site (for prokaryotic cells) immediately upstream of the open reading frame at the 5' end of the mRNA and the transcription termination sequence immediately downstream of the open reading frame at the 3' end of the mRNA. The coding sequence may include, but is not limited to, DNA, cDNA, and recombinant nucleic acid sequences. Herein, an expression frame refers to the complete elements required to express a gene of interest, including a promoter, a gene coding sequence, and a PolyA tailing signal sequence. In certain embodiments, the coding sequence or expression frame is integrated into the genome of the cell. Therefore, in these embodiments, the genome of the cell described herein is stably integrated with an expression frame encoding a polypeptide described herein.

The present invention also relates to variants of the above-mentioned nucleic acid molecules, which encode polypeptides or fragments, analogs and derivatives of polypeptides having the same amino acid sequence as the present invention. The variants of this polynucleotide can be naturally occurring allelic variants or non-naturally occurring variants. These nucleotide variants include substitution variants, deletion variants and insertion variants. As known in the art, an allelic variant is an alternative form of a polynucleotide, which may be a substitution, deletion or insertion of one or more nucleotides, but will not substantially change the function of the polypeptide encoded by it.

The present invention also relates to polynucleotides that hybridize with the aforementioned sequences and have at least 50%, preferably at least 70%, more preferably at least 80%, 85%, 90%, or 95% identity with the sequences. In particular, the present invention relates to polynucleotides that can hybridize with the polynucleotides of the present invention under stringent conditions. In the present invention, "stringent conditions" refer to: (1) hybridization and elution under conditions of low ionic strength and high temperature, such as $0.2 \times SSC$, 0.1% SDS at 60°C; or (2) hybridization in the presence of denaturants, such as 50% (v/v) formamide, 0.1% calf serum/0.1% Ficoll at 42°C, etc.; or (3) hybridization occurs only when the identity between the two sequences is at least 90%, more preferably 95% or higher.

The present invention also relates to complementary sequences of the aforementioned sequences or nucleic acid fragments that hybridize with the aforementioned sequences. As used herein, a "nucleic acid fragment" is at least 15 nucleotides in length, preferably at least 30 nucleotides, more preferably at least 50 nucleotides, and most preferably at least 100 nucleotides or longer. Nucleic acid fragments can be used in nucleic acid amplification techniques (such as PCR) to identify and/or isolate polynucleotides encoding the desired polypeptides or domains. "Hybridization" as used herein primarily refers to the pairing of nucleic acid sequences under stringent conditions. Exemplary stringent conditions include hybridization and membrane washing in a solution of $0.1 \times SSPE$ (or $0.1 \times SSC$) and 0.1% SDS at 65°C.

In certain embodiments, the nucleic acid molecule is a nucleic acid construct, which contains the coding sequence of the polypeptide described herein and one or more regulatory sequences operably linked to these sequences. The polynucleotides of the present invention can be manipulated in various ways to ensure the expression of the polypeptide. Before inserting the nucleic acid construct into a vector, the construct may be manipulated according to the type of expression vector or requirements. Techniques for altering polynucleotide sequences using recombinant DNA methods are known in the art.

The regulatory sequence can be a suitable promoter sequence. A promoter sequence is usually operably linked to the coding sequence of the protein to be expressed. The promoter can be any nucleotide sequence that exhibits transcriptional activity in the selected host cell, including mutated, truncated, and hybrid promoters, and can be derived from genes encoding extracellular or intracellular polypeptides homologous or heterologous to the host cell. The regulatory sequence can also be a suitable transcription terminator sequence, a sequence recognized by the host cell to terminate transcription. The terminator sequence is operably linked to the 3' end of the nucleotide sequence encoding the polypeptide. Any terminator functional in the selected host cell can be used herein. The regulatory sequence can also be a suitable leader sequence, a non-translated region of mRNA important for translation in the host cell. The leader sequence is operably linked to the 5' end of the nucleotide sequence encoding the polypeptide. Any leader sequence functional in the selected host cell can be used in the present invention.

In certain embodiments, the nucleic acid construct is a vector. The vector can be a cloning vector, an expression vector, or a homologous recombination vector. Specifically, the coding sequence of the polypeptide herein can be cloned into many types of vectors, including but not limited to plasmids, phagemids, phage derivatives, animal viruses, and cosmids. Cloning vectors can be used to provide the coding sequence of the polypeptide of the present invention.

Expression vectors can be provided to cells in vector form. Expression of the polynucleotide of the present invention is typically achieved by operably linking the coding sequence of the present invention to a promoter and incorporating the construct into an expression vector. The vector can be suitable for replication and integration in eukaryotic cells. Typical cloning vectors contain transcriptional and translational terminators, initiation sequences, and promoters that can be used to regulate the expression of the desired nucleic acid sequence. The vector can be a virus; viruses usable as vectors include but are not limited to retroviruses, adenoviruses, adeno-associated viruses, herpes viruses, and lentiviruses. Vector technology is well known in the art and is described in, for example, Sambrook et al. (2001, *Molecular Cloning: A Laboratory Manual*, Cold Spring Harbor Laboratory, New York) and other virology and molecular biology handbooks. Homologous recombination vectors are used to integrate the expression cassettes described herein into the host genome.

Typically, a suitable vector contains an origin of replication functional in at least one organism, a promoter sequence, convenient restriction enzyme sites, and one or more selectable markers.

Suitable promoters include but are not limited to the immediate early cytomegalovirus (CMV) promoter sequence, elongation factor-1$\alpha$ (EF-1$\alpha$), simian virus 40 (SV40) early promoter, mouse mammary tumor virus (MMTV) promoter, human immunodeficiency virus (HIV) long terminal repeat (LTR) promoter, MoMuLV promoter, avian leukosis virus promoter, Epstein-Barr virus immediate early promoter, Rous sarcoma virus promoter, and human gene promoters such as, but not limited to, actin promoter, myosin promoter, heme promoter, and creatine kinase promoter.

To evaluate the expression of the polypeptide, the expression vector introduced into cells may also contain a selectable marker gene to facilitate the identification and selection of expressing cells from a population of cells sought to be transfected or infected by the viral vector. The selectable marker can be carried on a separate segment of DNA and used in co-transfection procedures. Both sides of the selectable marker can be flanked by appropriate regulatory sequences to enable expression in the host cell. Useful selectable markers include, for example, antibiotic resistance genes such as neo, etc.

The polynucleotides described herein can generally be obtained by PCR amplification. Specifically, primers can be designed based on the nucleotide sequences disclosed herein, especially the open reading frame sequences, and the relevant sequences can be amplified using a commercially available cDNA library or a cDNA library prepared by conventional methods known to those skilled in the art as a template. When the sequence is long, it is often necessary to perform two or more rounds of PCR amplification, and then splice the amplified fragments in the correct order. Alternatively, the nucleic acid molecules described herein can be directly synthesized.

Methods for introducing nucleic acid molecules into cells and expressing genes in cells are known in the art. Nucleic acid molecules such as vectors or mRNA can be easily introduced into host cells, such as mammalian, bacterial, yeast, or insect cells, by any method in the art. For example, expression vectors can be transferred into host cells by physical, chemical, or biological means. Physical methods include calcium phosphate precipitation, lipid nanoparticle transfection, particle bombardment, microinjection, electroporation, etc. Biological methods include the use of DNA and RNA vectors. Chemical means include colloidal dispersion systems such as macromolecular complexes, nanocapsules, microspheres, beads; and lipid-based systems including oil-in-water emulsions, micelles, mixed micelles, and liposomes.

As described herein, the host cell expresses the polypeptide described herein or contains the nucleic acid molecule described herein. Host cells include both final product cells (e.g., TILs) and various cells used in the process of producing the product cells, such as E. coli cells, for purposes such as providing the coding sequence of the protein of the present invention or providing the vectors described herein. In certain embodiments, provided herein is a TIL that stably expresses the polypeptide described herein (membrane-anchored IL-7). Typically, prior to the introduction of membrane-anchored IL-7, the TIL does not express membrane-anchored IL-7, or the TIL is an unmodified TIL.

TILs suitable for the present invention can be various types from various sources, for example, derived from tissues containing tumor cells. Typically, the tumor cell-containing tissue is pretreated, including fragmentation and/or dissociation of the tumor tissue. The diameter of the fragmented and/or dissociated tumor tissue is approximately 1 mm to 10 mm, for example, approximately 2 mm to 10 mm, approximately 3 mm to 10 mm, approximately 4 mm to 10 mm, approximately 5 mm to 10 mm, approximately 6 mm to 10 mm, approximately 7 mm to 10 mm, or approximately 8 mm to 10 mm. The tumor cells are derived from tumors selected from the group consisting of: melanoma, glioma, thyroid tumor, gastric cancer, lung cancer, gastrointestinal stromal tumor, intestinal cancer, liver cancer, cervical cancer, ovarian cancer, breast cancer, endometrial stromal sarcoma, pelvic poorly differentiated adenocarcinoma, gallbladder cancer, cholangiocarcinoma, head and neck cancer, colorectal cancer, glioblastoma, pancreatic cancer, bladder cancer, prostate cancer, renal cancer, and osteosarcoma. Preferably, the tumor cell-containing tissue is a tumor tissue or body fluid from a cancer subject. The body fluids include blood, pleural effusion, interstitial fluid, lymphatic fluid, and/or ascites. For example, the body fluids may include peritoneal effusion (e.g., peritoneal effusion from a gastric cancer patient, or peritoneal effusion from a breast cancer patient) and/or pleural effusion (e.g., pleural effusion from a patient with endometrial stromal sarcoma).

The preparation method of TIL is not limited. In a preferred embodiment, TIL is obtained using the method described in PCT/CN2021/133059, which is incorporated herein by reference in its entirety. In an exemplary embodiment, the TIL is prepared by the following method: a sample containing TIL is incubated with a TIL seed cell medium to obtain a TIL population.

The TIL seed cell medium herein includes any TIL seed medium known in the art, such as those described in PCT/CN2021/096585 or PCT/CN2021/133059, which are incorporated herein by reference in their entirety. In specific embodiments, the TIL seed cell medium comprises the following components: cell culture components, cytokines, and immune checkpoint antibodies or antigen-binding fragments thereof, wherein the cytokines include IL-2, the immune checkpoints include PD-1, LAG-3, TIGIT, and/or CTLA-4, and the cell culture components are serum-containing medium or serum-free medium. In one or more embodiments, the concentration of IL-2 is about 3000 IU/mL or less. The cytokines may further include one or more selected from the group consisting of IL-7, IL-15, TNF$\alpha$, GM-CSF, G-CSF, M-CSF, IFN-$\gamma$, IFN-$\alpha$, IFN-$\beta$, IL-4, IL-1$\alpha$, IL-1$\beta$, IL-6, IL-9, IL-18, IL-12, IL-21, and IL-10. Exemplarily, the concentration of IL-7 is about 200 - about 1000 U/mL; the concentration of IL-15 is about 200 - about 500 U/mL; the concentration of TNF$\alpha$ is about 10 - about 100 pg/mL; the concentration of GM-CSF is about 200 - about 5000 U/mL; the concentration of G-CSF is about 300 U/mL - about 1000 U/mL; the concentration of M-CSF is about 300 U/mL - about 800 U/mL; the concentration of IFN-$\gamma$ is about 10 - about 1000 ng/mL; the concentration of IFN-$\gamma$ is about 300 - about 1000 U/mL; the concentration of IFN-$\alpha$ is about 500 U/mL - about 1000 U/mL; the concentration of IFN-$\beta$ is about 200 U/mL - about 500 U/mL; the concentration of IL-4 is about 200 - about 500 U/mL; the concentration of IL-1$\alpha$ is about 200 - about 500 U/mL; the concentration of IL-1$\beta$ is about 200 - about 500 U/mL; the concentration of IL-6 is about 200 - about 500 U/mL; the concentration of IL-9 is about 200 - about 500 U/mL; the concentration of IL-18 is about 200 - about 500 U/mL; the concentration of IL-12 is about 200 - about 1000 U/mL; the concentration of IL-21 is about 200 - about 500 U/mL; and the concentration of IL-10 is about 200 - about 500 U/mL.

The immune checkpoint antibody or antigen-binding fragment thereof in the TIL seed cell medium may be a human anti-PD-1 antibody or antigen-binding fragment thereof at a concentration of about 1 to about 100 $\mu$g/mL, or an anti-LAG-3 antibody or antigen-binding fragment thereof at a concentration of about 3 to about 10 $\mu$g/mL, or an anti-TIGIT antibody or antigen-binding fragment thereof at a concentration of about 1 to about 25 $\mu$g/mL, or an anti-CTLA-4 antibody or antigen-binding fragment thereof at a concentration of about 1 - about 100 $\mu$g/mL.

The TIL seed cell medium may further include costimulatory receptor antibodies or antigen-binding fragments thereof, such as: anti-CD40 antibody or antigen-binding fragment thereof, anti-OX-40 antibody or antigen-binding fragment thereof, anti-CD137 antibody or antigen-binding fragment thereof, and/or anti-CD28 antibody or antigen-binding fragment thereof. The concentration of the anti-CD137 antibody or antigen-binding fragment thereof is about 1 - about 100 $\mu$g/mL; the concentration of the anti-CD28 antibody or antigen-binding fragment thereof is about 1 - about 10 $\mu$g/mL; the concentration of the anti-CD40 antibody or antigen-binding fragment thereof is about 5 - about 10 $\mu$g/mL; and the concentration of the anti-OX-40 antibody or antigen-binding fragment thereof is about 3 - about 10 $\mu$g/mL.

In addition, the seed cell culture medium may further include M2 macrophage inhibitors, such as RRx001 and/or CNI-1493. The concentration of the M2 macrophage inhibitor is approximately 0.1-100 $\mu$M. The seed cell culture medium may further include regulatory T cell inhibitors, such as CAL-101, dasatinib, imatinib, and/or Panobinostat. The concentration of the regulatory T cell inhibitor is about 0.1 - about 100 $\mu$M. The seed cell culture medium may further include inhibitors of myeloid-derived suppressor cells (MDSCs), such as AG490, decitabine, sunitinib, and/or BBI608. The concentration of the myeloid-derived suppressor cell inhibitor is about 0.1 - about 100 $\mu$g/mL. The seed cell culture medium further includes T cell activators, such as LYC-55716, GNE-1858, and/or methylene blue. The concentration of the T cell activator is about 1 - about 10 $\mu$M. The seed cell culture medium may further include T cell differentiation inhibitors, such as TWS119. The concentration of the T cell differentiation inhibitor is about 1 - about 10 $\mu$M.

In specific embodiments, the TIL seed cell medium comprises any one of the following component combinations: 1) IL-2, IL-6, IL-21, IFN-$\gamma$, anti-TIGIT antibody, anti-PD-1 antibody, TNF-$\alpha$, and basal medium; 2) IL-2, IL-4, IL-10, IL-21, anti-CD137 antibody, anti-LAG3 antibody, anti-PD-1 antibody, TNF-$\alpha$, and basal medium; 3) IL-2, IL-7, IL-12, IL-21, anti-CD137 antibody, anti-CD28 antibody, anti-PD-1 antibody, and basal medium; 4) IL-1$\beta$, IL-2, IL-7, G-CSF, GM-CSF, IFN-$\gamma$, anti-LAG3 antibody, anti-PD-1 antibody, TNF-$\alpha$, and basal medium; 5) IL-2, IL-4, IL-12, GM-CSF, M-CSF, IFN-$\beta$, IFN-$\gamma$, anti-TIGIT antibody, anti-CTLA-4 antibody, and basal medium; 6) IL-2, IL-7, IL-15, GM-CSF, anti-CD137 antibody, anti-PD-1 antibody, TNF-$\alpha$, and basal medium; 7) IL-2, IL-4, IL-10, IL-15, G-CSF, M-CSF, anti-CD28 antibody, anti-OX-40 antibody, anti-PD-1 antibody, and basal medium; 8) IL-2, IL-7, IL-15, IFN-$\gamma$, anti-CD137 antibody, anti-CD40 antibody, anti-OX-40 antibody, anti-TIGIT antibody, anti-PD-1 antibody, and basal medium; 9) IL-2, IL-7, IL-15, GM-CSF, IFN-$\gamma$, anti-CD137 antibody, anti-CD28 antibody, anti-PD-1 antibody, TNF-$\alpha$, and basal medium; 10) IL-2, IL-7, IL-12, G-CSF, GM-CSF, IFN-$\alpha$, IFN-$\gamma$, anti-CD28 antibody, anti-CD40 antibody, anti-TIGIT antibody, anti-PD-1 antibody, TNF-$\alpha$, and basal medium; 11) IL-2, IL-7, IL-15, GM-CSF, anti-PD-1 antibody, RRx-001, CAL-101, and basal medium; 12) IL-2, IL-7, IL-15, GM-CSF, M-CSF, anti-PD-1 antibody, CNI-1493, and basal medium; 13) IL-2, IL-7, IL-15, anti-CD137 antibody, anti-CD28 antibody, anti-LAG3 antibody, anti-PD-1 antibody, dasatinib, and basal medium; 14) IL-2, IL-6, IL-12, G-CSF, M-CSF, IFN-$\beta$, IFN-$\gamma$, anti-CTLA-4 antibody, anti-PD-1 antibody, dasatinib, LYC-55716, GENE-1858, and basal medium; 15) IL-1$\alpha$, IL-2, IL-9, IL-15, GM-CSF, anti-CD137 antibody, anti-CD28 antibody, anti-LAG3 antibody, anti-TIGIT antibody, CNI-1493, and basal medium; 16) IL-2, IL-7, IL-12, IL-15, IL-21, G-CSF, M-CSF, IFN-$\gamma$, anti-CD28 antibody, anti-CD40 antibody, anti-LAG3 antibody, anti-PD-1 antibody, CNI-1493, dasatinib, GNE-1858, and basal medium. Preferably, the combinations of the above components further include serum and/or antibiotics. The serum concentration is about 1 - about 10% (v/v). The antibiotics include penicillin and/or streptomycin (e.g., PS dual antibiotics). The antibiotic concentration is about 1 - about 200 U/mL.

In other embodiments, the TIL is prepared by the following method: (1) incubating a TIL-containing sample with a TIL seed cell medium to obtain a first TIL population; and (2) incubating the first TIL population with a TIL expansion medium to obtain a second TIL population. The TIL seed cell medium is as described above.

The TIL expansion medium may be any medium capable of promoting TIL proliferation in the art, such as the culture composition for expanding TILs and the TIL expansion medium containing the culture composition described in PCT/CN2021/133059, which is incorporated herein by reference in its entirety. In specific embodiments, the TIL expansion medium comprises IL-2, IL-7, IL-15, an immune checkpoint antibody or antigen-binding fragment thereof, and a basal medium. Additionally, the expansion medium further comprises any one, two, three, or four selected from the group consisting of IL-21, IL-12, GM-CSF, and small molecules that enhance the proportion of memory T cells. Preferably, the expansion medium further comprises IL-21 and IL-12, or IL-21, IL-12, and GM-CSF, or GM-CSF and a small molecule that enhances the proportion of memory T cells. Preferably, the small molecule that enhances the proportion of memory T cells is TWS119. The immune checkpoint antibody comprises any one or more selected from the group consisting of an anti-PD-1 antibody, an anti-CTLA-4 antibody, an anti-LAG3 antibody, an anti-TIM3 antibody, an anti-TIGIT antibody, and an anti-BTLA antibody.

The TIL expansion medium may further comprise autologous platelets or allogeneic platelets. The TIL expansion medium may further comprise any one or more selected from the group consisting of an anti-CD3 antibody, an anti-CD28 antibody, an anti-CD137 antibody, an anti-OX40 antibody, an anti-GITR antibody, an anti-ICOS antibody, an anti-CD206 antibody, and an anti-CD40 antibody. Preferably, the culture composition further comprises any one, two, three, or four selected from the group consisting of an anti-CD3 antibody, an anti-CD28 antibody, an anti-CD137 antibody, and an anti-GITR antibody. For example, the expansion medium further comprises (1) an anti-CD3 antibody and an anti-CD28 antibody, or (2) an anti-CD3 antibody, an anti-CD28 antibody, an anti-CD137 antibody, and an anti-GITR antibody.

In one or more embodiments, the TIL expansion medium comprises any one of the following component combinations: 17) IL-2, IL-7, IL-15, anti-PD-1 antibody, IL-21, IL-12, anti-CD3 antibody, anti-CD28 antibody, and GM-CSF; 18) IL-2, IL-7, IL-15, anti-PD-1 antibody, IL-21, IL-12, anti-CD3 antibody, anti-CD28 antibody, anti-CD137 antibody, and anti-GITR antibody; 19) IL-2, IL-7, IL-15, anti-PD-1 antibody, anti-CD3 antibody, and anti-CD28 antibody, preferably, the anti-CD3 antibody and anti-CD28 antibody are conjugated to a matrix; 20) IL-2, IL-7, IL-15, anti-PD-1 antibody, IL-21, IL-12, anti-CD3 antibody, anti-CD28 antibody, and GM-CSF, preferably, the anti-CD3 antibody and anti-CD28 antibody are conjugated to a matrix; 21) IL-2, IL-7, IL-15, anti-PD-1 antibody, anti-CD3 antibody, anti-CD28 antibody, and anti-CD137 antibody, preferably, the anti-CD3 antibody, anti-CD137 antibody, and anti-CD28 antibody are conjugated to a matrix; 22) IL-2, IL-7, IL-15, anti-PD-1 antibody, anti-GITR antibody, anti-CD3 antibody, anti-CD28 antibody, TWS119, and anti-CD137 antibody, preferably, the anti-CD3 antibody, anti-CD137 antibody, and anti-CD28 antibody are conjugated to a matrix; 23) IL-2, IL-7, IL-15, anti-PD-1 antibody, anti-TIGIT antibody, anti-CD3 antibody, anti-CD28 antibody, anti-CD137 antibody, and GM-CSF, preferably, the anti-CD3 antibody, anti-CD137 antibody, and anti-CD28 antibody are conjugated to a matrix; 24) IL-2, IL-7, IL-15, anti-LAG3 antibody, IL-21, IL-12, anti-CD3 antibody, anti-CD28 antibody, anti-CD137 antibody, and anti-GITR antibody, preferably, the anti-CD3 antibody, anti-CD137 antibody, and anti-CD28 antibody are conjugated to a matrix; 25) IL-2, IL-7, IL-15, anti-PD-1 antibody, anti-TIGIT antibody, anti-CD3 antibody, anti-CD28 antibody, anti-CD137 antibody, anti-CD40 antibody, anti-OX-40 antibody, and autologous platelets, preferably, the anti-CD3 antibody, anti-CD137 antibody, and anti-CD28 antibody are conjugated to a matrix; 26) IL-2, IL-7, IL-15, anti-PD-1 antibody, anti-CTLA-4 antibody, anti-CD3 antibody, anti-CD28 antibody, anti-CD137 antibody, anti-GITR antibody, GM-CSF, TWS119, and allogeneic platelets, preferably, the anti-CD3 antibody and anti-CD28 antibody are conjugated to a matrix.

In one or more embodiments, the TIL expansion medium comprises a component selected from any one of the following and a basal medium, optionally further comprising serum and/or platelets: (1) 200-6000 IU/mL IL-2, 5-100 ng/mL IL-7, 5-100 ng/mL IL-15, and 3-100 $\mu$g/mL anti-PD-1 antibody, or equivalently proportional concentrates of these components; (2) 200-6000 IU/mL IL-2, 5-100 ng/mL IL-7, 5-100 ng/mL IL-15, and 1-100 $\mu$g/mL anti-PD-1 antibody, or equivalently proportional concentrates of these components; (3) 200-6000 IU/mL IL-2, 5-100 ng/mL IL-7, 5-100 ng/mL IL-15, 3-100 $\mu$g/mL anti-PD-1 antibody, 5-100 ng/mL IL-21, 5-100 ng/mL IL-12, 1-10 $\mu$g/mL anti-CD3 antibody, 1-10 $\mu$g/mL anti-CD28 antibody, and 200-5000 U/mL GM-CSF, or equivalently proportional concentrates of said components; (4) 200-6000 IU/mL IL-2, 5-100 ng/mL IL-7, 5-100 ng/mL IL-15, 3-100 $\mu$g/mL anti-PD-1 antibody, 5-100 ng/mL IL-21, 5-100 ng/mL IL-12, 1-10 $\mu$g/mL anti-CD3 antibody, 0.5-10 $\mu$g/mL anti-CD28 antibody, and 200-5000 U/mL GM-CSF, or equivalently proportional concentrates of said components; (5) 200-6000 IU/mL IL-2, 5-100 ng/mL IL-7, 5-100 ng/mL IL-15, 3-100 $\mu$g/mL anti-PD-1 antibody, 5-100 ng/mL IL-21, 5-100 ng/mL IL-12, 1-10 $\mu$g/mL anti-CD3 antibody, 1-10 $\mu$g/mL anti-CD28 antibody, 1-100 $\mu$g/mL anti-CD137 antibody, and 1-100 $\mu$g/mL anti-GITR antibody, or equivalently proportional concentrates of these components; (6) 200-6000 IU/mL IL-2, 5-100 ng/mL IL-7, 5-100 ng/mL IL-15, 1-100 $\mu$g/mL anti-PD-1 antibody, 5-100 ng/mL IL-21, 5-100 ng/mL IL-12, 0.5-10 $\mu$g/mL anti-CD3 antibody, 1-10 $\mu$g/mL anti-CD28 antibody, 1-100 $\mu$g/mL anti-CD137 antibody, and 1-100 $\mu$g/mL anti-GITR antibody, or equivalently proportional concentrates of these components; (7) 200-6000 IU/mL IL-2, 5-100 ng/mL IL-7, 5-100 ng/mL IL-15, 3-100 $\mu$g/mL anti-PD-1 antibody, 1-10 $\mu$g/mL anti-CD3 antibody, 1-10 $\mu$g/mL anti-CD28 antibody, 1-100 $\mu$g/mL anti-CD137 antibody, 1-100 $\mu$g/mL anti-GITR antibody, 200-5000 U/mL GM-CSF, and 1-500 $\mu$M TWS119, or equivalently proportional concentrates of these components; (8) 200-6000 IU/mL IL-2, 5-100 ng/mL IL-7, 5-100 ng/mL IL-15, 1-100 $\mu$g/mL anti-PD-1 antibody, anti-CD3 antibody conjugated to a matrix, anti-CD28 antibody conjugated to a matrix, 1-100 $\mu$g/mL anti-CD137 antibody, and 1-100 $\mu$g/mL anti-GITR antibody, or equivalently proportional concentrates of these components; (9) 200-6000 IU/mL IL-2, 5-100 ng/mL IL-7, 5-100 ng/mL IL-15, 1-100 $\mu$g/mL anti-PD-1 antibody, 5-100 ng/mL IL-21, 5-100 ng/mL IL-12, anti-CD3 antibody conjugated to a matrix, anti-CD28 antibody conjugated to a matrix, 1-100 $\mu$g/mL anti-CD137 antibody, and 1-100 $\mu$g/mL anti-GITR antibody, or equivalently proportional concentrates of these components; (10) 200-6000 IU/mL IL-2, 5-100 ng/mL IL-7, 5-100 ng/mL IL-15, 1-100 $\mu$g/mL anti-PD-1 antibody, anti-CD3 antibody conjugated to a matrix, anti-CD28 antibody conjugated to a matrix, and anti-CD137 antibody conjugated to a matrix, or equivalently proportional concentrates of these components; (11) 200-6000 IU/mL IL-2, 5-100 ng/mL IL-7, 5-100 ng/mL IL-15, 1-100 $\mu$g/mL anti-PD-1 antibody, anti-CD3 antibody conjugated to a matrix, anti-CD28 antibody conjugated to a matrix, anti-CD137 antibody conjugated to a matrix, 1-100 $\mu$g/mL anti-GITR antibody, and 1-500 $\mu$M TWS119, or equivalently proportional concentrates of these components; (12) 200-6000 IU/mL IL-2, 5-100 ng/mL IL-7, 5-100 ng/mL IL-15, 1-100 $\mu$g/mL anti-PD-1 antibody, anti-CD3 antibody conjugated to a matrix, anti-CD28 antibody conjugated to a matrix, anti-CD137 antibody conjugated to a matrix, 1-100 $\mu$g/mL anti-TIGIT antibody, and 200-5000 U/mL GM-CSF, or equivalently proportional concentrates of these components; (13) 200-6000 IU/mL IL-2, 5-100 ng/mL IL-7, 5-100 ng/mL IL-15, 1-100 $\mu$g/mL anti-LAG3 antibody, 5-100 ng/mL IL-21, 5-100 ng/mL IL-12, 0.5-10 $\mu$g/mL anti-CD3 antibody, 0.5-10 $\mu$g/mL anti-CD28 antibody, 1-100 $\mu$g/mL anti-CD137 antibody, and 1-100 $\mu$g/mL anti-GITR antibody, or equivalently proportional concentrates of these components; (14) 200-6000 IU/mL IL-2, 5-100 ng/mL IL-7, 5-100 ng/mL IL-15, 1-100 $\mu$g/mL anti-PD-1 antibody, anti-CD3 antibody conjugated to a matrix, anti-CD28 antibody conjugated to a matrix, anti-CD137 antibody conjugated to a matrix, 1-100 $\mu$g/mL anti-TIGIT antibody, 1-100 $\mu$g/mL anti-CD40 antibody, 1-100 $\mu$g/mL anti-OX-40 antibody, and $1\times10^8$-$5\times10^8$ autologous platelets per mL, or equivalently proportional concentrates of these components; (15) 200-6000 IU/mL IL-2, 5-100 ng/mL IL-7, 5-100 ng/mL IL-15, 1-100 $\mu$g/mL anti-PD-1 antibody, 1-100 $\mu$g/mL anti-CTLA-4 antibody, anti-CD3 antibody conjugated to a matrix, anti-CD28 antibody conjugated to a matrix, 1-100 $\mu$g/mL anti-CD137 antibody, 1-100 $\mu$g/mL anti-GITR antibody, 200-5000 U/mL GM-CSF, and $1\times10^8$-$5\times10^8$ allogeneic platelets per mL, or equivalently proportional concentrates of these components.

The antibody in the medium herein can be antibody from any species or recombinant antibody, preferably monoclonal antibody. These antibodies are well known in the art, for example, anti-PD-1 antibody Tislelizumab, anti-PD-1 antibody Sintilimab, anti-PD-1 antibody Toripalimab, anti-PD-1 antibody Camrelizumab, anti-CD3 antibody ab86883, anti-CD28 antibody MAB342, anti-GITR antibody as shown in SEQ ID NO: 104 and SEQ ID NO: 105 of CN103951753B, and CD137 antibody as shown in SEQ ID NO: 10 and SEQ ID NO: 11 of CN111182919A. These patent documents or publications are incorporated herein by reference in their entirety. One or more antibodies in the TIL seed cell medium and/or expansion medium described herein can be conjugated to a matrix, such as microspheres, well plates, petri dishes, cell culture flasks, or cell culture bags.

The serum described herein can be any serum known in the art to be usable for the culture of animal cells (e.g., TILs), such as human AB serum, the subject's autologous serum, or animal-derived serum. The concentration of the serum is 1-10%, e.g., 1%, 2%, 3%, 4%, 5%, 6%, 7%, 8%, 9%, 10%, or a range between any two of the above values.

The platelets described herein can be platelets derived from the subject's blood or platelets derived from allogeneic blood. The density of the platelets is $0.5\times10^8$/mL-$3\times10^8$/mL, e.g., $0.5\times10^8$/mL, $1\times10^8$/mL, $1.5\times10^8$/mL, $2\times10^8$/mL, $2.5\times10^8$/mL, $3\times10^8$/mL, or a range between any two of the above values.

The term "basal medium" described herein refers to any medium known in the art that can be used for TIL cell culture, including but not limited to any one or more of AIM-V, X-VIVO, DMEM, RPMI 1640, OpTmizerTM, and FUJIFILM Irvin MHM-C. The components and contents of these media are well known in the art, and their sources are as described in the examples.

The concentration at which TILs (either non-electroporated or electroporated) are inoculated into the TIL expansion medium is not limited and can be selected by those skilled in the art. In a preferred embodiment, TILs are inoculated into the TIL expansion medium at a concentration of $2.0\times10^5$-$5.0\times10^5$ cells/mL, e.g., about $2.0\times10^5$, about $2.5\times10^5$, about $3.0\times10^5$, about $4.5\times10^5$ cells/mL, or a range between any two of these values.

The duration of "culture" or "incubation" described herein can be about 3-20 days. For example, the culture or incubation time can be about 3-15 days, such as about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, or about 20 days, or a range between any two of these values. During the culture of TILs (e.g., during the incubation of TILs with the TIL expansion medium), the corresponding medium can be refreshed every 1, 2, 3, 4, 5, 6, 7, 8, 9, or 10 days.

For example, the temperature of the culture or incubation can be about 30-42°C. For example, it can be at least about 30°C, at least about 31°C, at least about 32°C, at least about 33°C, at least about 34°C, at least about 35°C, at least about 36°C, at least about 37°C, at least about 38°C, at least about 39°C, at least about 40°C, at least about 41°C, or at least about 42°C.

For example, the $CO_2$ concentration of the culture or incubation is about 1%-10%. For example, it can be at least about 1%, at least about 2%, at least about 3%, at least about 4%, at least about 5%, at least about 6%, at least about 7%, at least about 8%, at least about 9%, or at least about 10%.

The present invention includes a method for preparing the modified TILs and the modified TILs obtained thereby, the method comprising the step of introducing the membrane-anchored IL-7 or a nucleic acid molecule encoding the same described herein into the TILs obtained by the culture as described above. In some embodiments, the introduction is achieved by electroporation. After introduction (e.g., electroporation) of the coding sequence the cells may be activated (or stimulated) before subsequent operations (e.g., culture). Generally, activation is performed by incubating the electroporated cells in a TIL expansion medium for at least 1 day. The antibodies in the TIL expansion medium may be coated on the surface of the container or suspended in the medium. The incubation is preferably 1-10 days, e.g., 1-5 days. The conditions for the incubation can be determined by those skilled in the art, e.g., 37°C, 5% $CO_2$. In some embodiments, during the activation incubation, the electroporated cells are re-suspended in the TIL expansion medium at a concentration of $1.0 \times 10^5$-$1.0 \times 10^5$ cells/mL, e.g., $1.0 \times 10^5$, $2.0 \times 10^5$, $2.5 \times 10^5$, $5.0 \times 10^5$, $5.2 \times 10^5$, $1.0 \times 10^6$ cells/mL, or a range between any two of these values.

Thus, in one or more embodiments, the present invention provides a modified TIL that expresses the membrane-anchored IL-7 described herein, wherein the modified TIL is prepared by the following method: incubate a TIL-containing sample with the TIL seed cell medium described herein to obtain a first TIL population; 2) introduce a nucleic acid molecule (e.g., an expression vector) encoding the membrane-anchored IL-7 (e.g., via electroporation) into the first TIL population to obtain a second TIL population stably expressing the membrane-anchored IL-7; and optionally 3) activate the electroporated cells.

In one or more embodiments, the present invention provides a modified TIL that expresses the membrane-anchored IL-7 described herein, wherein the modified TIL is prepared by the following method: incubate a TIL-containing sample with the TIL seed cell medium described herein to obtain a first TIL population; 2) incubate the first TIL population with the TIL expansion medium described herein to obtain a second TIL population; 3) introduce a nucleic acid molecule (e.g., an expression vector) encoding the membrane-anchored IL-7 (e.g., via electroporation) into the second TIL population to obtain a third TIL population stably expressing the membrane-anchored IL-7; and optionally 4) activate the electroporated cells.

In one or more embodiments, the present invention provides a modified TIL that expresses the membrane-anchored IL-7 described herein, wherein the modified TIL is prepared by the following method: incubate a TIL-containing sample with the TIL seed cell medium described herein to obtain a first TIL population; 2) introduce a nucleic acid molecule (e.g., an expression vector) encoding the membrane-anchored IL-7 (e.g., via electroporation) into the first TIL population to obtain a second TIL population; optionally 3) activate the electroporated cells; and 4) incubate the second TIL population with the TIL expansion medium described herein to obtain a third TIL population stably expressing the membrane-anchored IL-7.

In some embodiments of preparing the modified TILs described herein, the TIL seed cell medium is selected from any one or more of the following (a), (b), and (c): TIL seed cell medium (a): IL-2 (3000 U/mL), IL-7 (50 ng/mL), IL-15 (50 ng/mL), IFN-$\gamma$ (1000 U/mL), anti-CD137 activating monoclonal antibody (5 $\mu$g/mL), anti-CD40 activating monoclonal antibody (5 $\mu$g/mL), anti-OX-40 activating monoclonal antibody (5 $\mu$g/mL), anti-TIGIT blocking monoclonal antibody (5 $\mu$g/mL), anti-PD-1 blocking monoclonal antibody (15 $\mu$g/mL), PS antibiotics (100 U/mL), and X-VIVO 15 basal medium to make up to the final volume; TIL seed cell medium (b): IL-2 (3000 U/mL), IL-7 (20 ng/mL), IL-15 (20 ng/mL), GM-CSF (500 U/mL), IFN-$\gamma$ (1000 U/mL), anti-CD137 activating monoclonal antibody (3 $\mu$g/mL), anti-CD28 activating monoclonal antibody (3 $\mu$g/mL), anti-PD-1 blocking monoclonal antibody (3 $\mu$g/mL), TNF-$\alpha$ (10 pg/mL), and X-VIVO 15 basal medium to make up to the final volume; TIL seed cell medium (c): IL-2 (3000 U/mL), IL-7 (20 ng/mL), IL-15 (30 ng/mL), anti-CD137 activating antibody (12 $\mu$g/mL), anti-CD28 activating monoclonal antibody (5 $\mu$g/mL), anti-LAG-3 blocking monoclonal antibody (5 $\mu$g/mL), anti-PD-1 blocking monoclonal antibody (3 $\mu$g/mL), dasatinib (3 $\mu$M), 5 v/v % human AB serum, and X-VIVO 15 basal medium to make up to the final volume. Alternatively, or in addition, in some embodiments of preparing the modified TILs described herein, the TIL expansion medium is selected from any one or more of the following (d) and (e): TIL expansion medium (d): IL-2 (500 U/mL), IL-7 (5 ng/mL), IL-15 (10 ng/mL), anti-PD-1 blocking monoclonal antibody (3 $\mu$g/mL), anti-CD3 activating monoclonal antibody, anti-CD28 activating monoclonal antibody, 5% (v/v) human AB serum, and X-VIVO 15 basal medium to make up the final volume. The anti-CD3 activating monoclonal antibody and anti-CD28 activating monoclonal antibody are coated on the surface of the culture vessel; TIL expansion medium (e): IL-2 (500 U/mL), IL-7 (7 ng/mL), IL-15 (30 ng/mL), anti-PD-1 blocking monoclonal antibody (1 $\mu$g/mL), anti-CD3 activating monoclonal antibody, anti-CD28 activating monoclonal antibody, anti-CD137 activating monoclonal antibody, 5% (v/v) human AB serum, and X-VIVO 15 basal medium to make up the final volume. The anti-CD3 activating monoclonal antibody, anti-CD137 activating monoclonal antibody, and anti-CD28 activating monoclonal antibody are coated on the surface of the culture vessel. In specific embodiments of preparing the modified TILs described herein, combinations of the TIL seed cell medium and TIL expansion medium were used as follows: (a) and (d), (a) and (e), (b) and (d), (b) and (e), (c) and (d), or (c) and (e).

In specific embodiments of the modified TILs or methods for preparing the same, the TIL-containing sample is derived from gastric cancer tissue, melanoma, ovarian cancer tissue, cervical cancer tissue, intestinal cancer tissue, lung cancer tissue, osteosarcoma, or cervical cancer recurrent bladder metastatic tissue; the membrane-anchored IL-7 comprises, sequentially linked: (i) a CD52 signal peptide, IL-7, a linker, an extracellular domain of BCMA or a variant thereof, and a CD8 transmembrane domain; (ii) a CD52 signal peptide, IL-7, a linker, an extracellular domain of BCMA or a variant thereof, and CD52; (iii) a CD52 signal peptide, IL-7, a linker, an extracellular domain of BCMA or a variant thereof, a hinge region or linker, and CD52; (iv) a CD52 signal peptide, IL-7, an extracellular domain of BCMA or a variant thereof, a hinge region or linker, and CD52; (v) (vi) an IL-7 signal peptide, IL-7, a linker, an extracellular domain of BCMA or a variant thereof, and a CD8 transmembrane domain; or (vii) an IL-7 signal peptide, IL-7, a linker, an extracellular domain of BCMA or a variant thereof, and CD52; the seed cell culture medium used for preparing TILs is the aforementioned TIL seed cell medium (a), (b), or (c); the culture duration in the seed cell culture medium is about 11-15 days; the TIL expansion medium is the aforementioned TIL expansion medium (d) or (e); the culture duration in the expansion medium is about 11-15 days; the cell seeding density in the expansion medium is $2.0 \times 10^5$-$4.5 \times 10^5$. Preferably, the sequence of the membrane-anchored IL-7 is as shown in any one or more of SEQ ID NO: 31, 33, 35, 37, 39, 41, 43, and 45.

In the first set of embodiments of the modified TILs or the methods for preparing the same, the TIL-containing sample is derived from gastric cancer tissue; the membrane-anchored IL-7 comprises, sequentially linked, a CD52 signal peptide, IL-7, a linker, the extracellular domain of BCMA or a variant thereof, and CD52; the seed cell culture medium used for preparing the TILs is the aforementioned TIL seed cell medium (a); the culture duration in the seed cell culture medium is about 11 days; the TIL expansion medium is the aforementioned TIL expansion medium (e); the culture duration in the expansion medium is about 12 days; and the cell seeding density in the expansion medium is $2.5 \times 10^5$. Preferably, the sequence of the membrane-anchored IL-7 is as shown in SEQ ID NO: 31.

In the second set of embodiments of the modified TILs or the methods for preparing the same, the TIL-containing sample is derived from melanoma; the membrane-anchored IL-7 comprises, sequentially linked, a CD52 signal peptide, IL-7, a linker, the extracellular domain of BCMA or a variant thereof, and CD52; the seed cell culture medium used for preparing the TILs is the aforementioned TIL seed cell medium (b); the culture duration in the seed cell culture medium is approximately 12 days; the TIL expansion medium is the aforementioned TIL expansion medium (d); the culture duration in the expansion medium is approximately 12 days; and the cell seeding density in the expansion medium is $2.5 \times 10^5$. Preferably, the sequence of the membrane-anchored IL-7 is as shown in SEQ ID NO: 33.

In the third set of embodiments of the modified TILs or the methods for preparing the same, the TIL-containing sample is derived from ovarian cancer tissue; the membrane-anchored IL-7 comprises, sequentially linked, a CD52 signal peptide, IL-7, a linker, the extracellular domain of BCMA or a variant thereof, and CD52; the seed cell culture medium used for preparing the TIL is the aforementioned TIL seed cell medium (a); the culture duration in the seed cell culture medium is approximately 12 days; the TIL expansion medium is the aforementioned TIL expansion medium (e); the culture duration in the expansion medium is approximately 12 days; and the cell seeding density in the expansion medium is $2.0 \times 10^5$. Preferably, the sequence of the membrane-anchored IL-7 is as shown in SEQ ID NO: 33.

In the fourth set of embodiments of the modified TILs or the methods for preparing the same, the TIL-containing sample is derived from cervical cancer tissue; the membrane-anchored IL-7 comprises, sequentially linked, a CD52 signal peptide, IL-7, a linker, the extracellular domain of BCMA or a variant thereof, and CD52; the seed cell culture medium used for preparing the TIL is the aforementioned TIL seed cell medium (a); the culture duration in the seed cell culture medium is approximately 12 days; the TIL expansion medium is the aforementioned TIL expansion medium (e); the culture duration in the expansion medium is approximately 12 days; and the cell seeding density in the expansion medium is $2.5 \times 10^5$. Preferably, the sequence of the membrane-anchored IL-7 is as shown in SEQ ID NO: 31.

In the fifth set of embodiments of the modified TILs or the methods for preparing the same, the TIL-containing sample is derived from ovarian cancer tissue; the membrane-anchored IL-7 comprises, sequentially linked, a CD52 signal peptide, IL-7, a linker, the extracellular domain of BCMA or a variant thereof, and CD52; the seed cell culture medium used for preparing the TILs is the aforementioned TIL seed cell medium (a); the culture duration in the seed cell culture medium is approximately 12 days; the TIL expansion medium is the aforementioned TIL expansion medium (e); the culture duration in the expansion medium is approximately 11 days; and the cell seeding density in the expansion medium is $2.5 \times 10^5$. Preferably, the sequence of the membrane-anchored IL-7 is as shown in SEQ ID NO: 35.

In the sixth set of embodiments of the modified TILs or the methods for preparing the same, the TIL-containing sample is derived from intestinal cancer tissue; the membrane-anchored IL-7 comprises, sequentially linked, a CD52 signal peptide, IL-7, the extracellular domain of BCMA or a variant thereof, a linker, and CD52; the seed cell culture medium used for preparing the TILs is the aforementioned TIL seed cell medium (a); the culture duration in the seed cell culture medium is approximately 13 days; the TIL expansion medium is the aforementioned TIL expansion medium (e); the culture duration in the expansion medium is approximately 11 days; and the cell seeding density in the expansion medium is $2.5 \times 10^5$L. Preferably, the sequence of the membrane-anchored IL-7 is as shown in SEQ ID NO: 39.

In the seventh set of embodiments of the modified TILs or the methods for preparing the same, the TIL-containing sample is derived from lung cancer tissue; the membrane-anchored IL-7 comprises, sequentially linked, a CD52 signal peptide, IL-7, a linker, the extracellular domain of BCMA or a variant thereof, and CD52; the seed cell culture medium used for preparing the TILs is the aforementioned TIL seed cell medium (a); the culture duration in the seed cell culture medium is about 12 days; the TIL expansion medium is the aforementioned TIL expansion medium (e); the culture duration in the expansion medium is about 12 days; and the cell seeding density in the expansion medium is $3.0 \times 10^5$. Preferably, the sequence of the membrane-anchored IL-7 is as shown in SEQ ID NO: 41.

In the eighth set of embodiments of the modified TILs or the methods for preparing the same, the TIL-containing sample is derived from osteosarcoma tissue; the membrane-anchored IL-7 comprises, sequentially linked, an IL-7 signal peptide, IL-7, a linker, the extracellular domain of BCMA or a variant thereof, and CD52; the seed cell culture medium used for preparing the TILs is the aforementioned TIL seed cell medium (a); the culture duration in the seed cell culture medium is about 15 days; the TIL expansion medium is the aforementioned TIL expansion medium (e); the culture duration in the expansion medium is about 15 days; and the cell seeding density in the expansion medium is $2.5 \times 10^5$. Preferably, the sequence of the membrane-anchored IL-7 is as shown in SEQ ID NO: 45.

In the ninth set of embodiments of the modified TILs or the methods for preparing the same, the TIL-containing sample is derived from bladder metastatic tissue from recurrent cervical cancer; the membrane-anchored IL-7 comprises, sequentially linked, a CD52 signal peptide, IL-7, a linker, the extracellular domain of BCMA or a variant thereof, and CD52; the seed cell culture medium used for preparing the TILs is the aforementioned TIL seed cell medium (a); the culture duration in the seed cell culture medium is about 12 days; the TIL expansion medium is the aforementioned TIL expansion medium (e); the culture duration in the expansion medium is about 12 days; and the cell seeding density in the expansion medium is $2.5 \times 10^5$. Preferably, the sequence of the membrane-anchored IL-7 is as shown in SEQ ID NO: 35.

In the tenth set of embodiments of the modified TILs or the methods for preparing the same, the TIL-containing sample is derived from ovarian cancer tissue; the membrane-anchored IL-7 comprises, sequentially linked, a CD52 signal peptide, IL-7, the extracellular domain of BCMA or a variant thereof, the extracellular hinge region of CD8, and CD52; the seed cell culture medium used for preparing the TILs is the aforementioned TIL seed cell medium (a); the culture duration in the seed cell culture medium is about 12 days; the TIL expansion medium is the aforementioned TIL expansion medium (e); the culture duration in the expansion medium is about 12 days; and the cell seeding density in the expansion medium is $4.5 \times 10^5$. Preferably, the sequence of the membrane-anchored IL-7 is as shown in SEQ ID NO: 37.

In the eleventh set of embodiments of the modified TILs or the methods for preparing the same, the TIL-containing sample is derived from ovarian cancer tissue; the membrane-anchored IL-7 comprises, sequentially linked, a CD52 signal peptide, IL-7, a linker, the extracellular domain of BCMA or a variant thereof, and the CD8 transmembrane domain; the seed cell culture medium used for preparing the TILs is the aforementioned TIL seed cell medium (a); the culture duration in the seed cell culture medium is about 12 days; the TIL expansion medium is the aforementioned TIL expansion medium (e); the culture duration in the expansion medium is about 12 days; and the cell seeding density in the expansion medium is $2.0 \times 10^5$. Preferably, the sequence of the membrane-anchored IL-7 is as shown in SEQ ID NO: 43.

The TIL population prepared by the method of the present invention can be used to prepare drugs for treating cancer. Therefore, provided herein is also a pharmaceutical composition comprising the modified TILs described herein and a pharmaceutically acceptable excipient. As used herein, a pharmaceutically acceptable excipient refers to a carrier, diluent, and/or vehicle that is pharmacologically and/or physiologically compatible with the subject and the active ingredient, including but not limited to: pH adjusters, surfactants, carbohydrates, adjuvants, antioxidants, chelating agents, ionic strength enhancers, and preservatives. More specifically, suitable pharmaceutically acceptable excipients may be those commonly used in the art for TIL infusion (e.g., intravenous infusion).

Typically, the pharmaceutical composition contains a therapeutically effective amount of the modified TIL. A therapeutically effective amount refers to a dosage that can achieve the treatment, prevention, alleviation and/or relief of a disease or disorder in a subject. The therapeutically effective amount can be determined based on factors such as the patient's age, gender, the disease or disorder suffered and its severity, and the patient's other physical conditions. As used herein, a subject or patient typically refers to a mammal, in particular a human.

The present invention also includes a cell therapy, including administering the modified TIL to a subject, wherein the TIL is genetically modified to express the membrane-anchored IL-7 described herein. The administered cells are capable of killing the recipient's tumor cells. Additionally, the immune response mediated by TILs can be part of the process of adoptive immunotherapy.

Herein, the diseases suitable for treatment with the pharmaceutical compositions described herein are related to the modified TIL. The diseases described herein include solid tumors and hematological tumors, such as solid tumors including adenocarcinoma, lung cancer, colon cancer, colorectal cancer, breast cancer, ovarian cancer, cervical cancer, gastric cancer, cholangiocarcinoma, gallbladder cancer, esophageal cancer, pancreatic cancer, and prostate cancer; and hematological tumors including leukemias and lymphomas such as B-cell lymphoma, mantle cell lymphoma, acute lymphoblastic leukemia, chronic lymphocytic leukemia, hairy cell leukemia, and acute myeloid leukemia.

The modified TIL of the present invention can be administered alone or as a pharmaceutical composition in a manner suitable for treating (or preventing) diseases. The amount and frequency of administration will be determined by various factors, such as the patient's condition, and the type and severity of the patient's disease. Administration of the composition can be performed in any convenient manner, including by nebulization, injection, ingestion, infusion, implantation, or transplantation. The compositions described herein can be administered to a patient subcutaneously, intradermally, intratumorally, intranodally, intraspinally, intramuscularly, via intravenous injection, or intraperitoneally. In one embodiment, the polypeptide, cell, or pharmaceutical composition of the present invention is administered to the patient via intradermal or subcutaneous injection (e.g., intravenous injection). Alternatively, the polypeptide, cell, or pharmaceutical composition can be directly injected into a tumor, lymph node, or site of infection.

In some embodiments of the present invention, the modified TIL of the present invention can be combined with other therapies known in the art. The therapies include but are not limited to chemotherapy, radiation therapy, and immunosuppressants. For example, treatment can be performed in combination with radiation therapy or chemotherapeutic agents known in the art for treating diseases mediated by tumor antigens.

In certain embodiments, the present invention further provides a kit, comprising the nucleic acid constructs described herein, such as vectors. The kit may also comprise various reagents suitable for transfecting the nucleic acid constructs into cells, as well as optional instructions for instructing those skilled in the art to transfect the nucleic acid constructs into cells.

In certain embodiments, the present invention further provides a cell cryopreservation preparation, comprising the modified TIL of the present invention and a cryopreservation solution. The cryopreservation solution herein may be a tissue cryopreservation solution or a cell cryopreservation solution, which can keep the TILs of the present invention alive under low temperature conditions, such as under dry ice cryopreservation or liquid nitrogen cryopreservation conditions. Preferably, the cryopreservation solution may be a serum-free cryopreservation solution, and the cell cryopreservation preparation can be directly reinfused to the individual without removal after recovery from low temperature without obvious toxic side effects to the individual.

The present invention will be illustrated below by way of specific examples. It should be understood that these examples are merely illustrative and are not intended to limit the scope of the present invention. The methods and materials used in the examples are conventional materials and methods in the art unless otherwise specified.

Examples

Example 1: Construction of expression vectors for membrane-anchored IL-7

Synthesizing the coding sequences of each membrane-anchored IL-7 anchored on the membrane surface in Table 1.

Table 1

| Name | Structure | Protein sequence | Encoding sequence |
|---|---|---|---|
| mbIL-7-1 | CD52 signal peptide -IL-7-linker 1-BCMA extracellular domain -CD52 | SEQ ID NO:31 | SEQ ID NO:32 |
| mbIL-7-2 | CD52 signal peptide -IL-7- linker 2-BCMA extracellular domain - CD52 | SEQ ID NO:33 | SEQ ID NO:34 |
| mbIL-7-3 | CD52 signal peptide -IL-7- linker 4-point-mutated BCMA extracellular domain - CD52 | SEQ ID NO:35 | SEQ ID NO:36 |
| mbIL-7-4 | CD52 signal peptide -IL-7- linker 5-truncated point-mutated BCMA extracellular domain - CD8 extracellular hinge region -CD52 | SEQ ID NO:37 | SEQ ID NO:38 |
| mbIL-7-5 | CD52 signal peptide -IL-7- point mutation BCMA extracellular domain - linker 4-CD52 | SEQ ID NO:39 | SEQ ID NO:40 |
| mbIL-7-6 | CD52 signal peptide -IL-7- linker 3-BCMA extracellular domain -CD52 | SEQ ID NO:41 | SEQ ID NO:42 |
| mbIL-7-7 | CD52 signal peptide -IL-7- linker 2-BCMA extracellular domain - CD8 transmembrane domain | SEQ ID NO:43 | SEQ ID NO:44 |
| mbIL-7-8 | IL-7 signal peptide -IL-7- linker 2-truncated BCMA extracellular domain -CD52 | SEQ ID NO:45 | SEQ ID NO:46 |

(continued)

| Name | Structure | Protein sequence | Encoding sequence |
|------|-----------|------------------|-------------------|
| mbIL-7-9 | CD52 signal peptide -IL-7- linker 5-truncated point-mutated BCMA extracellular domain - CD52 | SEQ ID NO:47 | SEQ ID NO:48 |

The pKB20 vector was constructed according to the method described in example 1 on page 21 of the specification of PCT Application WO2022078310A1. According to the method described in this example, the pKB20 vectors containing the expression cassettes of mbIL-7-1, mbIL-7-2, mbIL-7-3, mbIL-7-4, mbIL-7-5, mbIL-7-6, mbIL-7-7, mbIL-7-8, and mbIL-7-9 were constructed and named pKB20-mbIL-7-1, pKB20-mbIL-7-2, pKB20-mbIL-7-3, pKB20-mbIL-7-4, pKB20-mbIL-7-5, pKB20-mbIL-7-6, pKB20-mbIL-7-7, pKB20-mbIL-7-8 and pKB20-mbIL-7-9, respectively. The obtained recombinant plasmids were transformed into E. coli (DH5$\alpha$). After the sequence was verified as correct by sequencing, the plasmids were extracted and purified using the plasmid purification kit of Qiagen to obtain high-quality plasmids of each recombinant expression vector.

Example 2 Preparation of TILs expressing membrane-anchored IL-7

The components and NOs. of the TIL seed cell medium and TIL expansion medium used are as follows:

TIL seed cell medium:

(a) IL-2 (3000 U/mL), IL-7 (50 ng/mL), IL-15 (50 ng/mL), IFN-$\gamma$ (1000 U/mL), anti-CD137 agonistic monoclonal antibody (5 $\mu$g/mL), anti-CD40 agonistic monoclonal antibody (5 $\mu$g/mL), anti-OX-40 agonistic monoclonal antibody (5 $\mu$g/mL), anti-TIGIT blocking monoclonal antibody (5 $\mu$g/mL), anti-PD-1 blocking monoclonal antibody (15 $\mu$g/mL), PS dual antibiotic (100 U/mL), and X-VIVO 15 basal medium to reach the final volume;
(b) IL-2 (3000 U/mL), IL-7 (20 ng/mL), IL-15 (20 ng/mL), GM-CSF (500 U/mL), IFN-$\gamma$ (1000 U/mL), anti-CD137 agonistic monoclonal antibody (3 $\mu$g/mL), anti-CD28 agonistic monoclonal antibody (3 $\mu$g/mL), anti-PD-1 blocking monoclonal antibody (3 $\mu$g/mL), TNF-$\alpha$ (10 pg/mL), and X-VIVO 15 basal medium to reach the final volume;
(c) IL-2 (3000 U/mL), IL-7 (20 ng/mL), IL-15 (30 ng/mL), anti-CD137 agonistic antibody (12 $\mu$g/mL), anti-CD28 agonistic monoclonal antibody (5 $\mu$g/mL), anti-LAG-3 blocking monoclonal antibody (5 $\mu$g/mL), anti-PD-1 blocking monoclonal antibody (3 $\mu$g/mL), dasatinib (3 $\mu$M), 5 v/v % human AB serum, and X-VIVO 15 basal medium to reach the final volume.

TIL expansion medium

(d) IL-2 (500 U/mL), IL-7 (5 ng/mL), IL-15 (10 ng/mL), anti-PD-1 blocking monoclonal antibody (3 $\mu$g/mL), anti-CD3 agonistic monoclonal antibody, anti-CD28 agonistic monoclonal antibody, 5 v/v % human AB serum, and X-VIVO 15 basal medium to reach the final volume. The anti-CD3 agonistic monoclonal antibody and anti-CD28 agonistic monoclonal antibody are coated on the surface of the culture vessels;
(e) IL-2 (500 U/mL), IL-7 (7 ng/mL), IL-15 (30 ng/mL), anti-PD-1 blocking monoclonal antibody (1 $\mu$g/mL), anti-CD3 agonistic monoclonal antibody, anti-CD28 agonistic monoclonal antibody, anti-CD137 agonistic monoclonal antibody, 5 v/v % human AB serum, and X-VIVO 15 basal medium to reach the final volume. The anti-CD3 agonistic monoclonal antibody, anti-CD137 agonistic monoclonal antibody, and anti-CD28 agonistic monoclonal antibody are coated on the surface of the culture vessels.

Processing of solid tumor samples and preparation of TILs expressing membrane-anchored IL-7:
1) Physiological saline containing a final concentration of 100 U/mL penicillin, 100 $\mu$g/mL streptomycin, and 50 $\mu$g/mL gentamicin was prepared for use
2) Freshly isolated tumor tissue samples from tumor patients were washed in a 10 cm dish containing 30 mL of step 1)-prepared saline in a sterile environment in a Biological Safety Level II cabinet, and then transferred to a new 10 cm dish with 30 mL of saline prepared in step 1) for washing, with this step repeated 3 times in total.
3) Sterile surgical blades were used to remove adipose and necrotic tissues, and the tumor tissue was cut into small pieces approximately $3\times3\times3$ mm$^3$ in size. Two G-REX100 culture vessels (purchased from Wilsonwolf) were prepared, and 42 randomly selected tumor tissue pieces were placed into each vessel. One of the above-mentioned TIL seed cell medium a, b, and c was added to the culture vessel as the seed cell culture medium. Excess tumor tissue fragments were frozen in liquid nitrogen using CryoStor10 (purchased from BioLife Solutions) cryopreservation solution via a controlled-rate freezer.

4) 1L TIL seed cell medium was added to the G-REX100 culture vessel containing tumor tissue fragments in 3), and the tumor tissue fragments were cultured at 37°C with 5% $CO_2$. Half of the old seed cell culture medium was removed every 4 days and replenished with fresh seed cell culture medium. From the 11th to 15th day, the total number and the viability of cells were counted after the TIL seed cells were harvested by centrifugation.

5) The TIL seed cells harvested in step 4) were taken, and a population of TIL seed cells containing $1 \times 10^8$ to $2 \times 10^8$ viable cells were collected based on the detected viability. The expression vector expressing membrane-anchored IL-7 prepared in Example 1 was electroporated into the nucleus using a Lonza Nucleofector™2b electroporator, and the specific steps are as follows:

(1). Medium b prepared in Example 2 was pre-added to the wells of G-REX6 at 40 mL per well, and was subsequently transferred into a cell incubator and preheated at 37°C with 5% $CO_2$ for 1 hour.

(2). The electroporation solution for each well was prepared according to the table below:

|  | 100 μL Nucleocuvette™ Strip (μL) |
| --- | --- |
| Volume of the Nucleofector™ solution | 82 |
| Volume of the electroporation supplemental solution | 18 |

(3). TILs harvested in Step 5) were transferred into Eeppendorf tubes, with $1 \times 10^7$ cells added to each tube. The cells were centrifuged at 800g for 5 minutes, and the supernatant was discarded. Subsequently, the cells were resuspended in 500 μL of physiological saline, and the centrifugation step was repeated to wash the cell pellet.

(4). 0.6-1 μL (3-5 μg) of each expression vector expressing membrane-anchored IL-7 prepared in Example 1 with an adjusted concentration of 5 μg/mL was added to the electroporation solution prepared in step (2), and then stood at room temperature for less than 30 minutes (the total volume can be slightly larger than 100 μL at this time).

(5). TILs were resuspended in the plasmid-containing electroporation solution prepared in (4). The cell suspension was carefully aspirated and transferred into a LONZA 100-μL electroporation cuvette. The cuvette was placed in the LONZA Nucleofector™ 2b electroporation chamber and the electroporation program was started with T-020 selected.

(6). After the electroporation was completed, the electroporation cuvette was carefully removed, the cell suspension was transferred to the Eppendorf tube, and 200 μL of preheated X-VIVO 15 medium was added to each tube, and then transferred to the well of G-REX6 in (1) containing the preheated medium b of Example 2. The electroporation procedure was repeated until all $1 \times 10^8$ - $2 \times 10^8$ viable TIL seed cells had been electroporated. The cells were then cultured at 37°C with 5% $CO_2$. Half of the old seed cell culture medium was removed and replenished with fresh seed cell culture medium every 2 days until the cell viability recovered to over 90%, and preparations were made for subsequent operations.

6) The electroporated TIL seed cells from Step 5) were centrifuged at 800g for 5 minutes, resuspended at $1.0 \times 10^5$/mL to $1.0 \times 10^5$/mL in TIL expansion medium containing all components of the corresponding solution except for the coating components, and added to cell culture vessels pre-coated with the coating components. The cells were activated at 37°C with 5% $CO_2$ for 2-3 days. The activated cells were then centrifuged, collected, and seeded into G-REX500M culture vessels containing pre-warmed expansion medium, which was also the same expansion medium containing all components of the corresponding solution except for the coating components. The expansion medium volume in each G-REX500M is 5 L. The activated seed cells were seeded at a density of $2.0 \times 10^5$/cm$^2$ to $5.0 \times 10^5$/cm$^2$ and cultured at 37°C with 5% $CO_2$. Every four days, after cell counting, half of the volume of the old expansion medium was removed and replenished with an equal volume of fresh expansion medium. Once the total number of cells in each G-REX500M vessel reached $1.0 \times 10^{10}$, the cells were sub-cultured at a 1:2 ratio, and each new vessel was supplemented with fresh expansion medium to a final volume of 5 L before continuing the culture. The cells were harvested after a total culture duration of 10-12 days in the expansion medium of G-REX500M culture vessels, and TIL product cells that express membrane-anchored IL-7 were obtained.

Table 2 below shows the Nos. of tumor tissue samples, the Nos. of membrane-anchored IL-7-expressing TILs obtained through culture, the cancer types, the Nos. of the seed cell culture medium and expansion medium used in the culture process corresponding to Example 2, the respective culture durations of the seed cell culture medium and expansion medium for each tissue sample, the names of specific vectors used for electroporation of each tumor tissue sample, and the G-REX500M cell seeding densities; Table 3 shows the types of coated vessels, the density of coated, activated, and resuspended cells, and the duration of coated activation for the corresponding tumor tissue samples in Step 6).

Table 2

| TIL No. | tumor tissue sample No. | cancer types | No. of the seed cell culture medium | culture duration of the seed cell culture medium (days) | vector names used for electroporation | No. of expansion medium | G-REX500M cell seeding densities ( cells/cm²) | culture duration of expansion medium (days) |
|---|---|---|---|---|---|---|---|---|
| T00 1-1 | T001 | gastric cancer | a | 11 | mbIL-7-1 | e | $2.5 \times 10^5$ | 12 |
| T00 2-2 | T002 | melanoma | b | 12 | mbIL-7-2 | d | $2.5 \times 10^5$ | 12 |
| T00 3-2 | T003 | ovarian cancer | b | 12 | mbIL-7-2 | e | $2.0 \times 10^5$ | 12 |
| T00 4-1 | T004 | cervical cancer | c | 12 | mbIL-7-1 | d | $2.5 \times 10^5$ | 12 |
| T00 5-3 | T005 | ovarian cancer | c | 12 | mbIL-7-3 | e | $2.5 \times 10^5$ | 11 |
| T00 6-5 | T006 | colon cancer | a | 13 | mbIL-7-5 | d | $2.5 \times 10^5$ | 11 |
| T00 7-6 | T007 | lung cancer | a | 12 | mbIL-7-6 | d | $3.0 \times 10^5$ | 12 |
| T00 8-8 | T008 | osteosarcoma | b | 15 | mbIL-7-8 | e | $2.5 \times 10^5$ | 15 |
| T00 9-3 | T009 | recurrence of cervical cancer with bladder metastasis | c | 12 | mbIL-7-3 | d | $2.5 \times 10^5$ | 12 |
| T01 0-4 | T010 | recurrence of ovarian cancer | a | 12 | mbIL-7-4 | e | $4.5 \times 10^5$ | 12 |
| T00 3-7 | T003 | ovarian cancer | b | 12 | mbIL-7-7 | e | $2.0 \times 10^5$ | 12 |
| T01 1-9 | T011 | cervical cancer | b | 12 | mbIL-7-9 | d | $2.5 \times 10^5$ | 12 |
| T01 2-9 | T012 | ovarian cancer | b | 12 | mbIL-7-9 | d | $2.5 \times 10^5$ | 13 |
| T01 3-9 | T013 | cervical cancer | b | 12 | mbIL-7-9 | d | $2.5 \times 10^5$ | 12 |
| T01 4-9 | T014 | ovarian cancer with mesenteric metastasis | b | 12 | mbIL-7-9 | d | $2.5 \times 10^5$ | 12 |

Table 3

| TIL No. | tumor tissue sample No. | cancer types | types of coated vessels | density of activated and resuspended cells (cells/mL) | duration of activation (days) |
|---|---|---|---|---|---|
| T001-1 | T001 | gastric cancer | cell culture bag | $2.0 \times 10^5$ | 2 |

(continued)

| TIL No. | tumor tissue sample No. | cancer types | types of coated vessels | density of activated and resuspended cells (cells/mL) | duration of activation (days) |
|---|---|---|---|---|---|
| T002-2 | T002 | melanoma | cell culture bag | $1.0\times10^5$ | 3 |
| T003-2 | T003 | ovarian cancer | cell culture bag | $2.5\times10^5$ | 3 |
| T004-1 | T004 | cervical cancer | 245mm cell culture dish | $5.2\times10^5$ | 2 |
| T005-3 | T005 | ovarian cancer | cell culture bag | $2.5\times10^5$ | 3 |
| T006-5 | T006 | colorectal cancer | cell culture bag | $2.5\times10^5$ | 3 |
| T007-6 | T007 | lung cancer | cell culture bag | $2.5\times10^5$ | 3 |
| T008-8 | T008 | osteosarcoma | cell culture bag | $5.0\times10^5$ | 3 |
| T009-3 | T009 | recurrence of cervical cancer with bladder metastasis | 245mm cell culture dish | $1.0\times10^6$ | 3 |
| T010-4 | T010 | recurrence of ovarian cancer | cell culture bag | $2.5\times10^5$ | 2 |
| T003-7 | T003 | ovarian cancer | cell culture bag | $2.5\times10^5$ | 3 |
| T011-9 | T011 | cervical cancer | cell culture bag | $2.5\times10^5$ | 3 |
| T012-9 | T012 | ovarian cancer | cell culture bag | $2.5\times10^5$ | 3 |
| T013-9 | T013 | cervical cancer | cell culture bag | $2.5\times10^5$ | 3 |
| T014-9 | T014 | ovarian cancer | cell culture bag | $2.5\times10^5$ | 3 |

Preparation of natural TILs

Natural TILs from T001-T014 tumor tissues were prepared according to the steps below.

1) Physiological saline containing a final concentration of 100 U/mL penicillin, 100 $\mu$g/mL streptomycin, and 50 $\mu$g/mL gentamicin was prepared for use

2) Freshly isolated tumor tissue samples from tumor patients were washed in a 10 cm dish containing 30 mL of saline prepared in step 1) in a sterile environment in a Biological Safety Level II cabinet, and then transferred to a new 10 cm dish with 30 mL of saline prepared in step 1) for washing, with this step repeated 3 times in total.

3) Sterile surgical blades were used to remove adipose and necrotic tissues, and the tumor tissue was cut into small pieces approximately $3\times3\times3$ mm$^3$ in size. Two G-REX100 culture vessels (purchased from Wilsonwolf) were prepared, and 42 randomly selected tumor tissue pieces were placed into each vessel. One of the above-mentioned TIL seed cell medium a, b, and c was added to the culture vessel as the seed cell culture medium. Excess tumor tissue fragments were frozen in liquid nitrogen using CryoStor10 (purchased from BioLife Solutions) cryopreservation solution via a controlled-rate freezer.

4) 1L of TIL seed cell medium was added to the G-REX100 culture vessel containing tumor tissue fragments in 3), and the tumor tissue fragments were cultured at 37°C with 5% $CO_2$. Half of the old seed cell culture medium was removed every 4 days and replenished with fresh seed cell culture medium. From the 11th to 15th day, the total number of cells and the viability were counted after the TIL seed cells were harvested by centrifugation.

5) The TIL seed cells from Step 4) were centrifuged at 800g for 5 minutes, resuspended at $1.0\times10^5$/mL to $1.0\times10^5$/mL in TIL expansion medium containing all components of the corresponding solution except for the coating components, and added to cell culture vessels pre-coated with the coating components. The cells were activated at 37°C with 5% $CO_2$ for 2-3 days. The activated cells were then centrifuged, collected, and seeded into G-REX500M culture vessels containing pre-warmed expansion medium, which was also the same expansion medium containing all components of the corresponding solution except for the coating components. The expansion medium volume in each G-REX500M is 5 L. The activated seed cells were seeded at a density of $2.0\times10^5$/cm$^2$ to $5.0\times10^5$/cm$^2$ and cultured at 37°C with 5% $CO_2$. Every four days, after cell counting, half of the volume of the old expansion medium was removed, and replenished with an equal volume of fresh expansion medium. Once the total number of cells in each G-REX500M vessel reached $1.0\times10^{10}$, the cells were sub-cultured at a 1:2 ratio, and each new vessel was supplemented with fresh expansion medium to a final volume of 5 L before continuing the culture. The cells were harvested after a total culture duration of 10-12 days in the expansion medium in the G-REX500M culture vessels, and natural TIL product cells were obtained.

The seed cell medium and expansion medium used during the culture of each tumor tissue sample, the respective culture durations of the seed cell medium and expansion medium for each tissue sample, and the G-REX500M cell seeding density for each tumor tissue sample were the same as those in Table 2; The types of coated vessels, the density of coated, activated, and resuspended cells, and the duration of coated activation for the corresponding tumor tissue samples in Step 5) were the same as those in Table 3.

The various groups of prepared natural TILs were named T001-TIL, T002-TIL, T003-TIL, T004-TIL, T005-TIL, T006-TIL, T007-TIL, T008-TIL, T009-TIL, T010-TIL, T011-TIL, T012-TIL, T013-TIL and T014-TIL, respectively.

Example 3: Phenotype detection of TILs expressing membrane-anchored IL-7

1. Cell viability rate and positive rate of TILs expressing membrane-anchored IL-7

Cell viability rate of each group was detected by trypan blue staining and counting with a cell counter. The results showed that the cell viability rates of each group of both membrane-anchored IL-7-expressing TILs and natural TILs prepared in example 2 were all above 95%.

Using the extracellular domain of BCMA contained in the extracellular domain of each IL-7-expressing cell prepared above as a label, the proportion of cells positive for exogenous gene expression was detected by flow cytometry. The method is as follows:

1) Cells from each group of T001-1, T002-2, T003-2, T004-1, T005-3, T006-5, T007-6, T008-8, T009-3, T010-4, T003-7, T011-9, T012-9, T013-9, T014-9 were collected, with $1\times10^6$ cells per group, and centrifuged at 800g for 3 minutes; Natural T002-TILs not expressing membrane-anchored IL-7 were used as control cells.

2) The supernatant was discarded, and normal saline was added to each group to resuspend the cells, followed by centrifugation at 800g for 3 minutes;

3) The supernatant was discarded, and 100 $\mu$L of normal saline was added to each tube to resuspend the cells. 2 $\mu$L of BCMA flow antibody (purchased from Biolegend, catalog number.: 357504) was added to each tube, followed by incubation at room temperature for 30 minutes;

4) An appropriate amount of normal saline was added to each group, and the cells were centrifuged at 800g for 3 minutes, with this step repeated twice, and the supernatant was discarded;

5) The cells were resuspended in 400 $\mu$L of normal saline and analyzed by flow cytometry. The results showed that the positive rates of cells in each group are shown in Table 4 below.

Table 4

| Sample Name | Positive rate (%) |
|---|---|
| T001-1 | 49.31 |
| T002-2 | 45.43 |
| T003-2 | 51.22 |
| T004-1 | 40.19 |
| T005-3 | 54.07 |

(continued)

| Sample Name | Positive rate (%) |
|---|---|
| T006-5 | 44.68 |
| T007-6 | 12.35 |
| T008-8 | 42.46 |
| T009-3 | 51.95 |
| T010-4 | 43.77 |
| T003-7 | 15.07 |
| T011-9 | 47.6 |
| T012-9 | 50.1 |
| T013-9 | 45.9 |
| T014-9 | 41.1 |
| T002-TIL | 0.01 |

The results in Table 4 show that when the positive expression of the BCMA extracellular domain was used as the control for TIL samples electroporated with membrane-anchored IL-7, except for T007-6, which used a flexible linker in its structural design, and T003-7, which used a conventional CD8 transmembrane domain structure, the proportion of positive cells in all samples exceeded 40%, and the proportions of positive cells in T003-2, T005-3, and T009-3 exceeded 50%. The positive rates of T007-6 and T003-7 were both below 20%, significantly lower than other samples. Among them, T003-2 had a more obvious advantage over T003-7.

The above results indicate that: 1) When the BCMA extracellular domain or the truncated BCMA extracellular domain as an extracellular tag is connected to the transmembrane domain through a flexible linker, its recognition by the antibody may be affected, failing to truly function as an extracellular tag; 2) Compared with the conventional transmembrane domain, the cytokine IL-7 anchored on the TIL surface through the GPI anchor region can more effectively stimulate the expansion of positive TILs and significantly increase the proportion of positive TILs.

2. Lymphocytic phenotypes and cytokine secretion levels

The harvested TILs expressing membrane-anchored IL-7 and natural TILs from each group were incubated with CD45, CD3, CD4 and CD8 flow cytometry antibodies, and the unbound antibodies were washed and counted on a flow cytometer. The HTRF IFN-$\gamma$ detection kit (Cisbio Human IFN gamma kit, catalog number: 62HIFNGPET) was used to detect the IFN-$\gamma$ secretion level according to the method described in the instruction manual. The results are shown in Table 5 below:

Table 5

| Nos. of TIL | IFN-$\gamma$ (pg/mL) | Ratio of CD45+ cells (%) | Ratio of CD3+ cells to CD45+ cells (%) | Ratio of CD4+ cells to CD45+ cells (%) | Ratio of CD8+ cells to CD45+ cells (%) |
|---|---|---|---|---|---|
| T001-1 | 11025.32 | 99.55 | 97.39 | 39.05 | 60.51 |
| T002-2 | 9712.95 | 99.84 | 98.94 | 12.64 | 82.96 |
| T003-2 | 10258.25 | 100 | 96.95 | 46.97 | 50.84 |
| T004-1 | 10412.15 | 99.87 | 99.70 | 38.71 | 60.87 |
| T005-3 | 11069.89 | 98.97 | 80.98 | 32.48 | 62.87 |
| T006-5 | 10098.47 | 100 | 99.23 | 43.99 | 53.33 |
| T007-6 | 8915.88 | 100 | 97.36 | 45.73 | 48.38 |
| T008-8 | 10730.76 | 100 | 99.43 | 45.21 | 54.23 |
| T009-3 | 10213.74 | 99.99 | 99.25 | 32.59 | 50.65 |
| T010-4 | 9998.82 | 99.99 | 92.59 | 39.09 | 49.77 |

(continued)

| Nos. of TIL | IFN-γ (pg/mL) | Ratio of CD45+ cells (%) | Ratio of CD3+ cells to CD45+ cells (%) | Ratio of CD4+ cells to CD45+ cells (%) | Ratio of CD8+ cells to CD45+ cells (%) |
|---|---|---|---|---|---|
| T003-7 | 8988.05 | 100 | 98.90 | 45.11 | 51.45 |
| T011-9 | 10719.35 | 100 | 99.57 | 50.25 | 45.65 |
| T012-9 | 9853.9 | 99.53 | 95.88 | 44.15 | 49.88 |
| T013-9 | 9484.91 | 100 | 99.34 | 48.99 | 47.43 |
| T014-9 | 11291.71 | 98.94 | 99.09 | 46.76 | 50.24 |
| T001-TIL | 9892.43 | 100 | 93.64 | 50.42 | 45.08 |
| T002-TIL | 10922.79 | 100 | 94.25 | 39.36 | 55.67 |
| T003-TIL | 10103.18 | 99.95 | 99.76 | 32.34 | 52.31 |
| T004-TIL | 12372.01 | 99.25 | 99.34 | 31.23 | 64.78 |
| T005-TIL | 9624.49 | 100 | 94.06 | 40.09 | 49.97 |
| T006-TIL | 13169.76 | 98.38 | 99.61 | 46.94 | 49.35 |
| T007-TIL | 11279.83 | 100 | 98.43 | 48.27 | 47.67 |
| T008-TIL | 12433.24 | 100 | 99.16 | 29.73 | 65.93 |
| T009-TIL | 9669.69 | 97.64 | 99.72 | 38.79 | 60.17 |
| T010-TIL | 9798.61 | 100 | 98.90 | 29.69 | 62.31 |
| T011-TIL | 12488.26 | 99.22 | 98.88 | 42.06 | 51.24 |
| T012-TIL | 11959.74 | 98.61 | 93.29 | 32.58 | 62.88 |
| T013-TIL | 11813.74 | 100 | 96.52 | 47.14 | 50.61 |
| T014-TIL | 13270.95 | 100 | 99.43 | 35.71 | 61.43 |

Example 4. *in vitro* cytotoxicity of TILs expressing membrane-anchored IL-7 against tumor cell

1. Culture of primary tumor cells from melanoma tissue

Tumor tissue from T002 melanoma was used and cut into $3\times3\times3$ mm fragments. After the fragments were mixed as uniformly as possible, primary melanoma cells were obtained by culturing according to the method described in the Materials and Methods section of Robert Suriano et al. Ex Vivo Derived Primary Melanoma Cells: Implications for Immunotherapeutic Vaccines J Cancer 2013; 4(5):371-382.

2. Enhancement of the cytotoxic function of TILs expressing membrane-anchored IL-7 against target cells *in vitro*

The *in vitro* cytotoxic activity of T002-2 and T002-TIL obtained in example 2 against their patient-matched primary melanoma cells was detected using the Real-Time Cell Analysis (RTCA) system from Agilent Technologies. The specific steps are as follows:

(1) Zero setting: 50 μL of DMEM medium was added per well and the plate was placed in the instrument. Step 1 was selected followed by zero setting.
(2) Target cell seeding: The primary melanoma cells obtained from the culture in (1) were seeded at a density of $1\times10^4$ cells/50 μL into plates containing detection electrodes, and stood for several minutes. After the cells had settled, they were then placed into the instrument, and Step 2 was initiated to culture the cells.
(3) Addition of effector cells: After the target cells had been cultured for 18-24 hours, the cell index was read. When the cell index reached 2, effector cells T002-2 and T002-TIL were added at 50 μL per well, respectively. The effector cells were counted based on viable cell numbers, and the effector-to-target ratio was set to 4:1. Step 3 was then initiated, and after 60 hours of co-culture, the cell proliferation curve was observed.

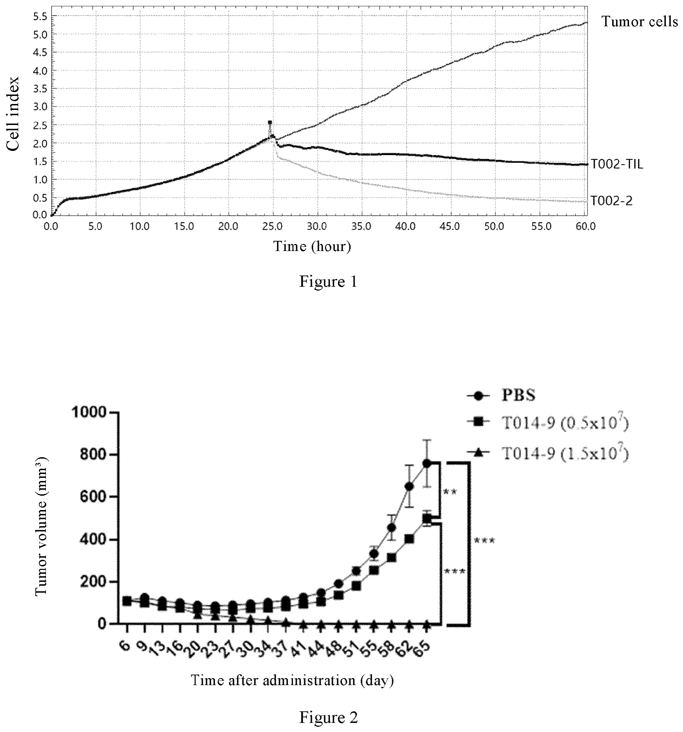

The results, as shown in Figure 1, showed that both T002-TIL and T002-2 exhibited significant cytotoxic effects on their patient-matched melanoma cells compared with the tumor cell group without effector cells. Notably, T002-2 expressing membrane-anchored IL-7 exhibited a more substantial enhancement in target cell killing compared with T002-TIL without exogenous gene expression, indicating that membrane-anchored IL-7 can significantly enhance the cytotoxic capacity of TILs against the tumor cells they recognize.

Example 5: Detection of the *in vitro* molecular brake function of TILs expressing membrane-anchored IL-7

Synthesis of antibody

The BCMA antibody was commercially synthesized, with the heavy chain sequence as shown in SEQ ID NO:29 and the light chain sequence as shown in SEQ ID NO:31 of Chinese Patent CN103562225B, which is incorporated herein by reference in its entirety.

Antibody-dependent cell-mediated cytotoxicity (ADCC) Assay

NK cells were obtained from healthy volunteers' blood using a NK cell culture kit (purchased from T&L Biotechnology Co.,Ltd., catalog NO.: AS-01). T001-1, T002-2, T003-2, T004-1, T005-3, T006-5, T007-6, T008-8, T009-3, T010-4, T003-7, T011-9, T012-9, T013-9, T011-9, T012-9, T013-9, and T014-9 prepared in example 2 were resuspended in AIM-V medium and used as target cells, and T002-TIL and T011-TIL were used as negative control target cells and subjected to the same treatment. The target cells were seeded into 96-well plates at a density of $1.0 \times 10^4$ cells/well/50 $\mu$L. Meanwhile, when the synthesized BCMA antibody was resuspended in AIM-V medium to reach a final concentration of 500 $\mu$g/mL, 10 $\mu$L of this antibody was added to each well, and pre-incubated with target cells in the wells at room temperature for 40 minutes. NK cells, as effector cells, were resuspended in AIM-V medium containing 2% FCS to reach a concentration of $2.5 \times 10^6$ cells/mL. After the 40-minute incubation of target cells with BCMA antibody, 40 $\mu$L of the above NK cell suspension was added to the mixture of target cells and BCMA antibody, resulting in an effector-to-target ratio of 10:1. Meanwhile, the final concentration of BCMA antibody in the system was 50 $\mu$g/mL, with a total volume of the system being 100 $\mu$L. The system was gently pipetted to mix thoroughly, and the plate was incubated in a 5% $CO_2$ incubator at 37°C for 3 hours. The antibody-dependent cellular cytotoxicity (ADCC) effect of BCMA antibody on the above target cells was evaluated using the LDH Lactate Dehydrogenase-Cytotoxicity Detection Assay Kit (LDH-Cytotoxicity Colorimetric Assay Kit, Biovision, Cat. No. K313-500).

Complement-Dependent Cytotoxicity (CDC) Assay

Target cells, including T001-1, T002-2, T003-2, T004-1, T005-3, T006-5, T007-6, T008-8, T009-3, T010-4, T003-7, T011-9, T012-9, T013-9, T011-9, T012-9, T013-9, and T014-9 prepared in Example 2, were re-suspended in AIM-V medium. T002-TIL and T011-TIL were used as negative control target cells and subjected to the same resuspension treatment. These target cells were seeded into the wells of a 96-well plate at a density of $1.0 \times 10^4$ cells/well in a volume of 50 $\mu$L per well. Meanwhile, the previously synthesized BCMA antibody was also re-suspended in AIM-V medium to a final concentration of 500 $\mu$g/mL. Then, 10 $\mu$L of the antibody solution was added to each well of the 96-well plate and pre-incubated with the target cells in the wells at room temperature for 40 minutes. Freshly thawed normal human serum complement (Normal Human Serum Complement, Quidel, Cat. No. A112) was diluted threefold with AIM-V medium. Following the 40-minute incubation of target cells with the BCMA antibody, 40 $\mu$L of the diluted human serum complement was added to a final BCMA antibody concentration of 50 $\mu$g/mL in a total reaction volume of 100 $\mu$L. The system was gently pipetted to ensure thorough mixing, and the plate was then incubated in a 37°C incubator with 5% $CO_2$ for 3 hours. The complement-dependent cytotoxicity (CDC) effects of the complement on each target cell were evaluated using the LDH-Lactate Dehydrogenase-Cytotoxicity Colorimetric Assay Kit (LDH-Cytotoxicity Colorimetric Assay Kit, Biovision, catalog number: K313-500). According to the instructions of the LDH-Lactate Dehydrogenase-Cytotoxicity Colorimetric Assay Kit, the cytotoxicity calculation formula is as follows:

$$\text{Cytotoxicity(\%)} = \frac{test\ sample\ value - low\ control\ value}{high\ control\ value - low\ control\ value} \times 100$$

wherein the low control value refers to the absorbance value corresponding to the LDH content in the supernatant under conditions where cells are not subjected to any lysis treatment; the high control value refers to the absorbance value of the supernatant under conditions where cells are subjected to complete lysis treatment.

The results of ADCC and CDC are shown in Table 6. Following the addition of BCMA antibody, except for T007-6 and T003-7, all other cell samples expressing IL-7 on the membrane surface showed very obvious ADCC and CDC toxicity, which was consistent with the positive rate results in table 4 of example 3. However, natural TILs (T002-TIL and T011-TIL) that do not express IL-7 on the membrane surface showed no obvious ADCC and CDC toxicity. The ADCC and CDC toxicity of T007-6, which contains a flexible linker in its extracellular structure, was significantly lower than that of other samples, indicating that the addition of BCMA antibody failed to sufficiently mediate the occurrence of ADCC and CDC effects. This suggests that when the extracellular domain of BCMA is linked to the transmembrane domain via a flexible linker, its epitope structure may be affected, resulting in the inability of BCMA antibody to bind to it normally.

Table 6

| TIL number | ADCC cell toxicity (%) | CDC cell toxicity (%) |
| --- | --- | --- |
| T001-1 | 42.93 | 44.08 |
| T002-2 | 41.85 | 39.58 |
| T003-2 | 49.03 | 45.12 |
| T004-1 | 37.51 | 35.33 |
| T005-3 | 50.72 | 48.15 |
| T006-5 | 40.35 | 41.03 |
| T007-6 | 10.23 | 9.89 |
| T008-8 | 40.06 | 38.38 |
| T009-3 | 49.19 | 49.71 |
| T010-4 | 42.21 | 40.77 |
| T003-7 | 12.47 | 11.68 |
| T011-9 | 45.03 | 41.55 |
| T012-9 | 45.41 | 47.45 |
| T013-9 | 43.83 | 41.92 |
| T014-9 | 39.43 | 38.43 |
| T002-TIL | 0.09 | 0.13 |
| T011-TIL | 0.03 | 0.11 |

Example 6. *In vivo* tumor cytotoxic function of membrane-anchored IL-7-expressing TILs in a CDX Model

Construction of experimental cells

The SKOV-3 cell line SKOV-3 320 stably expressing human CD3 antibody (OKT3) on its membrane surface was constructed as an ovarian cancer target cell, as follows:

1. Construction of lentiviral vector expressing anti-CD3 antibody on membrane surface

The structure encoding the membrane surface CD3 antibody (αCD3TM) includes a CD8 signal peptide, a CD3 scFv, a linker and a CD8 transmembrane domain, and its amino acid sequence is shown in SEQ ID NO:49, and its DNA sequence is shown in SEQ ID NO:50. A DNA sequence including SEQ ID NO:50 with PmeI and MluI restriction enzyme sites at the 5' and 3' ends, respectively, was commissioned for synthesis. After digestion, this sequence was ligated into the lentiviral vector pWPXL-EGFP expression plasmid (purchased from Addgene) to obtain pWPXL-αCD3TM-EGFP. A DNA sequence encoding the fusion protein (EGFP-puro) of EGFP, a P2A linker, and the puromycin resistance gene (puro) was commissioned for synthesis, with MluI and SpeI restriction sites at the 5' and 3' ends, respectively. After digestion, this sequence was ligated into pWPXL-αCD3TM-EGFP to obtain the lentiviral expression plasmid pWPXL-αCD3TM-EGFP-puro.

2. Preparation of lentivirus

The following packaging plasmids psPAX2 and pMD2.G were purchased from addgene.

1) 293T cells were passaged one day before transfection and seeded into 6-well plates at a cell confluence of 35%;

2) One hour before transfection on the next day, the medium in the 6-well plate was aspirated, and 2 mL of Opti-MEM™ (purchased from ThermoFisher) medium was slowly added to each well;

3) Preparation of plasmid transfection solution:

a) Dilution of Lipofectamine™ 3000 (purchased from ThermoFisher): 24 μL of Lipofectamine™ 3000 was added to 750 μL of Opti-MEM™ and mixed thoroughly;

b) DNA Dilution: The plasmids were added at a ratio of pWPXL-αCD3TM-EGFP-puro:pSPAX2:pMD2.G = 4:3:1, with a total amount of 2.5 μg per well. Specifically, for 6 wells, the amounts of the three plasmids added were 7.5 μg, 5.625 μg, and 1.875 μg, respectively. The corresponding volumes of the plasmids and 30 μL of P3000™ (purchased from ThermoFisher) were added to 750 μL of Opti-MEM™ and mixed thoroughly;

c) The DNA dilution was added to the Lipofectamine™ 3000 dilution and mixed thoroughly;

4) After incubation at room temperature for 13 minutes, 250 μL of the DNA-lipid complex was added dropwise to each well;

5) 6 hours later, the medium in the 6-well plates was aspirated, and 2 mL of complete medium (H-DMEM + 10 v/v% FBS, both purchased from ThermoFisher) was added to each well.

6) 48 hours after transfection, the entire cell supernatant was collected into 15-mL centrifuge tubes, centrifuged at 1,500 rpm for 10 minutes, filtered through a 0.22-μm membrane, aliquoted into 1.5-mL EP tubes (1.5 mL per tube), and stored at -80°C.

3. Lentiviral Infection of Target Cells

The target cells were the ovarian cancer cell line SKOV3-Luc, which stably expresses the luciferase gene, purchased from BPS Bioscience (Cat#78425).

1) One day before infection, SKOV3-Luc cells were passaged and seeded into two wells of a 6-well plate at a cell confluence of 20%

2) On the day of infection, one tube of viral solution was taken from the -80°C freezer and thawed at room temperature.

3) The medium in one of the wells was aspirated, followed by the addition of 1.5 mL of fresh complete medium (IMDM + 15 v/v% FBS + 1× non-essential amino acids NEAA; IMDM and FBS were purchased from ThermoFisher, and 100× non-essential amino acids were purchased from Cyagen) and 1.5 mL of viral solution. Polybrene was then added to a final concentration of 8 μg/mL. After gentle mixing, the plate was incubated in a 37°C $CO_2$ incubator. The other well remained untreated.

4. Screening of positive mixed clones and stable cell lines

1) On the third day of lentiviral infection, the cell confluence in both wells reached more than 90%;

2) The medium was discarded, and 3 mL of fresh complete medium was added to each well, followed by the addition of puromycin to a final concentration of 3 μg/mL;

3) After 48 hours of culture with puromycin, the death rate of the control cells not infected by the virus was close to 100%, and the cell confluence of the infected wells reached 90%; All infected cells were transferred to a T25 culture flask and cultured in medium supplemented with puromycin to a final concentration of 3 μg/mL;

4) After the cell proliferation time became stabilized, the cells were expanded, cryopreserved, and analyzed by flow cytometry for positive rate; The final mixed clone stable transfection cell line was named SKOV3-αCD3TM-EGFP;

5) The xCELLigence RTCA TP real-time label-free cell analyzer (Agilent) was employed to perform cytotoxicity assays on parental SKOV3-Luc cells and the mixed clonal stable cell line SKOV3-αCD3TM-EGFP using healthy human peripheral blood PBMC-derived T cells as effector cells. The results showed that, compared with the unmodified SKOV3-Luc, T cells exerted a significantly stronger cell-killing capacity against SKOV3-αCD3TM-EGFP.

5. Screening of monoclonal cell line

1) The prepared complete medium (IMDM + 15 v/v% FBS + 1× NEAA) was filtered through a 0.22-μm membrane to minimize impurities that might interfere with subsequent single-cell microscopic observation;

2) The mixed clonal stable cell line SKOV3-αCD3TM-EGFP was subjected to cell counting;

3) Two 96-well plates were seeded at a density of 0.8 cells/well: 100 μL of medium was added per well using an 8-channel pipette (maximum volume 50 μL), followed by 50 μL of cell suspension (16 cells/mL, prepare 15 mL), and 1000 cells were added to well A1 of each 96-well plate as a control;

4) After 6 hours of static culture in a 37°C $CO_2$ incubator, cells were observed under a microscope, and the wells containing a single cell was photographed and recorded;

5) Wells containing a single cell was photographed on days 1, 2, and 3 to capture the process of cell division;

6) Medium was replaced for the marked single-cell clones; 100 μL of medium was aspirated from each well, followed by the addition of 150 μL of fresh medium, and the medium was replaced every 3 days;

7) Once the single-cell clones reached >50% confluence, they were expanded sequentially in 96-well plates, 24-well plates, 6-well plates, T25 flasks, and finally T75 flasks;

8) The expanded monoclonal cell lines were analyzed by flow cytometry for transgene expression and cryopreserved for long-term storage;

9) The xCELLigence RTCA TP real-time label-free cell analyzer was employed to assess the cytotoxicity of different monoclonal cell lines. The results showed that monoclonal cell line numbered 320 responded well to T cell-mediated cytotoxicity; additionally, during the initial period, the cell cytotoxicity displayed a cell number-dependent relationship-with an increase in the effector-to-target ratio, the cytotoxic efficacy enhanced. This clone was designated as SKOV-3 320 and used as the tumor target cell for subsequent experiments.

Effector cells: TIL T014-9, derived and cultured from ovarian cancer tissue T014 and expressing membrane-anchored IL-7, were used as effector cells.

Experimental Animals

Immunodeficient B-NDG mice (purchased from Biocytogen) were selected as the experimental animals for the establishment of the CDX model.

Experimental design and grouping: as shown in Table 7 below.

Table 7 Dosing regimen and grouping of mice

| Groups | Drugs | Number of animals | Route of administration | Frequency | Volume (μL) | Time (Days) |
|---|---|---|---|---|---|---|
| 1 | PBS | 6 | tail vein | single | 200 | 65 |
| 2 | T014-9 ($0.5 \times 10^7$ per mouse) | 6 | tail vein | single | 200 | 65 |
| 3 | T014-9 ($1.5 \times 10^7$ per mouse) | 6 | tail vein | single | 200 | 65 |

The TILs were T014-9, a TIL product cell expressing membrane-anchored IL-7, which was prepared in Example 2 and derived from tissue sample T014. The cells were centrifuged and resuspended in PBS before tail vein injection. A cell suspension with a cell density of $2.5 \times 10^7$/mL was prepared for group 2, and a cell suspension with a cell density of $7.5 \times 10^7$/mL was prepared for group 3.

Animal Husbandry

After purchasing the required number of B-NDG mice, they were housed in an SPF-grade laboratory animal facility, with an acclimation period of 7-10 days.

Environment: Mice were housed in transparent resin plastic cages in an animal room. Cage bedding was autoclaved sawdust and corncob bedding, which was changed regularly. The animal room was equipped with a high-efficiency air filter and the temperature was maintained between 20-26°C (68-79°F) and the relative humidity was 40-70%. The temperature and humidity were continuously monitored and recorded. The lighting conditions were 12 hours of fluorescent light and 12 hours of no light per day.

Food and water: experimental mice had *ad libitum* access to specialized mouse chow (irradiated for sterilization, Shanghai Slac Laboratory Animal Co., Ltd., China) and had unimpeded access to sterilized clean drinking water at any time.

Establishment of subcutaneous ectopic tumor model

SKOV-3 320 cells were counted and used for tumor inoculation. Each mouse was subcutaneously inoculated with 0.2 mL of tumor cell solution containing 50% matrix gel (1E7/mouse) in the right axilla, and the day of inoculation was designated as D0.

Animal grouping and drug administration

When the average tumor volume reached 100 mm$^3$, 24 animals were randomly grouped according to tumor volume (mean 111 mm$^3$) from 36 animals to ensure that all groups were comparable at baseline. The day of grouping was recorded as P0. During the experiment, animal body weight and tumor volume were measured 3 times a week, and the clinical symptoms of the animals were observed daily. Tumor volume is expressed in mm$^3$, and the formula is: V = $0.5 \times a \times b^2$, where a and b are the long and short diameters of the tumor, respectively. Seven weeks after the first administration and at the end of the experiment, the tumor fluorescence value was measured using a small animal *In vivo* imaging system.

*In vivo* imaging

On the last day of the experiment, the bioluminescent signal of mice was detected using IVIS Lumina XR (Perkin Elmer). The animals were first injected intraperitoneally with 150 mg/kg sodium fluorescein and anesthetized with isoflurane. Bioluminescent images were collected 10 minutes after the animals were injected with sodium fluorescein, with an exposure time of 60 seconds. Images were quantified as photons/second using Living Image software.

Result

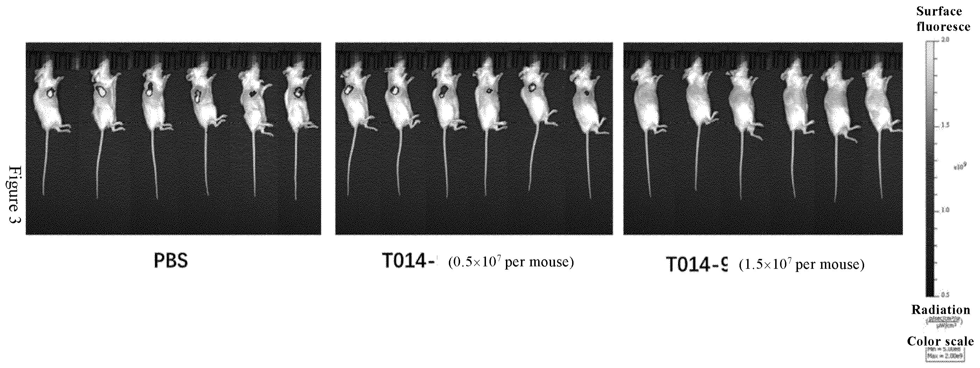

The experimental results are shown in Figure 2 and 3. Figure 2 shows the difference in tumor volume between groups. The difference in tumor volume between different groups was analyzed by one-way ANOVA analysis, followed by post hoc testing using the Bonferroni method (Bonferroni post hoc test) to observe whether there were significant differences between different groups. *: $p<0.05$; **: $p<0.01$; ***: $p<0.001$. Figure 3 shows the results of fluorescent *In vivo* imaging of experimental mice in each group on the last day of the experiment. The results in Figure 2 show that compared with the PBS group, the volume of mouse tumors in the $0.5 \times 10^7$/mouse T014-9 infusion dose group was significantly smaller; while the tumors of mice in the $1.5 \times 10^7$/mouse TIL infusion dose group had completely disappeared by D65. Figure 3 shows that at D65, no tumor cell fluorescence was observed in the $1.5 \times 10^7$/mouse T014-9 infusion dose group. The above results indicate that T014-9 cells have a highly significant cytotoxic effect on ovarian cancer CDX tissues in mice.

Example 7 *In vivo* tumor cytotoxic function of TILs expressing membrane-anchored IL-7 against PDX model

T011 cervical cancer tumor tissue was used to establish a PDX tumor model in immunodeficient mice, and the model mice were used to detect the TIL-PDX animal tumor killing efficacy in vivo. Immunodeficient B-NDG mice (purchased from Biocytogen) were selected as PDX model experimental animals.

Experimental design and grouping: as shown in Table 8 below

Table 8 Dosing regimen and grouping of mice

| Groups | Drugs | Number of animals | Route of administration | Frequency | Dosing Volume (µL) | Time (Days) |
|--------|-------|-------------------|-------------------------|-----------|--------------------|-------------|
| 1 | PBS | 8 | tail vein | single | 200 | 30-42 |
| 2 | T011-TIL ($2 \times 10^7$ per mouse) | 8 | tail vein | single | 200 | 30-42 |
| 3 | T011-9 ($2 \times 10^7$ per mouse) | 8 | tail vein | single | 200 | 30-42 |

The TILs were TIL product cells derived from tissue sample T011 and prepared in Example 2. Before tail vein injection, the cells were centrifuged and re-suspended in PBS to prepare a PBS cell suspension with a cell density of $1 \times 10^8$/mL.

Animal Husbandry

After purchasing the required number of B-NDG mice, they were housed in an SPF-grade laboratory animal facility, with an acclimation period of 7-10 days.

Environment: Mice were housed in transparent resin plastic cages in an animal room. Cage bedding was autoclaved sawdust and corncob bedding, which was changed regularly. The animal room was equipped with a high-efficiency air filter and the temperature was maintained between 20-26°C (68-79°F) and the relative humidity was 40-70%. The temperature and humidity were continuously monitored and recorded. The lighting conditions were 12 hours of fluorescent light and 12 hours of no light per day.

Food and Water: Experimental mice had *ad libitum* access to specialized mouse chow (irradiated for sterilization, Shanghai Slac Laboratory Animal Co., Ltd., China) and had unimpeded access to sterilized clean drinking water at any time.

Establishment of the PDX model

1) Processing of patient tumor tissue samples: A portion of T011 tumor tissue was taken. Under sterile conditions, necrotic tissue, adipose tissue, connective tissue, etc., were removed. After being rinsed thoroughly, the tissue was cut into a number of $5\times5\times5$ mm$^3$ tissue fragments with a scalpel, placed in the tumor sample transport preservation solution UW, and prepared for inoculation of B-NDG mice with the tumor fragments;

2) Inoculation of tumor tissue samples: a number of B-NDG mice were selected. After depilation of the scapular region, the mice were fixed with a mouse subcutaneous tumor inoculation fixator. Following disinfection with povidone-iodine and local anesthesia with lidocaine, the tumor fragments from step 1) were inoculated into the right inguinal region using a PDX model tumor fragment inoculation trocar. The day of inoculation was designated as P0. Tumors were measured twice a week, and the tumor volume was calculated using the formula: $V = 0.5\times a\times b^2$, where a and b represent the longest and shortest diameters of the tumor, respectively;

3) Passaging of PDX tissues: The growth of tumor tissues in each inoculated mouse was observed. Once the volume of tumor tissues exceeded 300 mm$^3$, the mice were anesthetized, and the tumor fragments were excised. Under sterile conditions, the fragments were cut into $5\times5\times5$ mm$^3$ tissue pieces with a scalpel, followed by repetition of Step 2). The tissue pieces were inoculated into the right inguinal region of new mice, and waited for the growth of the next generation of PDX tumors.

4) Step 3) was repeated, and after continuous passaging for 2-3 generations, PDX tissues from some mice were collected for histopathological analysis via tissue sections. Upon confirming that the PDX tissues remained human (rather than murine), mice were further inoculated with PDX tissues at 1.5 times the number of mice used in the experimental plan in Table 8 (i.e., 36 mice were inoculated with PDX tissue fragments). The tumor formation in mice was monitored. The tumors were measured twice a week, and tumor formation was expected.

Animal grouping and drug administration

When the tumor volume of PDX-inoculated mice reaches approximately 50 mm$^3$, 24 animals with appropriate tumor volumes were selected from the 36 animals and randomly grouped according to tumor volume, with n=8 per group, to ensure all groups are comparable at baseline. The day of grouping was designated as D0, and drug administration was performed according to the protocol in Table 8. During the experiment, animal body weight and tumor volume were measured 3 times per week, and clinical symptoms of the animals were observed daily. Tumor volume was expressed in mm$^3$, and the tumor measurement formula was the same as described above.

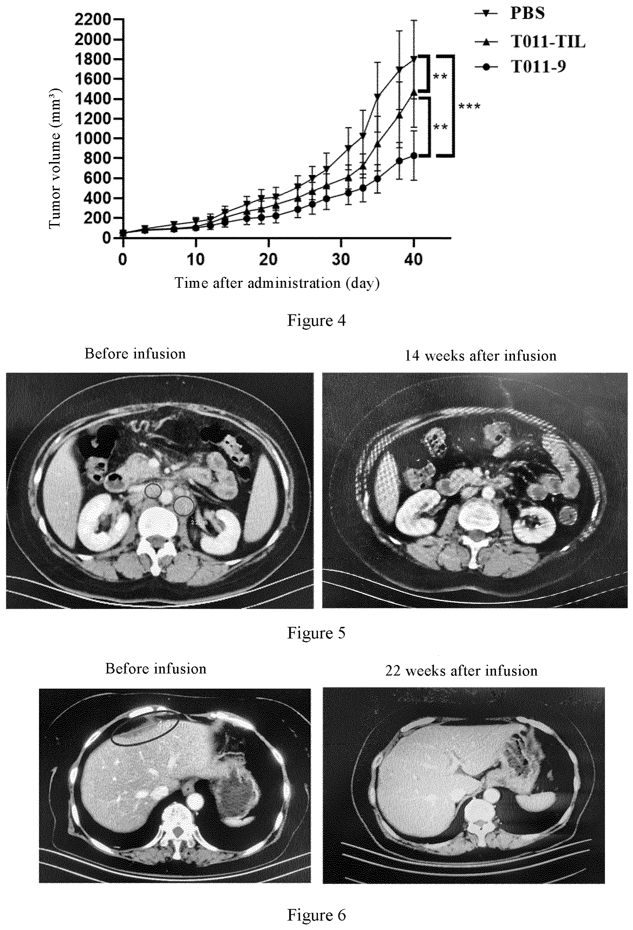

The results are shown in Figure 4. Differences in tumor volume between groups were analyzed using one-way ANOVA (one-way ANOVA analysis), followed by post hoc testing using the Bonferroni method (Bonferroni post hoc test) to assess pairwise significant differences between groups. *: $p < 0.05$; **: $p < 0.01$; ***: $p < 0.001$. The experimental results indicate that by Day 40 after drug administration, compared with tumor-bearing mice in the PBS-injected control group, tumor volume increase in mice of the T011-TIL group was highly significantly inhibited. Meanwhile, T011-9 exerted a more significant inhibitory effect on tumor volume increase compared with the T011-TIL group, indicating that natural TIL product cells derived from T011 exert a highly significant cytotoxic effect on tumor PDX tissues from the same patient as their source in vivo, and modification of natural TILs with membrane-anchored IL-7 can further enhance this effect.

Example 8 Clinical infusion of TILs expressing membrane-anchored IL-7

To verify the clinical safety and efficacy of the TILs expressing membrane-anchored IL-7 described in the present invention, a clinical trial of TIL infusion therapy for solid tumors was conducted. This clinical trial has been approved by the ethics committee of Shanghai Tenth People's Hospital. For details, please refer to www.clinicaltrials.gov, with the registration number: NCT05468307.

Study protocol

1. Subject inclusion, exclusion criteria and allocation method

Inclusion criteria:

1) Age between 18 and 75 years (inclusive);
2) Subjects with pathologically diagnosed primary/recurrent/metastatic gynecological malignancies;
3) Life expectancy > 3 months;
4) Karnofsky $\geq$ 60% or ECOG score of 0 - 2;
5) Subjects who have currently failed standard treatment or have no available standard treatment;
6) Subjects must have tumor sites suitable for biopsy or resection, or malignant effusions from which TILs can be isolated;
7) At least 1 evaluable tumor lesion;
8) Hematological and biochemical indicators (within 7 days before enrollment)

$$\text{Absolute white blood cell count} \geqslant 2.5 \times 10^9/\text{L};$$

$$\text{Absolute neutrophil count} \geqslant 1.5 \times 10^9/\text{L};$$

$$\text{Absolute lymphocyte count} \geqslant 0.7 \times 10^9/\text{L};$$

$$\text{Platelet count} \geqslant 100 \times 10^9/\text{L};$$

$$\text{Hemoglobin} \geqslant 90 \text{ g/L};$$

Activated partial thromboplastin time (APTT) $\leq 1.5 \times$ ULN (except for subjects who received anticoagulant therapy within the previous 3 days);

International normalized ratio (INR) $\leq 1.5 \times$ ULN (except for subjects who received anticoagulant therapy within the previous 3 days);

Serum creatinine $\leq 1.5$ mg/dL (or $\leq 132.6 \, \mu$ mol/L), or serum creatinine clearance $\geq 50$ mL/min;

Serum ALT (alanine transaminase)/AST (aspartate transaminase) $\leq 3 \times$ ULN (for subjects with liver metastasis, $\leq 3 \times$ ULN);

$$\text{Total bilirubin} \leqslant 1.5 \times \text{ULN};$$

9) No absolute or relative contraindications to surgery or puncture;
10) Subjects with reproductive potential must agree to use approved highly effective contraceptive methods starting from the time of informed consent and continue for 1 year after completion of the lymphodepletion regimen;
11) Any anti-malignancy treatments, including radiotherapy, chemotherapy, and biological agents, must have been discontinued at least 28 days before TILs collection;
12) Adequate understanding ability and voluntary signing of the informed consent form;
13) Able to comply with the follow-up schedule and other protocol requirements.

Exclusion criteria:

1) Requiring glucocorticoid therapy with a daily dose exceeding 15 mg prednisone (or equivalent dose of other steroids), or having autoimmune diseases requiring immunosuppressive therapy;
2) Forced expiratory volume in 1 second (FEV1) < 2 L, or corrected diffusing capacity for carbon monoxide (DLCO) < 40%;
3) Any of the following definitions of significant cardiovascular abnormalities: New York Heart Association (NYHA) class III or IV congestive heart failure, clinically significant hypotension, clinically uncontrolled hypertension, uncontrolled symptomatic coronary artery disease, or ejection fraction < 35%; severe cardiac rhythm and conduction abnormalities (e.g., ventricular arrhythmias requiring clinical intervention, second- or third-degree atrioventricular block, etc.);
4) Subjects with human immunodeficiency virus (HIV) infection or positive HIV antibody test, active hepatitis B or C virus infection (HBsAg-positive and/or anti-HCV-positive), syphilis infection or positive Treponema pallidum antibody;
5) Severe physical or psychiatric illnesses;
6) Having active systemic infection requiring treatment, or positive blood cultures (or radiographic evidence of infection);
7) Having received or currently receiving other drugs, or other biological, chemotherapy, or radiotherapy within 1 month;
8) History of allergic reactions to compounds with chemical or biological compositions similar to those of the cell therapy;
9) Previous immunotherapy with irAE grade $\geq$ 3;
10) Adverse reactions from prior anti-tumor treatments have not resolved to CTCAE version 5.0 grade $\leq$ 1 (except for toxicities such as alopecia, which investigators judge to pose no safety risk);
11) Pregnant or lactating women;
12) History of organ transplantation, allogeneic stem cell transplantation, or kidney replacement therapy;
13) Investigators judge that subjects have other severe systemic disease histories or other reasons making them unsuitable for participation in this clinical study.

2. Efficacy evaluation criteria

Primary efficacy indicators:

1) Objective Response Rate (ORR);
2) Disease Control Rate (DCR);
3) Duration of Response (DOR);
4) Progression-Free Survival (PFS);
5) Overall Survival (OS).

Secondary efficacy indicators:

1) Clinical efficacy assessment: Complete Response (CR), Partial Response (PR), Stable Disease (SD), Progressive Disease (PD), etc.;
2) Changes in quality of life before and after treatment.

Efficacy is defined as the investigator-observed ORR, which is based on the criteria of RECIST v1.1.

3. Recording and management measures for adverse events

3.1 Definition of adverse events

Adverse Event (AE): Adverse medical events that occur between the signing of informed consent and enrollment in the trial and the last medical follow-up can manifest as symptom signs, diseases, or laboratory abnormalities, but are not necessarily causally related to the treatment or trial drug.

A new condition or worsening of an existing condition is considered an AE. Stable chronic diseases present before enrollment that do not worsen during the study (e.g., arthritis) are not considered AEs. Abnormal laboratory findings, clinical symptoms, or signs judged by the investigator to be clinically significant are considered as AEs.

Serious Adverse Event (SAE): An adverse event occurring during the trial that meets one or more of the following conditions: (1) resulting in death; (2) life-threatening referring to an immediate risk of death for the subject, not a hypothetical risk of death if it develops seriously in the future; (3) resulting in hospitalization or prolongation of existing hospitalization; (4) permanent or significant loss of function; (5) teratogenicity, birth defects; (6) other important medical events.

3.2 Criteria for evaluating the severity of adverse events

The severity of all adverse events occurring during the study will be evaluated according to the National Cancer Institute Common Terminology Criteria for Adverse Events (NCI-CTCAE) Version 5.0, classified into Grades 1 to 5:

☐Grade 1: Mild; asymptomatic or mild symptoms; clinical or diagnostic findings only; no intervention needed.

☐Grade 2: Moderate; requires minor, local, or non-invasive intervention; limited instrumental activities of daily living (IADL) appropriate for age.

☐Grade 3: Severe or medically significant but not immediately life-threatening; results in hospitalization or prolongation of hospitalization; disabling; limited activities of daily living (ADL).

☐Grade 4: Life-threatening; requires urgent intervention.

☐Grade 5: Death related to the adverse event.

4. Pretreatment before reinfusion

Before TIL infusion, patients received a lymphocyte pretreatment regimen (hydroxychloroquine 600-800 mg × 1 day, cyclophosphamide 20-25 mg/kg/d × 3 days). The researchers would make adjustment to the drugs, dosages, and days (frequency of administration) used in the pretreatment regimen based on the actual conditions of the patients.

The day of infusion was defined as day 0. Before TIL infusion, researchers needed to perform the following chemotherapy pretreatment on patients (patients will experience leukopenia after pretreatment, and attention should be paid to infection prevention, such as avoiding unnecessary flow of personnel in the ward, and patients wearing masks, etc.):

Day -5 intravenous injection of cyclophosphamide (Cy) 20-25mg/kg + oral hydroxychloroquine (HCQ) 600-800mg
Day -4 intravenous injection of cyclophosphamide 20-25mg/kg
Day -3 intravenous injection of cyclophosphamide 20-25mg/kg
Days -2, -1 patient rest
Day 0 TIL infusion.

5. TIL infusion and efficacy evaluation

The patients were reinfused on day 0 after the above 4 pretreatments were completed before the reinfusion. Each patient received a single infusion, with a infusion dose of $1.0 \times 10^9$-$5.0 \times 10^9$ TILs, a cell density of $1.0 \times 10^7$/mL-$5.0 \times 10^7$/mL, and a volume of 100mL. The patients underwent TIL infusion on day 0, and no cytokines were administered after the infusion. The patients needed to be observed in the hospital post-infusion for 5-8 days, and the conditions of the patients after infusion were observed and recorded. With the informed consent of the patients, peripheral blood was drawn from the patients at different time points to detect the composition and changes of PBMCs, and efficacy evaluation using MRI imaging was performed 1-6 months post-infusion.

6. Results of clinical infusion of TILs

The comprehensive information regarding the clinical infusion and efficacy evaluation results of some subjects who participated in the infusion are shown in table 9 and table 10 below (the evaluation time ranged from 1 to 6 months after the infusion).

Table 9 Comprehensive information of TIL clinical reinfusion in patients

| Number | Tumor type | Amount of infused TILs | Pretreatment before infusion | Clinical efficacy |
|---|---|---|---|---|
| T011 | cervical cancer | $1.52 \times 10^{10}$ | Cy 20mg/kg+ HCQ 600mg | CR |
| T012 | ovarian cancer | $1.67 \times 10^{9}$ | Cy 25mg/kg+ HCQ 600mg | CR |
| T013 | cervical cancer (liver and lung metastasis) | $4.97 \times 10^{9}$ | Cy 25mg/kg+ HCQ 600mg | PR |
| T014 | ovarian cancer (me-senteric metastasis) | $2.89 \times 10^{9}$ | Cy 20mg/kg+ HCQ 800mg | primary lesion CR, overall PR |

The tumor diameter of patient T011 was 2.2 cm before infusion. 14 weeks after infusion, the tumor lesions could not be detected by nuclear magnetic resonance imaging, and T011 was determined as complete response (CR), as shown in Figure 5.

The tumor diameter of patient T012 was 4.5 cm before infusion. 22 weeks after infusion, the tumor lesions could not be detected by nuclear magnetic resonance imaging, and T012 was determined as partial response (CR), as shown in Figure 6.

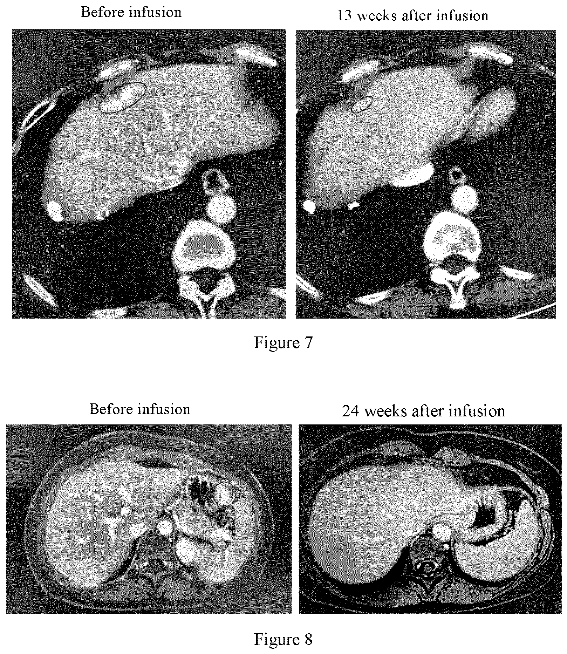

The tumor diameter of patient T013 was 2.1 cm before infusion. 13 weeks after infusion, the tumor lesions were significantly reduced as detected by nuclear magnetic resonance imaging, and T013 was determined as partial response (PR), as shown in Figure 7.

The tumor diameter of patient T014 was 2.6 cm × 2.5 cm before infusion. 24 weeks after infusion, the primary lesions could not be detected by nuclear magnetic resonance imaging, the primary lesions were determined as complete response (CR), and the comprehensive assessment conclusion was partial response (PR), as shown in Figure 8.

Although the specific embodiments of the present invention have been described in detail, it will be understood by those skilled in the art that various modifications and substitutions may be made to those details according to all the teachings disclosed, and these changes are within the scope of protection of the present invention. The full scope of the present invention is given by the appended claims and any equivalents thereof.

Partial sequences in this description •

mbIL-7-1
SEQ ID NO:31

MKRFLFLLLLTISLLVMVQIQTGLSDCDIEGKDGKQYESVLMVSIDQLLDSMKEIGSNCLNNEF
NFFKRHICDANKEGMFLFRAARKLRQFLKMNSTGDFDLHLLKVSEGTTILLNCTGQVKGRKPAAL
GEAQPTKSLEENKSLKEQKKLNDLCFLKRLLQEIKTCWNKILMGTKEHAEAAAKEAAAKAMLQM
AGQCSQNEYFDSLLHACIPCQLRCSSNTPPLTCQRYCNASVTNSVKGTNAGQNDTSQTSSPSA
SSNISGGIFLFFVANAIIHLFCFS

mbIL-7-2
SEQ ID NO:33

MKRFLFLLLLTISLLVMVQIQTGLSDCDIEGKDGKQYESVLMVSIDQLLDSMKEIGSNCLNNEF
NFFKRHICDANKEGMFLFRAARKLRQFLKMNSTGDFDLHLLKVSEGTTILLNCTGQVKGRKPAAL
GEAQPTKSLEENKSLKEQKKLNDLCFLKRLLQEIKTCWNKILMGTKEH
RADAAPTVSMLQMAGQCSQNEYFDSLLHACIPCQLRCSSNTPPLTCQRYCNASVTNSVKG
TNAGQNDTSQTSSPSASSNISGGIFLFFVANAIIHLFCFS

mbIL-7-3

SEQ ID NO:35

MKRFLFLLLTISLLVMVQIQTGLSDCDIEGKDGKQYESVLMVSIDQLLDSMKEIGSNCLNNEF
NFFKRHICDANKEGMFLFRAARKLRQFLKMNSTGDFDLHLLKVSEGTTILLNCTGQVKGRKPAAL
GEAQPTKSLEENKSLKEQKKLNDLCFLKRLLQEIKTCWNKILMGTKEHRTVAAPSVFMLQMAGQ
CSQNEYFDSLLHACIPCDLRCSSNTPPLTCQRYCNASVTNSVKGTNAGQNDTSQTSSPSASSNI
SGGIFLFFVANAIIHLFCFS

mbIL-7-4
SEQ ID NO:37

MKRFLFLLLTISLLVMVQIQTGLSDCDIEGKDGKQYESVLMVSIDQLLDSMKEIGSNCLNNEF
NFFKRHICDANKEGMFLFRAARKLRQFLKMNSTGDFDLHLLKVSEGTTILLNCTGQVKGRKPAAL
GEAQPTKSLEENKSLKEQKKLNDLCFLKRLLQEIKTCWNKILMGTKEHPSPLFPGPSKPMLQMA
GQCSQNEYFDSLLHACIPCDLRCSSNTPPLTCQRYCNAFVPVFLPAKPTTTPAPRPPTPAPTIAS
QPLSLRPEACRPAAGGAVHTRGLDFACDGQNDTSQTSSPSASSNISGGIFLFFVANAIIHLFCFS

mbIL-7-5
SEQ ID NO:39

MKRFLFLLLTISLLVMVQIQTGLSDCDIEGKDGKQYESVLMVSIDQLLDSMKEIGSNCLNNEF
NFFKRHICDANKEGMFLFRAARKLRQFLKMNSTGDFDLHLLKVSEGTTILLNCTGQVKGRKPAAL
GEAQPTKSLEENKSLKEQKKLNDLCFLKRLLQEIKTCWNKILMGTKEHMLQMAGQCSQNEYFD
SLLHACIPCDLRCSSNTPPLTCQRYCNASVTNSVKGTNARTVAAPSVFGQNDTSQTSSPSASSN
ISGGIFLFFVANAIIHLFCFS

mbIL-7-6
SEQ ID NO:41

MKRFLFLLLTISLLVMVQIQTGLSDCDIEGKDGKQYESVLMVSIDQLLDSMKEIGSNCLNNEF
NFFKRHICDANKEGMFLFRAARKLRQFLKMNSTGDFDLHLLKVSEGTTILLNCTGQVKGRKPAAL
GEAQPTKSLEENKSLKEQKKLNDLCFLKRLLQEIKTCWNKILMGTKEHGGGGSGGGGMLQMAG
QCSQNEYFDSLLHACIPCQLRCSSNTPPLTCQRYCNASVTNSVKGTNAGQNDTSQTSSPSASS
NISGGIFLFFVANAIIHLFCFS

mbIL-7-7
SEQ ID NO:43

MKRFLFLLLTISLLVMVQIQTGLSDCDIEGKDGKQYESVLMVSIDQLLDSMKEIGSNCLNNEF
NFFKRHICDANKEGMFLFRAARKLRQFLKMNSTGDFDLHLLKVSEGTTILLNCTGQVKGRKPAAL
GEAQPTKSLEENKSLKEQKKLNDLCFLKRLLQEIKTCWNKILMGTKEHRADAAPTVSMLQMAGQ
CSQNEYFDSLLHACIPCQLRCSSNTPPLTCQRYCNASVTNSVKGTNAIYIWAPLAGTCGVLLLSL
VITLYC

mbIL-7-8
SEQ ID NO:45

MFHVSFRYIFGLPPLILVLLPVASSDCDIEGKDGKQYESVLMVSIDQLLDSMKEIGSNCLNNE
FNFFKRHICDANKEGMFLFRAARKLRQFLKMNSTGDFDLHLLKVSEGTTILLNCTGQVKGRKPA
ALGEAQPTKSLEENKSLKEQKKLNDLCFLKRLLQEIKTCWNKILMGTKEHRADAAPTVSMLQMA
GQCSQNEYFDSLLHACIPCQLRCSSNTPPLTCQRYCNAGQNDTSQTSSPSASSNISGGIFLFFV
ANAIIHLFCFS

mbIL-7-9
SEQ ID NO:47

MKRFLFLLLTISLLVMVQIQTGLSDCDIEGKDGKQYESVLMVSIDQLLDSMKEIGSNCLNNEF
NFFKRHICDANKEGMFLFRAARKLRQFLKMNSTGDFDLHLLKVSEGTTILLNCTGQVKGRKPAAL
GEAQPTKSLEENKSLKEQKKLNDLCFLKRLLQEIKTCWNKILMGTKEHPSPLFPGPSKPMLQMA
GQCSQNEYFDSLLHACIPCDLRCSSNTPPLTCQRYCNAGQNDTSQTSSPSASSNISGGIFLFFV
ANAIIHLFCFS

Membrane-surface CD3 scFv
SEQ ID NO:49

MALPVTALLLPLALLLHAARPQVQLQESGAELARPGASVKMSCKASGYTFTRYTMHWVKQ
RPGQGLEWIGYINPSRGYTNYNQKFKDKATLTTDKSSSTAYMQLSSLTSEDSAVYYCARYYDDH
YCLDYWGQGTTLTVSSGGGGSGGGGSGGGGSDIVLTQSPAIMSASPGEKVTMTCSASSSVSY
MNWYQQKSGTSPKRWIYDTSKLASGVPAHFRGSGSGTSYSLTISGMEAEDAATYYCQQWSSN
PFTFGSGTKLEINRRADAAPTVSIYIWAPLAGTCGVLLLSLVITLYC

## Claims

1. A modified TIL that expresses membrane-anchored IL-7,
   preferably, the membrane-anchored IL-7 comprises IL-7 or a functional fragment thereof, and a transmembrane domain.

2. The modified TIL according to claim 1, wherein,

   the transmembrane domain is a protein transmembrane domain; preferably, the protein transmembrane domain is any one selected from the group consisting of: CD8 transmembrane domain, CD28 transmembrane domain, and IL-7Rα transmembrane domain, or
   the transmembrane domain is a GPI anchor domain; preferably, the GPI anchor domain comprises one or more membrane anchor domains of GPI-anchored proteins selected from the group consisting of: CD44, CD56, CD73, CD55, Thy1, AchE, IAP, ALPP, CD59, CD14, CD16, CD24, CD28, CD48, CD52, CD58, CD66a, CD66c, CD66d, CD66e, CD67, CD87, CD108, CD157, uPAR, JMH protein, GDNFR, CNTFR, TAG-1, PrP, phosphatidylinositol protein, semaphorin 7, CEA, GFR, Ly6G, transferrin receptor, contactin (F3), and T-cadherin.

3. The modified TIL according to claim 1, wherein the membrane-anchored IL-7 further comprises a membrane surface tag,

   preferably, the membrane surface tag comprises an extracellular domain of a B cell surface antigen or a variant thereof that retains the function of binding to antibody,
   more preferably, the membrane surface tag is an extracellular domain of BCMA or a variant thereof that retains the function of binding to anti-BCMA antibody,
   more preferably, the variant is a truncated variant, insertion variant, deletion variant, substitution variant, or a combination thereof; more preferably, the amino acid sequence of the variant is SEQ ID NO:23 or SEQ ID NO:25.

4. The modified TIL according to claim 3, wherein:

the membrane-anchored IL-7 further comprises a linker positioned between IL-7 and the membrane surface tag; preferably, the linker is a rigid linker or a flexible linker, preferably a rigid linker, and/or
the membrane-anchored IL-7 further comprises a hinge region or linker positioned at the N-terminus of the transmembrane domain; preferably, the hinge region includes but is not limited to: the CD4 extracellular hinge region, the CD8 extracellular hinge region, the CD28 extracellular hinge region, the IgG1 Fc extracellular hinge region, and the IgG4 Fc extracellular hinge region, and the linker is a rigid linker or a flexible linker; more preferably, the sequence of the rigid linker is selected from any one or more of SEQ ID NOs: 9, 11, 15, and 17; the sequence of the flexible linker is SEQ ID NO: 13;
the membrane-anchored IL-7 further comprises a signal peptide; preferably, the signal peptide is the CD52 signal peptide or the IL-7 signal peptide.

5. The modified TIL according to any one of claims 1 to 3, wherein the membrane-anchored IL-7 comprises, sequentially linked: an optional signal peptide, IL-7, an optional linker, an extracellular domain of BCMA or a variant thereof, an optional hinge region or linker, and a transmembrane domain, wherein the signal peptide is the CD52 signal peptide or the IL-7 signal peptide, and the transmembrane domain is the CD8 transmembrane domain or CD52,

preferably, the membrane-anchored IL-7 comprises, sequentially linked: a signal peptide, IL-7, a linker, an extracellular domain of BCMA or a variant thereof, and a transmembrane domain,
more preferably, the membrane-anchored IL-7 comprises, sequentially linked:

a CD52 signal peptide, IL-7, a linker, an extracellular domain of BCMA or a variant thereof, and the CD8 transmembrane domain;
a CD52 signal peptide, IL-7, a linker, an extracellular domain of BCMA or a variant thereof, and CD52;
a CD52 signal peptide, IL-7, a linker, an extracellular domain of BCMA or a variant thereof, a hinge region or linker, and CD52;
a CD52 signal peptide, IL-7, an extracellular domain of BCMA or a variant thereof, a hinge region or linker, and CD52;
an IL-7 signal peptide, IL-7, a linker, an extracellular domain of BCMA or a variant thereof, and the CD8 transmembrane domain; or
an IL-7 signal peptide, IL-7, a linker, an extracellular domain of BCMA or a variant thereof, and CD52.

6. The modified TIL according to any one of claims 1 to 3, wherein:

the TIL is prepared by a method comprising incubating a TIL-containing sample with a TIL seed cell medium to obtain a TIL population, or
the TIL is prepared by a method comprising: (1) incubating a TIL-containing sample with a TIL seed cell medium to obtain a first TIL population, and (2) incubating the first TIL population with a TIL expansion medium to obtain a second TIL population.

7. The modified TIL according to claim 6, wherein the sample is selected from the group consisting of: ascites, surgically resected primary tumor samples, synchronous and metachronous surgically resected metastatic tumor samples, biopsy samples, and body fluids,

preferably, the sample is a tumor cell-containing tissue; more preferably, the tumor cells are derived from tumors selected from the group consisting of: melanoma, glioma, thyroid tumor, gastric cancer, lung cancer, gastrointestinal stromal tumor (GIST), intestinal cancer, liver cancer, cervical cancer, ovarian cancer, breast cancer, endometrial stromal sarcoma, pelvic poorly differentiated adenocarcinoma, gallbladder cancer, cholangiocarcinoma, head and neck cancer, colorectal cancer, glioblastoma, pancreatic cancer, bladder cancer, prostate cancer, renal cancer, and osteosarcoma,
preferably, the body fluids include blood, pleural effusion, interstitial fluid, lymphatic fluid, and/or ascites.

8. The modified TIL according to claim 6, wherein the TIL seed cell medium comprises the following components: cell culture components, cytokines, and immune checkpoint antibodies or antigen-binding fragments thereof, wherein the cytokines comprise IL-2, the immune checkpoints include PD-1, LAG-3, TIGIT, and/or CTLA-4, and the cell culture components are a serum-containing medium or a serum-free medium,

preferably, the TIL seed cell medium further comprises serum and/or antibiotics;

more preferably, the TIL seed cell medium comprises any one of the following combinations of components: IL-2, IL-6, IL-21, IFN-γ, TIGIT antibody, anti-PD-1 antibody, TNF-α, and a basal medium; 2) IL-2, IL-4, IL-10, IL-21, anti-CD137 antibody, anti-LAG3 antibody, anti-PD-1 antibody, TNF-α, and a basal medium; 3) IL-2, IL-7, IL-12, IL-21, anti-CD137 antibody, anti-CD28 antibody, anti-PD-1 antibody, and a basal medium; 4) IL-1β, IL-2, IL-7, G-CSF, GM-CSF, IFN-γ, anti-LAG3 antibody, anti-PD-1 antibody, TNF-α, and a basal medium; 5) IL-2, IL-4, IL-12, GM-CSF, M-CSF, IFN-β, IFN-γ, anti-TIGIT antibody, anti-CTLA-4 antibody, and a basal medium; 6) IL-2, IL-7, IL-15, GM-CSF, anti-CD137 antibody, anti-PD-1 antibody, TNF-α, and a basal medium; 7) IL-2, IL-4, IL-10, IL-15, G-CSF, M-CSF, anti-CD28 antibody, anti-OX-40 antibody, anti-PD-1 antibody, and a basal medium; 8) IL-2, IL-7, IL-15, IFN-γ, anti-CD137 antibody, anti-CD40 antibody, anti-OX-40 antibody, anti-TIGIT antibody, anti-PD-1 antibody, and a basal medium; 9) IL-2, IL-7, IL-15, GM-CSF, IFN-γ, anti-CD137 antibody, anti-CD28 antibody, anti-PD-1 antibody, TNF-α, and a basal medium; 10) IL-2, IL-7, IL-12, G-CSF, GM-CSF, IFN-α, IFN-γ, anti-CD28 antibody, anti-CD40 antibody, anti-TIGIT antibody, anti-PD-1 antibody, TNF-α, and a basal medium; 11) IL-2, IL-7, IL-15, GM-CSF, anti-PD-1 antibody, RRx-001, CAL-101, and a basal medium; 12) IL-2, IL-7, IL-15, GM-CSF, M-CSF, anti-PD-1 antibody, CNI-1493, and a basal medium; 13) IL-2, IL-7, IL-15, anti-CD137 antibody, anti-CD28 antibody, anti-LAG3 antibody, anti-PD-1 antibody, dasatinib, and a basal medium; 14) IL-2, IL-6, IL-12, G-CSF, M-CSF, IFN-β, IFN-γ, anti-CTLA-4 antibody, anti-PD-1 antibody, dasatinib, LYC-55716, GENE-1858, and a basal medium; 15) IL-1α, IL-2, IL-9, IL-15, GM-CSF, anti-CD137 antibody, anti-CD28 antibody, anti-LAG3 antibody, anti-TIGIT antibody, CNI-1493, and a basal medium; 16) IL-2, IL-7, IL-12, IL-15, IL-21, G-CSF, M-CSF, IFN-γ, anti-CD28 antibody, anti-CD40 antibody, anti-LAG3 antibody, anti-PD-1 antibody, CNI-1493, dasatinib, GNE-1858, and a basal medium.

**9.** The modified TIL according to claim 6, wherein the TIL expansion medium comprises IL-2, IL-7, IL-15, and immune checkpoint antibodies or antigen-binding fragments thereof, preferably, the immune checkpoint antibodies include anti-PD-1 antibody,

preferably, the TIL expansion medium further comprises one or more of serum, platelets, antibiotics, and a basal medium,

more preferably, the TIL expansion medium comprises any one of the following combinations of components: 17) IL-2, IL-7, IL-15, anti-PD-1 antibody, IL-21, IL-12, anti-CD3 antibody, anti-CD28 antibody, and GM-CSF; 18) IL-2, IL-7, IL-15, anti-PD-1 antibody, IL-21, IL-12, anti-CD3 antibody, anti-CD28 antibody, anti-CD137 antibody, and anti-GITR antibody; 19) IL-2, IL-7, IL-15, anti-PD-1 antibody, anti-CD3 antibody, and anti-CD28 antibody, preferably, the anti-CD3 antibody and anti-CD28 antibody are coupled to a matrix; 20) IL-2, IL-7, IL-15, anti-PD-1 antibody, IL-21, IL-12, anti-CD3 antibody, anti-CD28 antibody, and GM-CSF, preferably, the anti-CD3 antibody and anti-CD28 antibody are coupled to a matrix; 21) IL-2, IL-7, IL-15, anti-PD-1 antibody, anti-CD3 antibody, anti-CD28 antibody, and anti-CD137 antibody, preferably, the anti-CD3 antibody, anti-CD137 antibody, and anti-CD28 antibody are coupled to a matrix; 22) IL-2, IL-7, IL-15, anti-PD-1 antibody, anti-GITR antibody, anti-CD3 antibody, anti-CD28 antibody, TWS119, and anti-CD137 antibody, preferably, the anti-CD3 antibody, anti-CD137 antibody, and anti-CD28 antibody are coupled to a matrix; 23) IL-2, IL-7, IL-15, anti-PD-1 antibody, anti-TIGIT antibody, anti-CD3 antibody, anti-CD28 antibody, anti-CD137 antibody, and GM-CSF, preferably, the anti-CD3 antibody, anti-CD137 antibody, and anti-CD28 antibody are coupled to a matrix; 24) IL-2, IL-7, IL-15, anti-LAG3 antibody, IL-21, IL-12, anti-CD3 antibody, anti-CD28 antibody, anti-CD137 antibody, and anti-GITR antibody, preferably, the anti-CD3 antibody, anti-CD137 antibody, and anti-CD28 antibody are coupled to a matrix; 25) IL-2, IL-7, IL-15, anti-PD-1 antibody, anti-TIGIT antibody, anti-CD3 antibody, anti-CD28 antibody, anti-CD137 antibody, anti-CD40 antibody, anti-OX-40 antibody, and autologous platelets, preferably, the anti-CD3 antibody, anti-CD137 antibody, and anti-CD28 antibody are coupled to a matrix; 26) IL-2, IL-7, IL-15, anti-PD-1 antibody, anti-CTLA-4 antibody, anti-CD3 antibody, anti-CD28 antibody, anti-CD137 antibody, anti-GITR antibody, GM-CSF, TWS119, and allogeneic platelets, preferably, the anti-CD3 antibody and anti-CD28 antibody are coupled to a matrix.

**10.** The modified TIL according to claim 8 or 9, wherein,

the basal medium is any one or more selected from the group consisting of: AIM-V, X-VIVO, DMEM, RPM11640, OpTmizer™, and FUJIFILM Irvin MHM-C; and/or

the serum is selected from human AB serum, the subject's autologous serum, or animal-derived serum.

**11.** A method for preparing the modified TIL according to any one of claims 1 to 9, comprising introducing a nucleic acid encoding the membrane-anchored IL-7 into TILs,

preferably, the introduction is via electroporation or liposomal nanoparticle-mediated introduction,
preferably, the encoding nucleic acid is an expression vector, including a viral vector or a non-viral vector,
preferably, the expression vector comprises a nucleic acid sequence encoding the membrane-anchored IL-7 as described in any one of claims 1 to 5,
more preferably, the viral vector is a lentiviral vector, and the non-viral vector is a transposon system-based vector; further more preferably, the non-viral vector is a vector based on the PiggyBac transposon system or the Sleeping Beauty transposon system.

12. The method for preparing the modified TIL according to claim 11, wherein the TIL is prepared by the method for preparing TIL described in any one of claims 6 to 8,
preferably, the method for preparing the modified TIL comprises: 1) incubating a TIL-containing sample with a TIL seed cell medium to obtain a first TIL population; 2) introducing the nucleic acid construct expressing membrane-anchored IL-7 into the first TIL population via electroporation or liposomal nanoparticles to obtain a second TIL population; and 3) incubating the second TIL population with a TIL expansion medium to obtain a third TIL population.

13. The modified TIL prepared by the method for preparing the modified TIL according to claim 11 or 12.

14. Use of the modified TIL according to any one of claims 1 to 9 in the manufacture of a medicament for preventing or treating tumors,
preferably, the tumors are one or more selected from the group consisting of: melanoma, glioma, thyroid tumor, gastric cancer, lung cancer, gastrointestinal stromal tumor, intestinal cancer, liver cancer, cervical cancer, ovarian cancer, breast cancer, endometrial stromal sarcoma, pelvic poorly differentiated adenocarcinoma, gallbladder cancer, cholangiocarcinoma, head and neck cancer, colorectal cancer, glioblastoma, pancreatic cancer, bladder cancer, prostate cancer, renal cancer, and osteosarcoma.

15. A method for preventing or treating a tumor, comprising administering the modified TIL according to any one of claims 1 to 9 to a subject in need thereof,
preferably, the tumor is selected from one or more of the following: melanoma, glioma, thyroid tumor, gastric cancer, lung cancer, gastrointestinal stromal tumor, intestinal cancer, liver cancer, cervical cancer, ovarian cancer, breast cancer, endometrial stromal sarcoma, pelvic poorly differentiated adenocarcinoma, gallbladder cancer, cholangiocarcinoma, head and neck cancer, colorectal cancer, glioblastoma, pancreatic cancer, bladder cancer, prostate cancer, renal cancer, and osteosarcoma.

16. A polypeptide comprises, sequentially linked: an optional signal peptide, IL-7, an optional linker, an extracellular domain of BCMA or a variant thereof, an optional hinge region or linker, and a transmembrane domain, wherein the signal peptide is the CD52 signal peptide or the IL-7 signal peptide, and the transmembrane domain is the CD8 transmembrane domain or CD52.

17. The polypeptide according to claim 16, wherein the polypeptide comprises, sequentially linked:

a CD52 signal peptide, IL-7, a linker, an extracellular domain of BCMA or a variant thereof, and the CD8 transmembrane domain;
a CD52 signal peptide, IL-7, a linker, an extracellular domain of BCMA or a variant thereof, a hinge region or linker, and CD52;
a CD52 signal peptide, IL-7, an extracellular domain of BCMA or a variant thereof, a hinge region or linker, and CD52;
an IL-7 signal peptide, IL-7, a linker, an extracellular domain of BCMA or a variant thereof, and the CD8 transmembrane domain; or
an IL-7 signal peptide, IL-7, a linker, an extracellular domain of BCMA or a variant thereof, and CD52.

Figure 1

Figure 2

Figure 3

43

Figure 4

Before infusion                    14 weeks after infusion

Figure 5

Before infusion                    22 weeks after infusion

Figure 6

Before infusion

13 weeks after infusion

Figure 7

Before infusion

24 weeks after infusion

Figure 8

## INTERNATIONAL SEARCH REPORT

| International application No. |
|---|
| **PCT/CN2024/078802** |

| | |
|---|---|
| **A.** | **CLASSIFICATION OF SUBJECT MATTER** |

C12N 5/0783(2010.01)i; C07K 19/00(2006.01)i; C12N 15/62(2006.01)i; C12N 5/10(2006.01)i; A61K 39/00(2006.01)i; A61P 35/00(2006.01)i

According to International Patent Classification (IPC) or to both national classification and IPC

| | |
|---|---|
| **B.** | **FIELDS SEARCHED** |

Minimum documentation searched (classification system followed by classification symbols)

    IPC:C12N,C07K,A61K,A61P

Documentation searched other than minimum documentation to the extent that such documents are included in the fields searched

Electronic data base consulted during the international search (name of data base and, where practicable, search terms used)

    CNTXT, WPABSC, WPABS, ENTXTC, ENTXT, 超星读秀学术, Chaoxing Duxiu Scholar, 中国专利生物序列检索系统, China Patent Biological Sequence Search System, CNKI, 万方, WANFANG, ISI web of knowledge, Elsevier Science Direct, Springerlink, pubmed, genbank, ENA, EMBL, Uniport, STN, 百度, Baidu, 百度学术, Baidu Scholar, patentics: TIL, 肿瘤浸润淋巴细胞, IL-7, 膜, 锚定, 结合, 跨膜, 膜型, 标签, 接头, 铰链, CD52, 信号肽, CD8, BCMA, membrane, bound, anchor, mIL-7, GPI, signal peptide, tumor infiltrating lymphocytes, linker, SEQ ID NOs: 31, 33, 35, 37, 39, 41, 43, 45, 47

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| Y | BLOKON-KOGAN, D. et al. "Membrane Anchored IL-18 Linked to Constitutively Active TLR4 and CD40 Improves Human T Cell Antitumor Capacities for Adoptive Cell Therapy" *Journal for ImmunoTherapy of Cancer,* Vol. vol. 10, 02 September 2022 (2022-09-02), e001544 <br>    pp. 1-12 | 1-17 |
| Y | HURTON, L.V. et al. "IL-7 as a Membrane-Bound Molecule for the Costimulation of Tumor-Specific T Cells" *Blood,* Vol. 114, No. (22), 20 November 2009 (2009-11-20), <br>    Abstract 3053 | 1-17 |
| Y | WO 2022111571 A1 (SHANGHAI GENCELLS THERAPEUTICS CO., LTD.) 02 June 2022 (2022-06-02) <br>    claims 1-12 | 8-10 |

☑ Further documents are listed in the continuation of Box C.      ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **14 May 2024** | **20 May 2024** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/078802** |

## C. DOCUMENTS CONSIDERED TO BE RELEVANT

| Category* | Citation of document, with indication, where appropriate, of the relevant passages | Relevant to claim No. |
| --- | --- | --- |
| Y | CN 107406860 A (UCL BUSINESS PLC) 28 November 2017 (2017-11-28)<br>description, paragraphs 120-122, 215-223 and 234-235 | 1-17 |
| Y | SILVER. R.J. et al. "Tumor Infiltrating Lymphocytes Expressing Membrane-bound IL-2 and IL-12 Exhibit Enhanced Proliferation, Function, and Persistence without Requiring Exogenous IL-2 Support"<br>*Annals of Oncology*, Vol. 33, No. (S7), 31 December 2022 (2022-12-31),<br>S891 | 1-17 |
| Y | CN 115698267 A (SHANGHAI GENCELLS THERAPEUTICS CO., LTD.) 03 February 2023 (2023-02-03)<br>claims 1-78 | 8-10 |
| PX | CN 115724996 A (SHANGHAI GENCELLS THERAPEUTICS CO., LTD.) 03 March 2023 (2023-03-03)<br>claims 1-10 | 1-17 |
| PX | CN 116496417 A (BEIJING INSTITUTE FOR CANCER RESEARCH) 28 July 2023 (2023-07-28)<br>claims 1-10 | 1-17 |
| PX | WO 2023088246 A1 (SHANGHAI GENCELLS THERAPEUTICS CO., LTD.) 25 May 2023 (2023-05-25)<br>claims 1-10 | 1-17 |
| T | GUO, J. et al. "Membrane-Bound IL-7 Engineered TIL Therapy for Advanced Ovarian Cancer"<br>*Research Square*, 19 March 2024 (2024-03-19),<br>pages 1-11 | 1-17 |

Form PCT/ISA/210 (second sheet) (July 2022)

## INTERNATIONAL SEARCH REPORT

International application No.

**PCT/CN2024/078802**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/CN2024/078802** |

| Box No. II  Observations where certain claims were found unsearchable (Continuation of item 2 of first sheet) |
| --- |

This international search report has not been established in respect of certain claims under Article 17(2)(a) for the following reasons:

1. ☑ Claims Nos.: **15**
   because they relate to subject matter not required to be searched by this Authority, namely:

   Claim 15 relates to a method for treating a tumor, which falls within methods for treatment of diseases as defined in PCT Rule 39.1(iv); therefore, a search is conducted on the basis of the corresponding pharmaceutical use of the modified TIL.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2024/078802**

| Patent document cited in search report | | | Publication date (day/month/year) | Patent family member(s) | | | Publication date (day/month/year) |
|---|---|---|---|---|---|---|---|
| WO | 2022111571 | A1 | 02 June 2022 | KR | 20230135571 | A | 25 September 2023 |
| | | | | JP | 2024501127 | A | 11 January 2024 |
| | | | | EP | 4253530 | A1 | 04 October 2023 |
| CN | 107406860 | A | 28 November 2017 | SG | 11201706721 | PA | 28 September 2017 |
| | | | | IL | 253820 | A0 | 28 September 2017 |
| | | | | IL | 253820 | B | 25 March 2021 |
| | | | | CA | 2977472 | A1 | 09 September 2016 |
| | | | | US | 2018066280 | A1 | 08 March 2018 |
| | | | | US | 10954530 | B2 | 23 March 2021 |
| | | | | AU | 2016227474 | A1 | 31 August 2017 |
| | | | | GB | 201503500 | D0 | 15 April 2015 |
| | | | | BR | 112017018251 | A2 | 10 April 2018 |
| | | | | WO | 2016139463 | A1 | 09 September 2016 |
| | | | | RU | 2017133854 | A | 02 April 2019 |
| | | | | RU | 2017133854 | A3 | 30 July 2019 |
| | | | | JP | 2023060000 | A | 27 April 2023 |
| | | | | JP | 2018506983 | A | 15 March 2018 |
| | | | | JP | 6695347 | B2 | 20 May 2020 |
| | | | | CL | 2017002199 | A1 | 16 March 2018 |
| | | | | JP | 2020092722 | A | 18 June 2020 |
| | | | | EP | 3904523 | A1 | 03 November 2021 |
| | | | | EP | 3265570 | A1 | 10 January 2018 |
| | | | | EP | 3265570 | B1 | 05 May 2021 |
| | | | | US | 2021348191 | A1 | 11 November 2021 |
| | | | | US | 11814641 | B2 | 14 November 2023 |
| | | | | ES | 2877398 | T3 | 16 November 2021 |
| | | | | MX | 2017010553 | A | 15 March 2018 |
| | | | | KR | 20180002602 | A | 08 January 2018 |
| CN | 115698267 | A | 03 February 2023 | JP | 2023527531 | A | 29 June 2023 |
| | | | | WO | 2021239083 | A1 | 02 December 2021 |
| | | | | KR | 20230019460 | A | 08 February 2023 |
| | | | | US | 2023226111 | A1 | 20 July 2023 |
| | | | | EP | 4159843 | A1 | 05 April 2023 |
| CN | 115724996 | A | 03 March 2023 | None | | | |
| CN | 116496417 | A | 28 July 2023 | None | | | |
| WO | 2023088246 | A1 | 25 May 2023 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2021133059 W
- CN 2021096585 W
- CN 103951753 B
- CN 111182919 A
- WO 2022078310 A1
- CN 103562225 B

**Non-patent literature cited in the description**

- **WARD et al.** Binding Activities of a Repertoire of Single Immunoglobulin Variable Domains Secreted From Escherichia coli. *Nature*, 1989, vol. 341, 544-546
- **HUSTON et al.** Protein Engineering of Antibody Binding Sites: Recovery of Specific Activity in an Anti-Digoxin Single-Chain Fv Analogue Produced in Escherichia coli. *Proc. Natl. Acad. Sci. USA*, 1988, vol. 85, 5879-5883
- **MORGAN et al.** *Science*, 1976, vol. 193, 1007-1008
- *CHEMICAL ABSTRACTS*, 2055536-64-4
- *CHEMICAL ABSTRACTS*, T11438
- *CHEMICAL ABSTRACTS*, 601514-19-6
- *CHEMICAL ABSTRACTS*, 925206-65-1
- *CHEMICAL ABSTRACTS*, 164301-51-3
- **SAMBROOK et al.** Molecular Cloning: A Laboratory Manual. Cold Spring Harbor Laboratory, 2001
- **ROBERT SURIANO et al.** Ex Vivo Derived Primary Melanoma Cells: Implications for Immunotherapeutic Vaccines. *J Cancer*, 2013, vol. 4 (5), 371-382